# EUROPEAN PATENT SPECIFICATION

(11) **EP 2 421 889 B1**
(45) Date of publication and mention of the grant of the patent: **11.12.2013**
(21) Application number: 10717314.8
(22) Date of filing: 20.04.2010
(51) Int. Cl.: C07K 14/375

(54) **REGULATORS INVOLVED IN MUSHROOM FORMATION**
AN DER PILZBILDUNG BETEILIGTE REGULATOREN
RÉGULATEURS INTERVENANT DANS LA FORMATION DES CHAMPIGNONS

(30) Priority: 20.04.2009 US 212953 P
(43) Date of publication of application: 29.02.2012
(60) Divisional application: 13190443.5
(73) Proprietor: Universiteit Utrecht Holding B.V., 3584 CM Utrecht (NL); Stichting voor de Technische Wetenschappen, 3527 JP Utrecht (NL)
(72) Inventor: OHM, Robin Arthur, NL-3524 RP Utrecht (NL); DE JONG, Jan, Fokke, NL-3554 BB Utrecht (NL); LUGONES, Luis, Gaston, NL-3981 CX Bunnik (NL); WÖSTEN, Herman, Abel, Bernard, NL-3705 SN Zeist (NL)
(74) Representative: Nederlandsch Octrooibureau
(86) International application number: PCT/NL2010/050206
(87) International publication number: WO 2010/123350

(56) References cited:
- WO-A1-96/41882
- WO-A2-2004/039985
- US-A1- 2002 081 704
- DATABASE UniProt [Online] 26 February 2008 (2008-02-26), "SubName: Full=Predicted protein;" XP002589650 retrieved from EBI accession no. UNIPROT:B0DVA5 Database accession no. B0DVA5
- DATABASE UniProt [Online] 1 July 2008 (2008-07-01), "SubName: Full=C2H2 conidiation transcription factor FlbC;" XP002589651 retrieved from EBI accession no. UNIPROT:B2W700 Database accession no. B2W700
- DATABASE Geneseq [Online] 30 October 2008 (2008-10-30), "Candida albicans protein SEQ ID NO 19662." XP002589652 retrieved from EBI accession no. GSP:ATC99474 Database accession no. ATC99474
- DATABASE UniProt [Online] 15 January 2008 (2008-01-15), XP002589653 Database accession no. A8NBU3
- DATABASE UniProt [Online] 16 August 2004 (2004-08-16), "SubName: Full=YALI0B00660p;" XP002589654 retrieved from EBI accession no. UNIPROT:Q6CG59 Database accession no. Q6CG59
- DATABASE Geneseq [Online] 1 November 2007 (2007-11-01), "Saccharomyces pastorianus nonSc ORF DNA sequence SEQ ID 2659." XP002589655 retrieved from EBI accession no. GSN:AGB18476 Database accession no. AGB18476
- DE GROOT PIET W J ET AL: "Isolation of developmentally regulated genes from the edible mushroom Agaricus bisporus" MICROBIOLOGY, SOCIETY FOR GENERAL MICROBIOLOGY, READING, GB, vol. 143, no. 6, 1 January 1997 (1997-01-01), pages 1993-2001, XP002271468 ISSN: 1350-0872
- KONDOH O ET AL: "A fruiting body-specific cDNA, mfbAc, from the mushroom Lentinus edodes encodes a high-molecular-weight cell-adhesion protein containing an Arg-Gly-Asp motif" GENE, ELSEVIER, AMSTERDAM, NL LNKD- DOI:10.1016/0378-1119(94)00798-W, vol. 154, no. 1, 27 February 1995 (1995-02-27), pages 31-37, XP004042498 ISSN: 0378-1119
- ANDO A ET AL: "A GENE ENCODING A HYDROPHOBIN, FVH1, IS SPECIFICALLY EXPRESSED AFTER THE INDUCTION OF FRUITING IN THE EDIBLE MUSHROOM FLAMMULINA VELUTIPES" CURRENT GENETICS, NEW YORK, NY, US LNKD- DOI:10.1007/S002940100193, vol. 39, no. 3, 1 May 2001 (2001-05-01), pages 190-197, XP008027699 ISSN: 0172-8083

## Description

### Field of the invention

The invention relates to a mushroom and to a method of producing said mushroom wherein the mushroom has an increased expression level of a polypeptide and/or a decreased expression level of a polypeptide, wherein the polypeptide is encoded by a nucleotide sequence that encodes a protein that has at least 40% identity with SEQ ID NO: 56 and/or that has at least 50% identity with SEQ ID NO: 202.

### Background of the invention

Formation of mushrooms is a highly complex developmental process. As an example, we describe a generalized scheme for formation of agaric fruiting bodies such as those of *Agaricus bisporus* (see Kües, 2000; Umar and van Griensven, 1997). After a "critical mass" of submerged mycelium has been formed, hyphae escape the substrate to grow into the air. These hyphae form aggregates, which are called hyphal knots or nodules. Within the knots hyphae aggregate forming a fruiting body initial. Within the core of the initial differentiation of cells occurs. The lower part will develop into the stipe, while the cap will be formed from the upper part. Within the cap different tissues develop. In the inner part of the cap the pileus trama and gills with a hymenium can be distinguished. In the hymenium different cell types are formed, among which the basidia. In the basidia karyogamy and meioses take place, ultimately resulting in basidiospores. That development of fruiting bodies is complex is also exemplified by the fact that formation of the different tissues overlaps in time. Moreover, cells in the developing mushroom differ in diameter, length, the number of septa, nuclei and vacuoles as well as the molecular composition (e.g. the content of reserve carbohydrate).

Spores formed by *A. bisporus* contain two nuclei with a different mating type. Germination of these spores thus results in a self-fertile heterokaryotic mycelium, containing a variable number of both nuclear types. In contrast, the fertile stage of a majority of mushroom forming fungi results from a mating of two compatible strains with different mating type loci. During mating, partners exchange nuclei. These nuclei do not fuse but are maintained in the hyphal compartment. Such mycelia are therefore called heterokaryotic (in the case that each compartment contains one nucleus of each type it is called a dikaryon). They can form fruiting bodies under the appropriate environmental and nutritional conditions. The mating type loci are the master regulators of fruiting body development. Little is known about the mating type system of *A. bisporus.* It is assumed that this fungus contains a single mating type locus. The mating type loci of *Schizophyllum commune* and *Coprinus cinereus* and their role in development have been studied well (for a review see Kües, 2000). Both *S. commune* and *C. cinereus* contain two mating type loci. The A locus encodes homeodomain proteins. These proteins function by forming heterodimers with homeodomain proteins encoded in a compatible A locus. Some of these homeodomain proteins also seem to form functional homodimers. The B locus encodes pheromones and receptors. These receptors can bind pheromones encoded by other alleles of the B locus. Both the A and the B locus regulate distinct cellular processes involved in establishing the dikaryotic mycelium. However, they co-ordinately regulate fruiting body initiation. Clearly, the presence of compatible A and B mating type loci is not sufficient for fruiting. For instance, in *C*. *cinereus* aggregates formed by a dikaryon can develop into a fruiting initial or into sclerotia. Environmental conditions such as light and nutrient availability will determine which developmental program will be switched on.

Little is known about regulatory proteins other than those encoded by the mating type loci that are involved in mushroom formation in general and fruiting initiation in particular. In contrast to *A. bisporus,* at least some mutants and genes have been identified in S. *commune* and C. *cinereus.* The *fbf* mutation is a frequently observed recessive mutation in *S*. *commune* that suppresses dikaryon specific processes in vegetative hyphae as well as formation of fruiting bodies (Springer and Wessels, 1989). The *FBF* gene thus seems to be an activator. On the other hand, the *FRT* gene of *S*. *commune* suppresses expression of dikaryon-specific genes in monokaryons (Horton et al., 1999). Other mutant strains affected in fruiting body development in *S. commune* have been described but the genes involved have not been identified. These mutants are affected in the morphology of the fruiting body or its sporulation (Raper and Krongelb, 1958; Bromberg and Schwalb, 1977). Recently, several genes involved in fruiting body formation have been identified in C. *cinereus.* The *pcc1* gene functions in A regulated development and encodes a putative DNA binding protein (Murata et al., 1998). A mutation in the gene resulted in a complete program of sexual differentiation independent of the mating type genes. The function of *pcc1* is, however, not known. The *ich1* and the *eln2* genes are involved in differentiation of the primordium (Muraguchi et al., 1998; 2000). Mutations in these genes affect pileus and stipe formation, respectively. Clearly, these data do not present a picture how fruiting body formation is regulated at the molecular level.

So far, genes involved in regulation/initiation of mushroom production have not been described. These genes control mushroom formation and are therefore targets to improve mushroom production, to improve quality of mushrooms, to increase predictability of mushroom production and to enable production of mushrooms on substrates on which they can not yet be produced efficiently.

### Description of the invention

We have studied expression of putative transcription factors in the model system of mushroom formation *Schizophyllum commune. S. commune* can be found throughout the world. It is a wood rotting basidiomycete that forms fruiting bodies on felled hard woods but it can also be found on softwood and grass silage. The basidia that are formed in the gills are dispersed and can give rise to a monokaryotic mycelium. Fusion of monokaryons with different alleles in the MATA and MATB mating type loci results in a fertile dikaryon that can form fruiting bodies under the appropriate environmental conditions. *S. commune* can form sporulating fruiting bodies on simple synthetic media within a few days. This and the fact that monokaryons are available was the reason that *S. commune* became a model system for fruiting basidiomycetes. Both classical and molecular genetics have been established in *S. commune.* We are confident that regulatory programs in *S*. *commune, A. bisporus* and other basidiomycetes are quite conserved. We have shown that the promoters of the *sc3* and *gpd* genes of *S. commune* are active in the non-related basidiomycete *Pycnoporus cinnabarinus* (Alves et al., 2004).

Genes involved in timing of mushroom formation and in morphology and yield are targets to improve (i.e., for mushrooms that are currently produced commercially, including but not limited to the common white button mushroom [the "champignon"], the oyster mushroom and shiitake) or to enable (i.e. for mushrooms that are currently not produced in a commercial setting) commercial mushroom formation. We have identified 200 genes in the genome of *S. commune* that encode putative regulators whose expression change during mushroom formation. These genes could be involved in mushroom formation. We have found that inactivation of eight of these genes affected mushroom production. The production was either promoted or decreased, if present at all. Homologues of these putative regulatory genes can be found in other mushroom forming fungi. In the present invention as described further herein, we will make use of the genes which encode putative transcriptional regulators and whose expression change during mushroom formation to 1) enable commercial production of mushrooms that can not yet be produced in a commercial setting, 2) improve yield of mushrooms, 3) improve quality (e.g. shape and homogeneity of morphology), 4) improve predictability of the process of mushroom formation and/or 5) enable production of mushrooms on substrates that can not yet be used for commercial mushroom production.

Mushrooms may be defined as a fleshy, spore-bearing fruiting body of a fungus, typically produced above ground on soil or on its food source. The standard for the name "mushroom" is the cultivated white button mushroom, *Agaricus bisporus,* hence the word mushroom is most often applied to those fungi *(Basidiomycota, Agaricomycetes)* that have a stem (stipe), a cap (pileus), and gills (lamellae, sing. lamella) on the underside of the cap, just as do store-bought white mushrooms. Mushrooms may also have pores in stead of lamellae. The word "mushroom" can also be used for a wide variety of fungal fruiting bodies that produce sexual spores and that either or not have stems, and the term is used even more generally, to describe both the fleshy fruiting bodies of some *Ascomycota* and the woody or leathery fruiting bodies of some *Basidiomycota.* Forms deviating from the standard morphology usually have more specific names, such as "puffball", "stinkhorn", and "morel", and gilled mushrooms themselves are often called "agarics" in reference to their similarity to *Agaricus* or their place *Agaricales.* By extension, the term "mushroom" can also designate the entire fungus when in culture or the thallus (called a mycelium) of species forming the fruiting bodies called mushrooms, or the species itself.

### Polypeptide

In a first aspect, there are disclosed 200 polypeptides suspected to be involved in the regulation of the production of a mushroom. These polypeptides are identified in Table 1 and are available in a public database (http://jgi.doe.gov/Scommune). This application specifically concerns a polypeptide that has at least 40% amino acid identity with SEQ ID NO:56 (Table 1) and/or that has at least 50% amino acid identity with SEQ ID NO:202 (Table 4). Table 5 links the name of each polypeptide to a given SEQ ID NO corresponding to an amino acid sequence. These polypeptides may also be named regulator or transcription factor (TF) since they have been identified by the presence of a motif or domain known to be present in a TF such as a known DNA binding domain, such as a zinc finger domain (for more details see example 1). TF genes are preferably identified based on:
1) automatic annotation using GO (Gene Ontology) (Ashburner et al., 2000),
2) KOG (euKaryotic Orthologous Groups) (Koonin et al., 2004) and
3) PFAM algorithms (Finn et al., 2008).

Identified putative TFs are preferably subsequently blasted (Blast N and blastP) against the genome to identify putative TF genes that were missed in the automatic annotation.

In a second step, their expression is analysed during the production of a mushroom. A TF of the invention is expressed during at least part of the life cycle of a mushroom. For example, the expression of a TF of the invention may change during development of a mushroom.

"Polypeptide" as used herein refers to any peptide, oligopeptide, polypeptide, gene product, expression product, or protein. A polypeptide is comprised of consecutive amino acids. The term "polypeptide" encompasses naturally occurring or synthetic molecules.

Each amino acid sequence described herein by virtue of its identity or similarity percentage (at least 40% identity or similarity for SEQ ID NO's 1-200; at least 50% identity or similarity for SEQ ID NO's 201-208) with a amino acid sequence respectively has in a further preferred embodiment an identity of at least 42%, 45%, 50%, 55%, 60%, 65%, 70%, 75%, 80%, 82%, 85%, 90%, 95%, 96%, 97%, 98%, 99% or 100% identity or similarity with the given polypeptide. In a preferred embodiment, sequence identity or similarity is determined by comparing the whole length of the sequences as identified herein.

Each nucleotide sequence encoding a polypeptide as described herein may encode a fungal polypeptide, i.e. a polypeptide with an amino acid sequence that is identical to that of a polypeptide that naturally occurs in a fungal or a mushroom organism. The functionality of such polypeptide depends on the relatedness (identity or similarity percentage) of the amino acid sequence compared to that of the corresponding identified SEQ ID NO.

A TF is preferably said to be functional when said TF has a detectable transcriptional activity during at least part of the life cycle of a mushroom. Preferably a mushroom is a *Schizophyllum.* More preferably strain 4-8 (FGSC#9210) of *Schizophyllum commune* (Fungal Genetic Stock Center, Missouri, USA). The presence of an activity, preferably a transcriptional activity, is preferably assessed by inactivating a nucleotide sequence encoding said TF in said fungus or mushroom and analysing whether a mushroom will be produced compared to the mushroom production of a control mushroom wherein said nucleotide sequence has not been inactivated. If a mushroom is not produced or if less or more mushroom is produced, said TF is said to exhibit an activity, preferably a transcriptional activity, and therefore to be functional. Less or more mushroom are later defined herein.

Alternatively or in combination with a previous embodiment, a polypeptide of the invention may be a natural polypeptide or it may be a polypeptide that does not occur naturally. A polypeptide that does not occur naturally may be a polypeptide encoded by a nucleic acid sequence that is mutated for example by using site directed mutagenesis or a mutation prone PCR.

### Nucleic acid construct

In a further aspect, there is provided a nucleic acid construct comprising a nucleotide sequence encoding a polypeptide that comprises an amino acid sequence that is encoded by a nucleotide sequence selected from:
(a) a nucleotide sequence that encodes an amino acid sequence that has at least 40 % amino acid identity with SEQ ID NO:56; and,
(b) a nucleotide sequence that encodes an amino acid sequence that has at least 50 % amino acid identity with SEQ ID NO:202
wherein the nucleotide sequence is optionally operably linked to a promoter that is capable of driving expression of the nucleotide sequence in a fungus and/or in a mushroom.

A nucleic acid construct is defined as a nucleid acid molecule, which is isolated from a naturally occurring gene or which has been modified to contain segments of nucleic acid which are combined or juxtaposed in a manner which would not otherwise exist in nature. A nucleic acid molecule is represented by a nucleotide sequence. Optionally, a nucleotide sequence present in a nucleic acid construct is operably linked to one or more control sequences, which direct the production of said polypeptide in a fungus.

Operably linked is defined herein as a configuration in which a control sequence is appropriately placed at a position relative to the nucleotide sequence coding for the polypeptide of the invention such that the control sequence directs the production of the polypeptide of the invention in a fungal cell and/or in a mushroom.

Expression will be understood to include any step involved in the production of the polypeptide including, but not limited to transcription, post-transcriptional modification, translation, post-translational modification and secretion.

Control sequence is defined herein to include all components, which are necessary or advantageous for the expression of a polypeptide. At a minimum, the control sequences include a promoter and transcriptional and translational stop signals.

As used herein, the term "promoter" refers to a nucleic acid fragment that functions to control the transcription of one or more genes or nucleic acids, located upstream with respect to the direction of transcription of the transcription initiation site of the gene, and is related to the binding site identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one skilled in the art to act directly or indirectly to regulate the amount of transcription from the promoter. Within the context of the invention, a promoter preferably ends at nucleotide -1 of the transcription start site (TSS).

A promoter is preferably capable of driving expression of the nucleotide sequence in a fungus and/or in a mushroom. Preferred promoters include: promoters that are constitutively expressed such as that of glyceraldehyde-3-phosphate-dehydrogenase (gpd) of *Schizophyllum commune* (Harmsen et al., 1992) (SEQ ID NO: 209).

The invention also relates to an expression vector comprising a nucleic acid construct of the invention. Preferably, an expression vector comprises a nucleotide sequence of the invention, which is operably linked to one or more control sequences, which direct the production of the encoded polypeptide in a fungal cell and/or in a mushroom. An expression vector may be seen as a recombinant expression vector. An expression vector may be any vector which can be conveniently subjected to recombinant DNA procedures and can bring about the expression of a nucleotide sequence encoding a polypeptide of the invention in a fungus and/or in a mushroom. Depending on the identity of the fungus/mushroom wherein this expression vector will be introduced and on the origin of the nucleotide sequence of the invention, the skilled person will know how to choose the most suited expression vector and control sequences.

Single or multiple copies of a nucleic acid construct may be introduced into a fungal cell and/or a mushroom. A nucleic acid construct may be maintained episomally and thus comprises a sequence for autonomous replication, such as an ARS sequence. Suitable episomal nucleic acid constructs may e.g. be based on the yeast 2µ or pKD1 (Fleer et al., 1991) plasmids. Alternatively, a nucleic acid construct is integrated in one or more copies into the genome of a fungal cell and/or a mushroom. Integration into a fungal cell and/or a mushroom genome may occur at random by illegitimate recombination or via homologous recombination at a targeted integration site. Preferably, a nucleic acid construct integrates into the genome of a fungus and/or a mushroom. This type of nucleic acid construct may comprise a bacterial cloning vehicle, a nucleotide sequence encoding a TF and a selection marker. A selection marker may confer antibiotic resistance or be an auxotrophic marker. Such markers are known to the skilled person. Nucleic acid constructs comprising a bacterial cloning vehicle and a selection marker are for example disclosed in Schuren et al (1994) and Munoz-Rivas et al (1986). Alternatively, this type of nucleic acid construct may be synthesised using techniques such as for example PCR.

If the expression level of a polypeptide of the invention is to be increased, a nucleotide sequence encoding said polypeptide is introduced into an expression construct. If the expression level of a polypeptide of the invention is to be decreased, a nucleotide sequence encoding said polypeptide may be introduced into an inactivation construct. An inactivation construct is known to the skilled person. Such construct may comprise a nucleotide sequence encoding a mutated polypeptide or containing the flanking sequences of a nucleotide sequence encoding said polypeptide. Such a construct should integrate at the endogenous locus of said polypeptide to replace the endogenous gene and inactivate it. Alternatively, the inactivation construct may contain a sequence inducing RNAi. RNAi techniques are known to the skilled person (De Jong et al, 2006).

Inactivation of the polypeptide may be due to the inactivation of the corresponding gene or nucleotide sequence. In the case of an RNAi like inactivation, mRNA levels are reduced. The inactivation construct may also result in mRNA levels similar to that observed in the wild-type. In this case, the encoded mutated polypeptide has a decreased activity, wherein said decreased activity is assessed by comparison with the activity of the polypeptide the mutated polypeptide originates or derives from. An activity of a polypeptide may be assessed using an assay known to the skilled person. Such assay may include the introduction of said mutated polypeptide into a fungus and compare an activity of said expressed mutated polypeptide with corresponding activity of the polypeptide the mutated polypeptide originates from. An activity of a mutated polypeptide may be compared with the activity of a control polypeptide. If a fungus is a *Schizophyllum,* a control activity of a control polypeptide may be an activity as present in the strain *Schizophyllum commune* strain 4-8 (FGSC #9210). Preferably a mutated polypeptide has no detectable activity. An activity has the same meaning as a transcriptional activity or being functional as earlier defined herein.

Suitable procedures for transformation of fungus are well known to the skilled person. For example, the mushroom forming fungus can be transformed using protoplasts using for instance procedures according to van Peer et al (2009).

### Fungal cell/mushroom

In a further aspect, there is provided a fungus and/or a mushroom with an increased expression level of a polypeptide, wherein the polypeptide is encoded by an amino acid sequence that has at least 40% amino acid identity with SEQ ID NO:56, and/or at least 50% amino acid identity with SEQ ID NO: 202.

In a preferred embodiment, a fungus and/or a mushroom comprises a nucleic acid construct or expression vector of the invention as defined in the previous paragraph. The choice of the fungus and/or mushroom will to a large extent depend upon the source of the nucleic acid sequence of the invention. Depending on the identity of the fungus, the skilled person would know how to transform it with the construct or vector of the invention.

In a preferred embodiment, there is provided a fungus and/or a mushroom with an increased expression level of a polypeptide and a decreased expression level of another polypeptide. In another preferred embodiment, there is provided a fungus and/or a mushroom with an increased expression level of at least one polypeptide of the invention and/or a decreased expression level of at least another polypeptide of the invention.

A polypeptide of the invention that is expressed in a fungus and/or a mushroom, as is described above, may be a heterologous polypeptide or an endogenous polypeptide, as is further defined below. Preferably, a polypeptide of the invention that is expressed in a fungus and/or a mushroom is a heterologous polypeptide, for example when a polypeptide of the invention is used to improve the production of an edible mushroom. Where herein it is said that a fungus and/or a mushroom has an "increased expression level of a polypeptide", this includes expression of a heterologous polypeptide, although as a definition the heterologous polypeptide is not naturally expressed by a fungus and/or a mushroom. However, the "increased" expression level in this situation is construed to mean that there is a detectable expression of the heterologous polypeptide whereas a control fungus and/or mushroom does not have detectable expression of the heterologous polypeptide.
A polypeptide of which the expression level is decreased, as is described above, is an endogenous polypeptide.

The increased and/or decreased expression as identified earlier does not need to occur during the whole life cycle of the mushroom/fungus. Depending on the identity of the polypeptide, its expression may be increased respectively decreased during part of the life cycle of the mushroom/fungus. A fungus may be any fungus. Preferably a fungus is a fungus wherein a nucleotide encoding a polypeptide as identified earlier herein is involved during the production of said fungus. A fungus is preferably a fungus which produces a mushroom. More preferably, a mushroom that is attractive to be produced or which is suspected to be attractive to produce a substance of interest. It may be due to commercial reasons. In an embodiment, a fungus is a *Basidiomycete* or an *Ascomycete,* preferably a *Basidiomycete,* preferably an *Agaricales,* more preferably *a Schizophyllaceae* and even more preferably a *Schizophyllum.* More preferred is a *Schizophyllum commune.* Even more preferably *Schizophyllum commune* strain 4-8 (FGSC#9210). Preferred *Agaricales* are for example *Agaricus bisporus, Pleurotus ostreatus* and *Lentius edodus.*

According to a preferred embodiment, a fungus and/or a mushroom has an expression level of a polypeptide that has been increased, i.e. produces more than normal amounts or has an increased production level of said polypeptide of the invention and/or exhibits a higher activity for said polypeptide than the parental fungus/mushroom this fungus/mushroom derives from when both cultured and/or assayed under the same conditions.

"Producing more than normal amount or having an increased production level of said polypeptide" is herein defined as producing more of the polypeptide of the invention than what the parental fungus/mushroom the transformed fungus/mushroom is derived from will produce when both types of cells (parental and transformed cells) are cultured under the same conditions. The increase may occur during part of the life cycle of a fungus/mushroom and/or during a particular stage of the life cycle of said fungus/mushroom. Preferably, a fungus/mushroom of the invention produces at least 3%, 6%, 10% or 15% more of the polypeptide of the invention than the parental fungus/mushroom the transformed fungus/mushroom is derived from will produce when both types of cells (parental and transformed cells) are cultured under the same conditions. Also fungus which produces at least 20%, 30%, 40%, 50%, 60%, 70%, 80%, 90%, 100%, 150%, 500%, 1000%, 10.000% or more of said polypeptide than the parental fungus/mushroom are preferred. According to another preferred embodiment, the production level of the polypeptide of the fungus/mushroom of the invention is compared to the production level of the same polypeptide in a control strain. According to an even more preferred embodiment, when the fungus/mushroom of the invention is a *Schizophyllum* strain, the production level of the polypeptide of the fungus/mushroom of the invention is compared to the production level of the *Schizophyllum commune* strain 4-8 (FGSC #9210), which is taken as control.

The assessment of the production level of the polypeptide may be performed at the mRNA level by carrying out a Northern Blot or an array analysis and/or at the polypeptide level by carrying out a Western blot. All these methods are well known to the skilled person.

"Exhibiting a higher or increased polypeptide activity" is herein defined as exhibiting a higher polypeptide activity than the one of the parental fungus/mushroom the transformed fungus/mushroom derives from using an assay specific for an activity of said polypeptide. Preferably, the fungus/mushroom of the invention exhibits at least a 3%, 6%, 10% or 15% higher activity than the parental fungus/mushroom the transformed fungus/mushroom derives from will exhibit as assayed using a specific assay for an activity of said polypeptide. Also fungus/mushroom which exhibits at least 20%, 30%, 40%, 50%, 60%, 70%, 80%, 90%, 100%, 150%, 500%, 1000%, 10.000% or more of said activity than the parental fungus/mushroom is preferred. According to another preferred embodiment, the level of an activity of said polypeptide of the fungus/mushroom of the invention is compared to the corresponding activity of the *Schizophyllum commune* strain 4-8 (FGSC #9210) strain, which is taken as control.

The skilled person will know which activity could be used for each polypeptide. In a preferred embodiment, said activity is a transcriptional activity which may be assessed as defined earlier herein.

The over-expression or increase of expression or up-regulation may have been achieved by conventional methods known in the art, such as by introducing more copies of a nucleotide encoding a polypeptide into a fungus/mushroom, be it on a carrier or in the chromosome, than naturally present. Alternatively, a nucleotide encoding said polypeptide can be over-expressed by fusing it to a highly expressed or strong promoter suitable for high level protein expression in the selected fungus/mushroom, or combination of the two approaches. The skilled person will know which strong promoter is the most appropriate depending on the identity of the fungus/mushroom. In this context, a strong promoter preferably means a promoter which is able to induce a higher expression of the nucleotide sequence operably linked to it than the expression of said nucleotide sequence when it is operably linked to its endogenous promoter. Over-expression may also be achieved by other methods such as for example by increasing mRNA stability or by introducing introns.

According to a preferred embodiment, a fungus/mushroom has an expression level of a polypeptide that has been decreased, i.e. produces less than normal amounts or has a decreased production level of said polypeptide of the invention and/or exhibits a lower activity for said polypeptide than the parental fungus/mushroom this fungus/mushroom is derived from when both cultured and/or assayed under the same conditions.

"Producing less than normal amount or having a decreased production level of said polypeptide" is herein defined as producing less of the polypeptide of the invention than what the parental fungus/mushroom the transformed fungus/mushroom is derived from will produce when both types of cells (parental and transformed cells) are cultured under the same conditions. Preferably, a fungus/mushroom of the invention produces at least a 3%, 6%, 10% or 15% less of the polypeptide of the invention than the parental fungus/mushroom the transformed fungus/mushroom is derived from will produce when both types of cells (parental and transformed cells) are cultured under the same conditions. Also fungus/mushroom which produces at least 20%, 30%, 40%, 50%, 60%, 70%, 80%, 90%, 100% or 150% less of said polypeptide than the parental fungus are preferred. It is also encompassed by the present invention that no expression of said polypeptide is detectable. Preferably said expression is not detectable during at least part of the life cycle of said fungus/mushroom. According to another preferred embodiment, the production level of the polypeptide of the fungus/mushroom of the invention is compared to the production level of the production level of the same polypeptide in a control strain. According to an even more preferred embodiment, when the fungus/mushroom of the invention is a *Schizophyllum* strain, the production level of the polypeptide of the fungus/mushroom of the invention is compared to the production level of the *Schizophyllum commune* strain 4-8 (FGSC #9210), which is taken as control.

The assessment of the production level of the polypeptide may be performed at the mRNA level by carrying out a Northern Blot or an array analysis and/or at the polypeptide level by carrying out a Western blot. All these methods are well known to the skilled person.

"Exhibiting a lower or decreased polypeptide activity" is herein defined as exhibiting a lower polypeptide activity than the one of the parental fungus/mushroom the transformed fungus/mushroom is derived from using an assay specific for an activity of said polypeptide. Preferably, the fungus/mushroom of the invention exhibits at least.3%, 6%, 10% or 15% lower activity than the parental fungus/mushroom the transformed fungus/mushroom derives from will exhibit as assayed using a specific assay for an activity of said polypeptide. Also fungus/mushroom which exhibits at least 20%, 30%, 40%, 50%, 60%, 70%, 80%, 90%, 100% or 150% less of said activity than the parental fungus/mushroom is preferred. According to another preferred embodiment, the level of an activity of said polypeptide of the fungus/mushroom of the invention is compared to the corresponding activity of the *Schizophyllum commune* strain 4-8 (FGSC #9210) strain, which is taken as control. Here also an activity of a polypeptide of the invention preferably refers to a transcriptional activity of said polypeptide as earlier defined herein.

According to a more preferred embodiment, a fungus/mushroom does not produce any detectable amounts of the polypeptide of the invention and/or does not exhibit any detectable activity of said polypeptide. Preferably, a fungus/mushroom does not produce or produces substantially no polypeptide of the invention.

The lowering of the expression level of the polypeptide of the invention and/or the lowering of its activity level may have been achieved by conventional methods known in the art, such as by inactivating or down-regulating or decreasing the expression level of an endogenous nucleotide encoding said polypeptide of said fungus/mushroom.

The term "endogenous" when used with respect to a nucleic acid or polypeptide molecule refers to a nucleic acid or polypeptide as natively expressed in a fungus/mushroom, preferably in a wild type state.

The term "heterologous" is used as opposite of "endogenous". The term "heterologous" when used with respect to a nucleic acid or polypeptide molecule refers to a nucleic acid or polypeptide from a foreign fungus/mushroom which does not occur naturally as part of a given fungus/mushroom (genome or DNA or RNA from said fungus) or which is found in a fungus/mushroom or location or locations in the genome or DNA or RNA sequence that differ from that in which it is found in nature. Heterologous nucleic acids or proteins are not endogenous to the fungus/mushroom into which they are introduced, but have been obtained from another fungus/mushroom or synthetically or recombinantly produced. Generally, though not necessarily, such nucleic acids encode proteins or polypeptides that are not normally produced by the fungus in which the DNA is transcribed or expressed, similarly exogenous RNA codes for proteins not normally expressed in the fungus/mushroom in which the exogenous RNA is present. Furthermore, it is known that a heterologous protein or polypeptide can be composed of homologous elements arranged in an order and/or orientation not normally found in a fungus/mushroom in which it is transferred, *i.e.* the nucleotide sequence encoding said protein or polypeptide originates from the same species but is substantially modified from its native form in composition and/or genomic locus by deliberate human intervention. Heterologous nucleic acids and proteins may also be referred to as foreign nucleic acids or proteins. Any nucleic acid or protein that one skilled in the art would recognize as heterologous or foreign to a fungus/mushroom in which it is expressed is herein encompassed by the term heterologous nucleic acid or protein. The term heterologous also applies to non-natural combinations of nucleic acid or amino acid sequences, *i.e.* combinations where at least two of the combined sequences are foreign with respect to each other.

This inactivation or down regulation may have been achieved by deletion of one or more nucleotides in the encoding gene. Alternatively, it may have been caused by an RNAi-like mechanism. In another embodiment, the invention relates to a fungus/mushroom which has a mutation in its gene or nucleotide encoding said polypeptide. Preferably to construct a fungus/mushroom having an inactivated nucleotide encoding said polypeptide, a replacement or inactivation vector is prepared and is subsequently introduced into a fungus/mushroom by transformation. The skilled person knows how to construct such a vector. For example, such vector may comprise flanking regions of a nucleotide coding for a polypeptide with a selection marker gene present in between said flanking regions.

Alternatively or in combination with the inactivation of the endogenous nucleotide encoding said polypeptide, the expression of said nucleotide encoding said polypeptide can be lowered by fusing it to a weak promoter suitable for low level protein expression in the selected fungal organism/ selected mushroom. A weak promoter is herein defined as a promoter which is able to induce a lower expression of the nucleotide sequence operably linked to it than the expression of said nucleotide sequence when it is operably linked to its endogenous promoter.

Alternatively or in combination with any of the mentioned ways of obtaining a fungus/mushroom of the invention, one may cross natural isolates of fungus/mushroom with different degrees of expression of the target genes (i.e. nucleotide encoding polypeptide as identified herein) or one may modify the expression of these genes by mutation, by genetic modification, or by other methods such as the application of a chemical or a physical stimulus. A way of modifying the expression of these polypeptides is to submit a fungus to classical mutagenesis and screening for a fungus/mushroom having a desired expression level of said polypeptides as identified herein.

Further, a fungus/mushroom is disclosed wherein the polypeptide whose expression level is increased comprises an amino acid sequence that has at least 40% amino acid identity or similarity with a sequence selected from the group consisting of SEQ ID NO: 29; 177; 20; 56; 4; and/or that has at least 50% amino acid identity or similarity with a sequence selected from the group consisting of SEQ ID NO: 204; 207; 208; 202; 201, and wherein the polypeptide whose expression level is decreased comprises an amino acid sequence that has at least 40% amino acid identity or similarity with a sequence selected from the group consisting of SEQ ID NO: 55; 21; 54; and/or that has at least 50% amino acid identity or similarity with a sequence selected from the group consisting of SEQ ID NO: 206; 205; 203.

The 200 identified genes are present in Table 1. In order to know whether the expression of a given gene/nucleotide of Table 1 has to be increased or decreased, the skilled person may decrease, preferably inactivate this gene in a given fungus or mushroom. Preferably a mushroom is a *Schizophyllum.* More preferably strain 4-8 (FGSC#9210) of *Schizophyllum commune.* Subsequently, one analyzes whether a mushroom will be produced compared to the mushroom production of a control mushroom wherein said nucleotide sequence has not been inactivated. Ways of inactivating a gene have been described herein.

If a mushroom is not produced or if less mushroom is produced, the expression level of said gene/nucleotide sequence or corresponding expression level of activity of the encoded polypeptide is preferably to be increased in a fungus/mushroom encompassed by the present invention.

On the contrary, if a mushroom is produced or if more mushroom is produced, said gene/nucleotide sequence or corresponding expression level of activity of the encoded polypeptide is preferably to be decreased in a fungus/mushroom encompassed by the present invention. However, should clusters of mushrooms be avoided, then the expression level of activity of the encoded polypeptide is preferably to be increased in a fungus/mushroom encompassed by the present invention. For example, when it is desired that a mushroom develops that is not hindered by other, perhaps smaller, mushrooms in their vicinity that do not fully develop. For example, the inventors found that inactivation of SEQ ID NO: 21, 54, 55 led to more mushroom production. Therefore, in order to produce more mushrooms, it is preferred to reduce the expression of a polypeptide comprising an amino acid sequence with identity or similarity to SEQ ID NO: 21, 54, or 55 as further defined elsewhere herein. However, should fewer but larger mushrooms be desired, it is preferred to over-express a polypeptide comprising an amino acid sequence with identity or similarity to SEQ ID NO: 21, 54, or 55.

The meaning of less mushroom is preferably at least a 3%, 6%, 10% or 15% less of the mushroom than the parental mushroom the transformed mushroom is derived from will produce when both types of cells (parental and transformed cells) are cultured under the same conditions. Also at least 20%, 30%, 40%, 50%, 60%, 70%, 80%, 90%, 100% or 150% less of said mushroom than the parental mushrooms are preferred. It is also encompassed by the present invention that no mushroom is detectable.

The meaning of more mushroom is preferably at least a 1%, 3%, 6%, 10% or 15% more mushroom than the parental mushroom the transformed mushroom derives from will produce when both types of cells (parental and transformed cells) are cultured under the same conditions. Also at least 20%, 30%, 40%, 50%, 60%, 70%, 80%, 90%, 100%, 150%, 500%, 1000%, 10.000% or more of said mushroom than the parental mushrooms are preferred.

Detection of mushroom is preferably done visually, such as for example by determining the number of mushrooms in a petridish, a container with substrate or on a mushroom bed.

In the experimental part some genes identified in Table 1 have already been inactivated. Results are presented in Table 3. From these results, we conclude that in a preferred embodiment, a fungus/mushroom is provided wherein the polypeptide whose expression level is increased comprises an amino acid sequence that has at least 50%, 55%, 60%, 65%, 70%, 75%, 80%, 82%, 85%, 90%, 95%, 96%, 97%, 98%, 99% or 100% amino acid identity or similarity with a sequence selected from the group consisting of SEQ ID NO: 204, 207, 208, 202 and 201 and wherein the polypeptide whose expression level is decreased comprises an amino acid sequence that has at least 50%, 55%, 60%, 65%, 70%, 75%, 80%, 82%, 85%, 90%, 95%, 96%, 97%, 98%, 99% or 100% amino acid identity or similarity with a sequence selected from the group consisting of SEQ ID NO: 206, 205 and 203.

SEQ ID NO: 204, 207, 208, 202 and 201 encode parts of fst4, hom2, wc2, c2h2, briI respectively. Corresponding proteins are identified by the following ID in Table 1: 66861; 257987, 13988, 114363, 255701 (SEQ ID NO: 29; 177; 20; 56; 4, respectively).

SEQ ID NO: 206, 205 and 203 encode for parts of hom1, gat1, and fst3 respectively. Corresponding proteins are identified by the following ID in table 1: 257652, 255004, 257422 (SEQ ID NO: 55; 21; 54, respectively).

Such fungus/mushroom is attractive to be used in many different applications.

These procedures would improve/enable mushroom production. Improvement or enabling commercial mushroom production may result from
- growth on substrates that can not yet be used to grow mushrooms commercially
- controlled formation of mushrooms in time and space
- increased production levels
- increased quality (e.g. more homogeneity in size and outgrowth)

In another embodiment, our invention may enable to improve growth of mushrooms in a commercial setting, or to allow commercial production of species that can not yet be produced, thereby creating opportunities to produce edible fungi in a cheaper way or to produce (or improve production of) pharmaceuticals or proteins that are of interest for agriculture, food, feed or non-food or non-feed applications. These proteins may either or not originate from the mushroom forming fungus. Some possible applications of a fungus of the invention are below presented.

### Method for production

In a further aspect, there is provided a method for the production of a fungus/mushroom as identified in the previous section. The skilled person knows how to carry out such a method for instance described in Stamets and Chilton (1983) and van Griensven (1988). Such production includes colonization of a substrate (with or without a casing layer) followed by a phase where fruiting bodies are produced. The production may be carried out at a commercial scale with an optimal production level and/or quality level (more homogeneity in size and outgrowth).

The invention also relates to a method for producing a substance of interest using a fungus/mushroom of the invention. In this method, a fungus/mushroom may have been modified in order to be able to produce said substance. A substance of interest may be any substance that could be produced by a fungus/mushroom. Such substance includes a protein, a polypeptide, a metabolite. A protein or polypeptide in this context may a pharmaceutical protein or polypeptide and/or a protein or polypeptide for interest for food, feed, or non-food, non-feed applications. Such substance may be endogenous for a fungus/mushroom or not. Preferably, the method comprises the steps of: culturing of a fungus/mushroom of the invention that is able to produce a substance of interest under circumstances that are conducive for the generation of the substance of interest; and optional recovery of the substance of interest.

In an embodiment, a fungus/mushroom comprises a nucleic acid construct comprising a nucleotide encoding a protein/polypeptide to be produced. In this case, a substance of interest may be such protein/polypeptide. Alternatively, said protein/polypeptide may be involved in the production or synthesis of such substance of interest. Each feature of said nucleic acid construct has been earlier defined herein. It is also encompassed by the present invention to use a fungus/mushroom which has been further modified by increasing/decreasing the expression level of a protein/polypeptide known to be involved in the method of production of said substance.

### Method for identification

Monitoring the expression levels of such genes or nucleotide encoding said polypeptide may allow the identification of stimuli that are involved in a method for producing a mushroom.

Therefore in a further aspect there is provided a method for identification of a stimulus capable of influencing the production of a mushroom, the method comprising the steps of:
(a) providing a fungus/mushroom;
(b) applying said stimulus to said fungus/mushroom;
(c) determining the expression level of a nucleotide sequence or the activity or steady state level of a corresponding encoded polypeptide in the fungus/mushroom of step b) wherein said polypeptide comprises or consists of an amino acid sequence that has at least 40% amino acid identity with SEQ ID NO:56 or that has at least 50% amino acid identity with SEQ ID NO: 202;
(d) comparing the expression, activity or steady state level determined in (c) with the expression, activity or steady state level of the nucleotide sequence or of the polypeptide in a fungus/mushroom that had not been provided said stimulus; and,
(e) identifying a stimulus that produces a difference in expression level, activity or steady state level of said nucleotide sequence or polypeptide, between the fungus/mushroom that has been provided with said stimulus and the fungus/mushroom that has not been provided said stimulus.

A stimulus may be any kind of stimulus. It may be a physical stimulus as an increase/decrease in temperature, an increase/decrease in light intensity, a change in light wave length, creating some degree of damage by e.g. light or mechanical means. It may be a substance or the presence of a dead or living organism.

In a preferred method, the expression levels, activities or steady state levels of more than one nucleotide sequence or more than one polypeptides are compared. Preferred polypeptides and encoding nucleotide sequence have already been identified herein.

In one aspect the invention also pertains to a substance, treatment or a dead or living organism that is identified in said method.

Each feature of this method has already been explained herein.

The meaning of "produces a difference in expression level, activity or steady state level of the nucleotide sequence or the polypeptide" is the same as the one of "increased expression level of a polypeptide and/or a decreased expression level of a polypeptide".

In a further aspect, we will use a promoter-element that is responsive to (the identified) regulatory genes to:
(i) Identify (down-stream) genes that affect mushroom formation (e.g., but not limited to, the number of mushrooms, or the size of a mushroom);
(ii) Generate biological models, based on current or future commercial mushroom breeds that will allow:
   A. Identification, characterization and application of specific (sets) of regulatory genes, or genes encoding structural proteins relevant for a specific species;
   B. Characterization and improved application of state-of-the-art modulators used for breeding mushrooms;
   C. Generation or identification of derivates of state-of-the-art modulators used for breeding mushrooms with improved or new characteristics;
   D. Generation or identification of novel compounds that will allow improved breeding of mushrooms, or may enable commercial breeding of mushrooms that currently can not be produced commercially.

### Sequence identity

"Sequence identity" is herein defined as a relationship between two or more amino acid (polypeptide or protein) sequences or two or more nucleic acid (nucleotide, polynucleotide) sequences, as determined by comparing the sequences. In the art, "identity" also means the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods, including but not limited to those described in (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48:1073 (1988).

Preferred methods to determine identity are designed to give the largest match between the sequences tested. Methods to determine identity and similarity are codified in publicly available computer programs. Preferred computer program methods to determine identity and similarity between two sequences include e.g. the GCG program package (Devereux et al., 1984). BestFit, BLASTP, BLASTN, and FASTA (Altschul, et al., 1990). The BLAST X program is publicly available from NCBI and other sources (BLAST Manual, Altschul, S., et al., NCBI NLM NIH Bethesda, MD 20894). The well-known Smith Waterman algorithm may also be used to determine identity.

Preferred parameters for polypeptide sequence comparison include the following: Algorithm: Needleman and Wunsch (1970); Comparison matrix: BLOSSUM62 from Hentikoff and Hentikoff (1992); Gap Penalty: 12; and Gap Length Penalty: 4. A program useful with these parameters is publicly available as the "Ogap" program from Genetics Computer Group, located in Madison, WI. The aforementioned parameters are the default parameters for amino acid comparisons (along with no penalty for end gaps).

Preferred parameters for nucleic acid comparison include the following: Algorithm: Needleman and Wunsch, J. Mol. Biol. 48:443-453 (1970); Comparison matrix: matches=+10, mismatch=0; Gap Penalty: 50; Gap Length Penalty: 3. Available as the Gap program from Genetics Computer Group, located in Madison, Wis. Given above are the default parameters for nucleic acid comparisons.

Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to ser; Arg to lys; Asn to gln or his; Asp to glu; Cys to ser or ala; Gln to asn; Glu to asp; Gly to pro; His to asn or gln; Ile to leu or val; Leu to ile or val; Lys to arg; gln or glu; Met to leu or ile; Phe to met, leu or tyr; Ser to thr; Thr to ser; Trp to tyr; Tyr to trp or phe; and, Val to ile or leu.

In this document and in its claims, the verb "to comprise" and its conjugations is used in its non-limiting sense to mean that items following the word are included, but items not specifically mentioned are not excluded. In addition the verb "to consist" may be replaced by "to consist essentially of" meaning that a product or a composition as defined herein may comprise additional component(s) than the ones specifically identified, said additional component(s) not altering the unique characteristic of the invention. In addition, reference to an element by the indefinite article "a" or "an" does not exclude the possibility that more than one of the element is present, unless the context clearly requires that there be one and only one of the elements. The indefinite article "a" or "an" thus usually means "at least one".

The following examples are offered for illustrative purposes only, and are not intended to limit the scope of the present invention in any way.

### Description of the figures

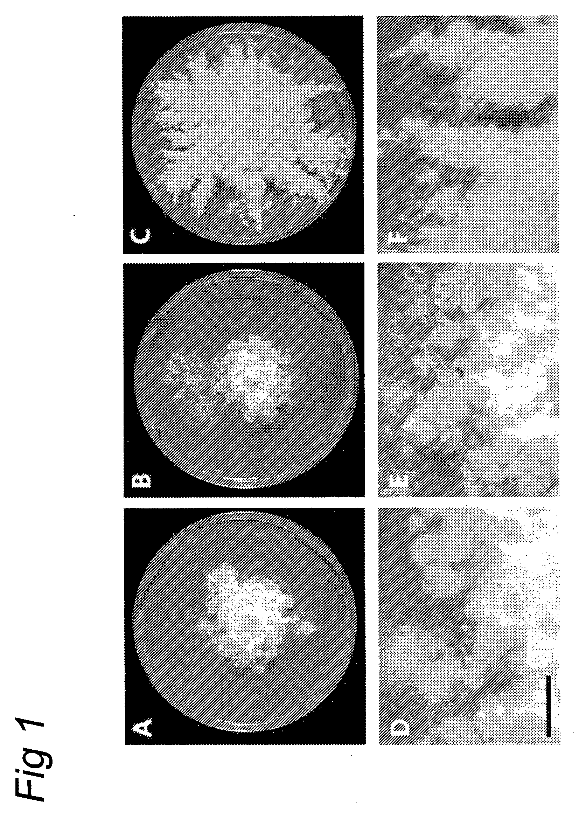

**Figure** 1: Fruiting body formation in a wild-type dikaryon (A, D) and in dikaryons in which the *fst3* (B, E) and *fst4* (C, F) genes have been inactivated. D-F show a magnification of part of the colonies shown in A-C. Bar represents 5 mm (D-F).
**Figure** 2:
   Alignment of protein sequences of WC2 of *S. commune* (proteinID 13988), *L. bicolor* (proteinID 306097) and *C*. *cinereus* (proteinID CC1G_01095).
   Sequence identity is indicated with an asterisk (*) and similarity with a semicolon (:) above the alignment. Alignment was made using CLUSTAL 2.0.12.
**Figure 3****:** Alignment of protein sequences of C2H2 of *S*. *commune* (proteinID 114363), *L. bicolor* (proteinID 310874) and *C. cinereus* (proteinID CC1G_08877).
   Sequence identity is indicated with an asterisk (*) and similarity with a semicolon (:) above the alignment. Alignment was made using CLUSTAL 2.0.12.
**Figure 4****:** Alignment of protein sequences of Fst3 of *S*. *commune* (proteinID 257422), *L. bicolor* (proteinID 307309) and C. *cinereus* (proteinID CC1G_02690).
   Sequence identity is indicated with an asterisk (*) and similarity with a semicolon (:) above the alignment. Alignment was made using CLUSTAL 2.0.12.
**Figure 5****:** Alignment of protein sequences of Fst4 of *S*. *commune* (proteinID 66861), L. *bicolor* (proteinID 308722) and *C. cinereus* (proteinID CC1G_05035).
   Sequence identity is indicated with an asterisk (*) and similarity with a semicolon (:) above the alignment. Alignment was made using CLUSTAL 2.0.12.
**Figure 6****:** Alignment of protein sequences of Gat1 of *S*. *commune* (proteinID 255004), *L. bicolor* (proteinID 292733) and C. *cinereus* (proteinID CC1G_01461).
   Sequence identity is indicated with an asterisk (*) and similarity with a semicolon (:) above the alignment. Alignment was made using CLUSTAL 2.0.12.
**Figure 7****:** Alignment of protein sequences of Hom1 of *S*. *commune* (proteinID 257652), *L. bicolor* (proteinID 324166) and C. *cinereus* (proteinID CC1G_01991).
   Sequence identity is indicated with an asterisk (*) and similarity with a semicolon (:) above the alignment. Alignment was made using CLUSTAL 2.0.12.
**Figure 8****:** Alignment of protein sequences of Hom2 of *S*. *commune* (proteinID 257987), *L. bicolor* (proteinID 293988) and *C. cinereus* (proteinID CC1G_01962).
   Sequence identity is indicated with an asterisk (*) and similarity with a semicolon (:) above the alignment. Alignment was made using CLUSTAL 2.0.12.
**Figure 9****:** Alignment of protein sequences of Bril of *S*. *commune* (proteinID 255701), *L. bicolor* (proteinID 300797) and C. *cinereus* (proteinID CC1G_03649).
Sequence identity is indicated with an asterisk (*) and similarity with a semicolon (:) above the alignment. Alignment was made using CLUSTAL 2.0.12.

### Examples

### Example 1: Identification of putative transcription factors in the genome of Schizophyllum commune

Automatic gene calling of the genome of the *Schizophyllum commune* strain 4-8 (FGSC #9210) (8.29X coverage) by the Joint Genome Institute resulted in 13181 predicted genes (see http://jgi.doe.gov/Scommune). These genes were automatically annotated using GO (Gene Ontology) (Ashburner et al., 2000), KOG (euKaryotic Orthologous Groups) (Koonin et al., 2004) and PFAM algorithms (Finn et al., 2008). This automatic annotation was used as a starting point to identify transcription factors (TFs):
- Based on known DNA binding domains, 190 genes were placed in the GO accession term "Transcription Factor Activity" (GO:0003700).
- The KOG annotation algorithm predicted 569 genes to be generally involved in transcription (Function ID: "K"). From these, 205 transcription factors were manually identified.
- A total of 151 POLYPEPTIDEs were identified based on the presence of PFAM domain PF00096/IPR007087 (Zinc finger, C2H2-type).
All putative TFs were manually inspected and gene model or annotation were adjusted when necessary. Duplicate entries in the list were removed. To identify TFs that were missed during the automatic annotation, the identified TFs were used in a BLASTp (to the database of predicted proteins) and BLASTn (to the genomic database) analysis. Resulting hits were manually inspected and newly identified TFs were added to the list. These procedures led to the identification of 472 putative TFs in the genome of *S*. *commune.*

### Expression profile of the putative transcription factors

In order to determine the expression profile of the putative TFs during fruiting body development, we used the technique MPSS (massive parallel signature sequencing). Total RNA was isolated from the monokaryotic strain 4-40 (CBS 340.81) and from the dikaryon resulting from a cross between 4-40 and 4-39 (CBS 341.81). A 7-day-old colony grown on solid MM at 30 C in the dark was homogenized in 200 ml MM using a Waring blender for 1 min at low speed. 2 ml of the homogenized mycelium was spread out over a polycarbonate membrane that was placed on top of solidified MM. Vegetative monokaryotic mycelium was grown for 4 days in the light. The dikaryon was grown for 2 and 4 days in the light to isolate mycelium with stage I aggregates and stage II primordia, respectively. Mature mushrooms of 3 days old were picked from dikaryotic cultures that had grown for 8 days in the light. RNA was isolated as described (van Peer et al., 2009). MPSS was performed essentially as described (Brenner et al., 2000) except that after DpnII digestion MmeI was used to generate 20 bp tags. Tags were sequenced using the Clonal Single Molecule Array technique (Illumina, Hayward, CA, US). Programs were developed in the programming language Python to analyze the data. Tag counts were normalized to transcripts per million (TPM). Tags with a maximum of < 4 TPM were removed from the data set. TPM values of tags originating from the same transcript were summed to assess their expression levels. If the gene of a putative TF did not contain a known 5' or 3' UTR, then 200 bp of genomic DNA was added to the respective end of the coding sequence of the gene. MPSS expression analysis agreed with expression studies that have been performed in the past (for a review see Wösten, H.A.B. & Wessels, 2006).

### Identification of interesting expression profiles

A TF is considered to be potentially involved in regulation of fruiting body formation when it is either up or down regulated in one of the developmental stages compared to the sterile monokaryon. There are 75 TFs which were at least two fold-up regulated, and 155 at least two-fold down regulated. More interestingly, 29 of these were at least four fold up-regulated and 77 at least 4 fold down-regulated. Even more interestingly, 9 were at least ten fold up-regulated and 27 at least ten-fold down regulated.
The proteinIDs and the MPSS values of these interesting TFs are indicated in Table 1.

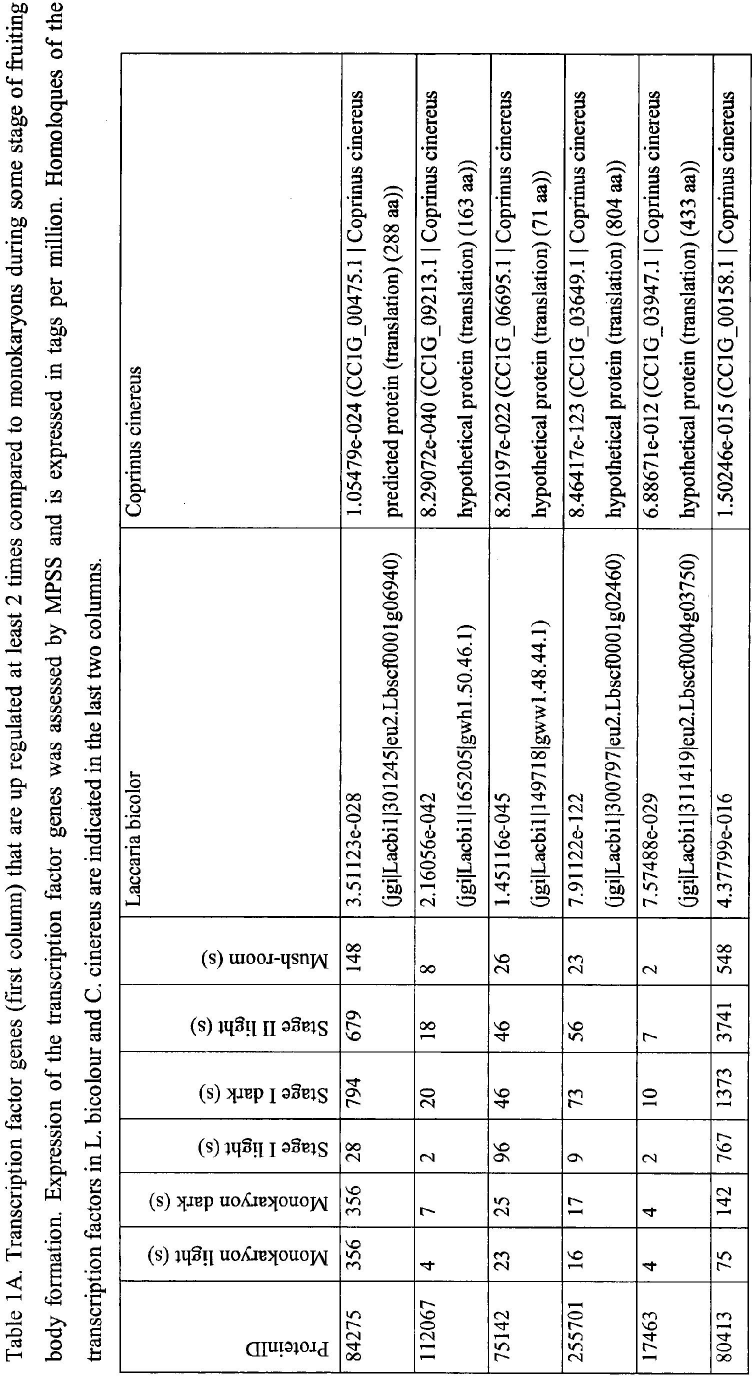

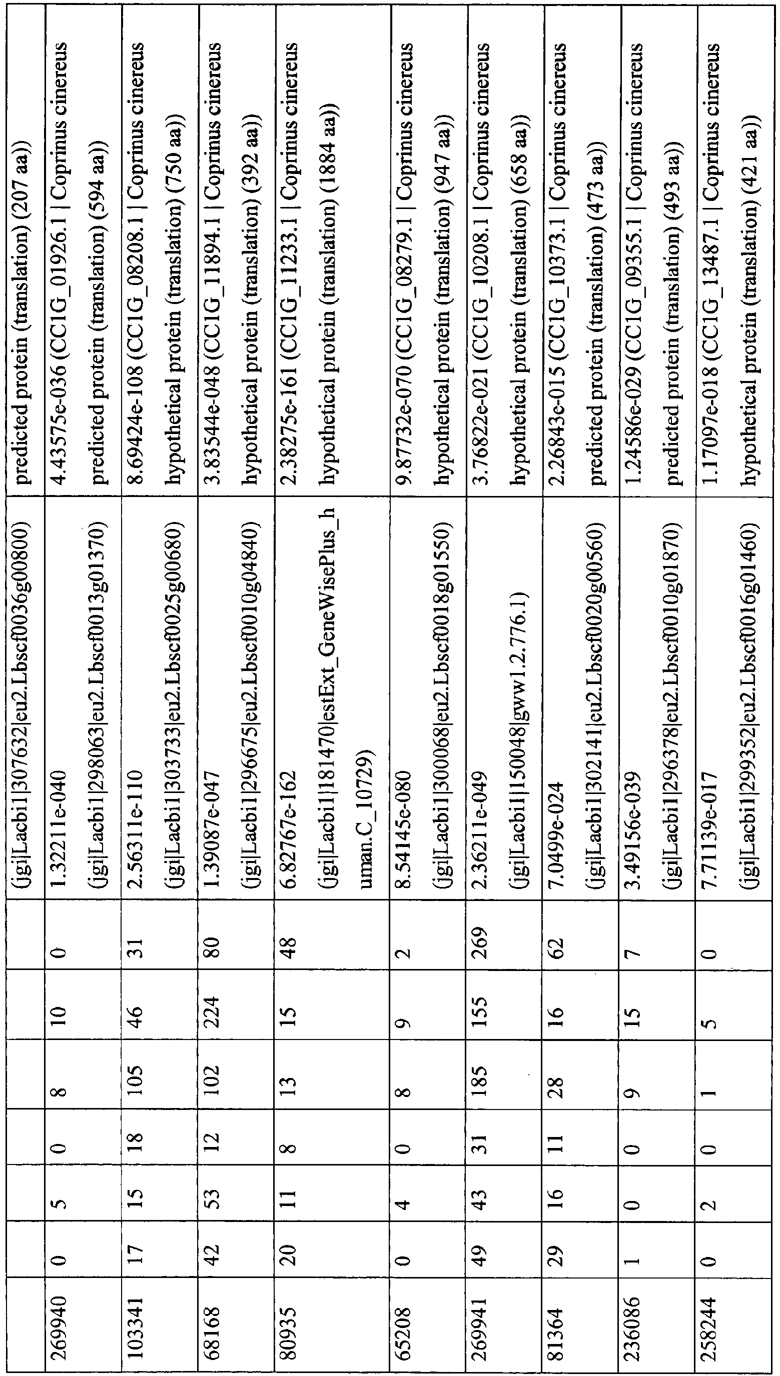

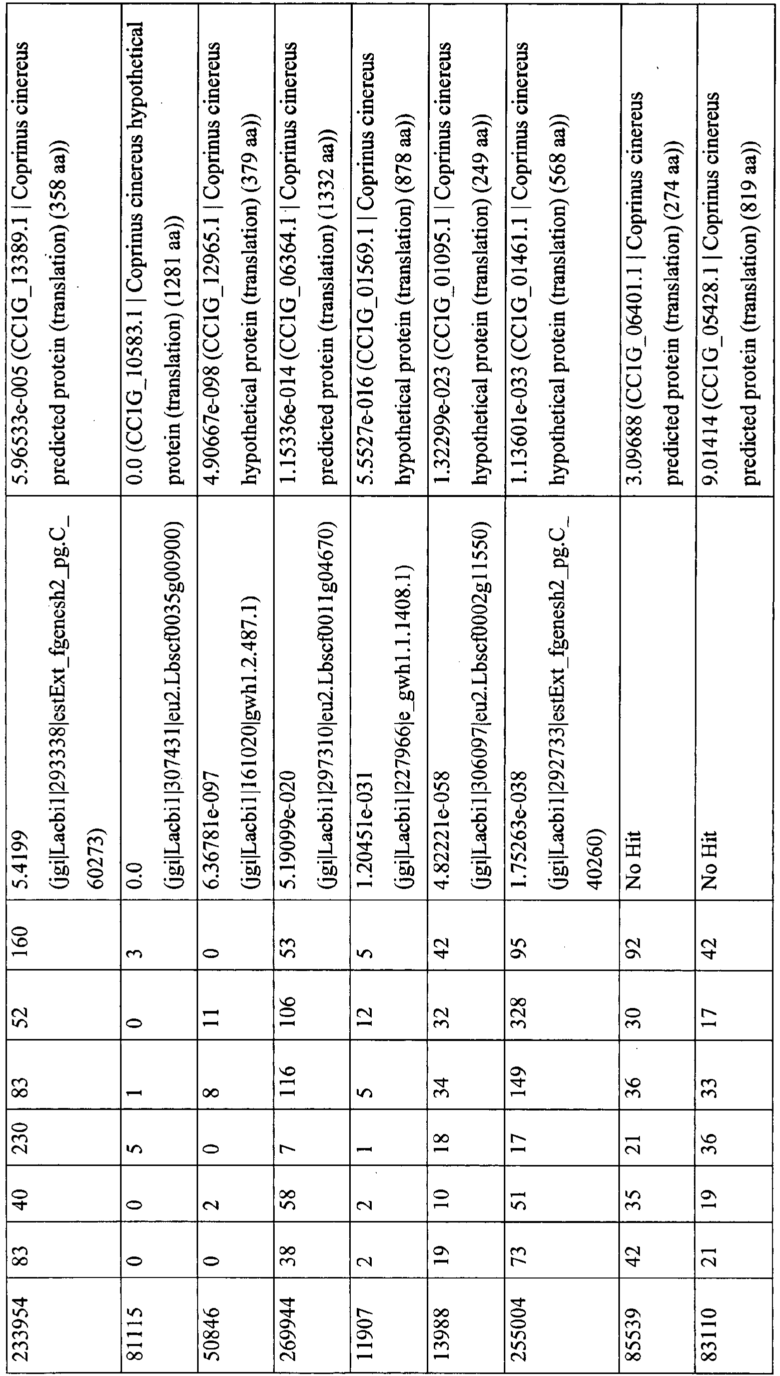

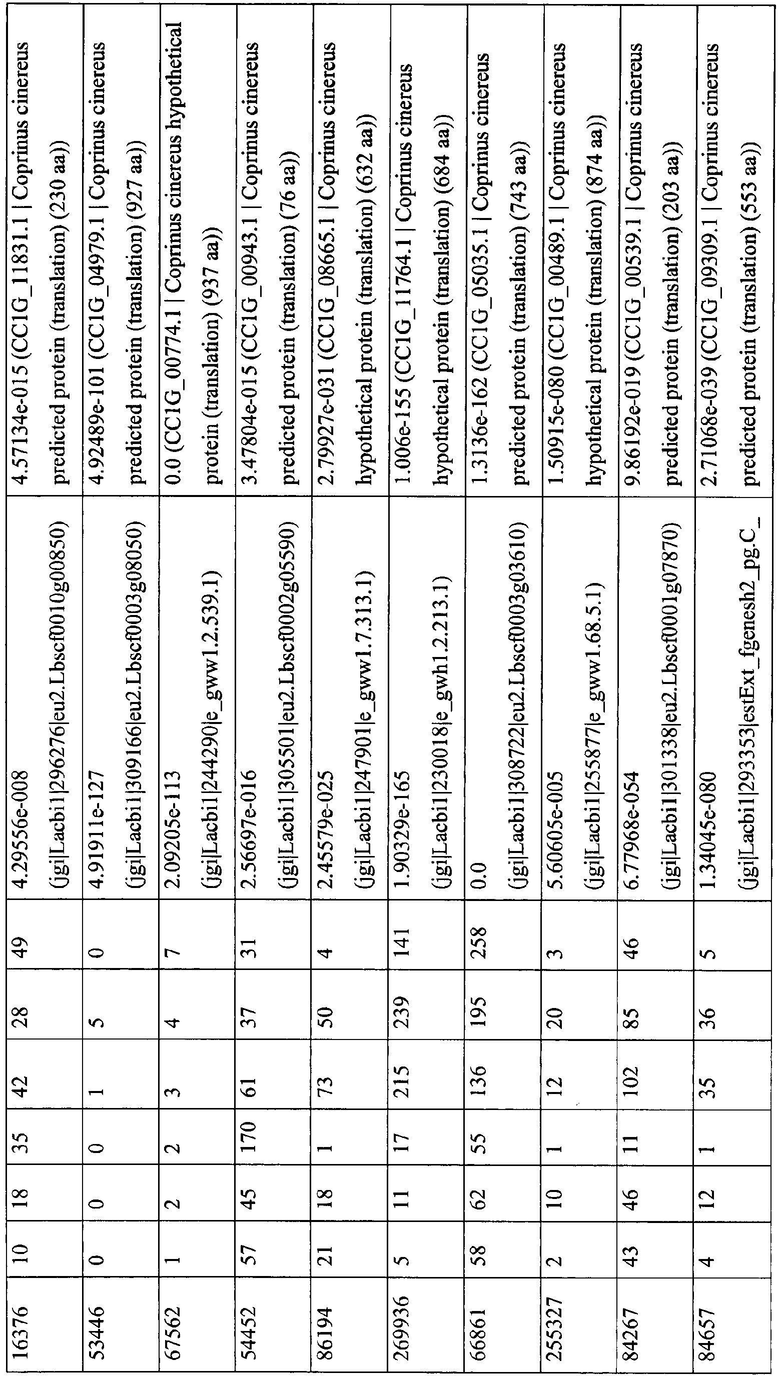

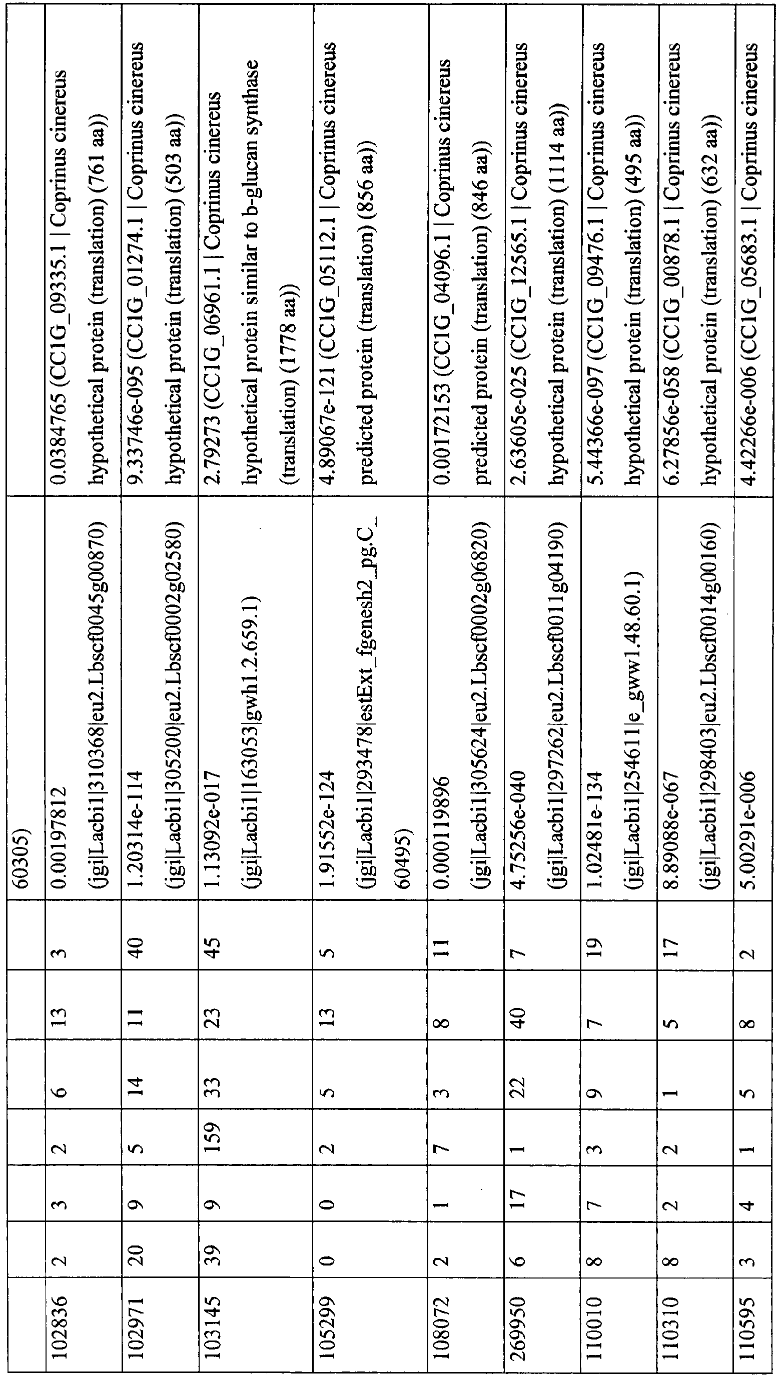

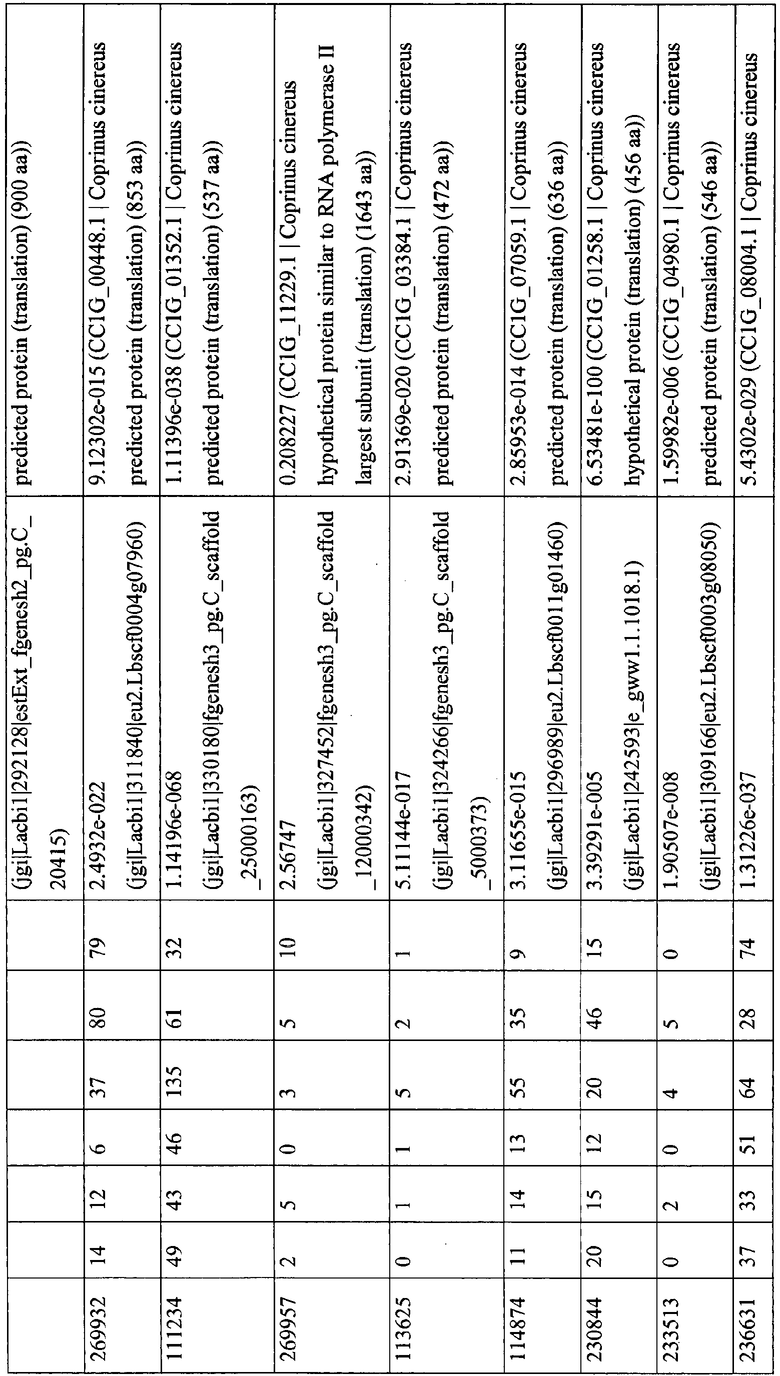

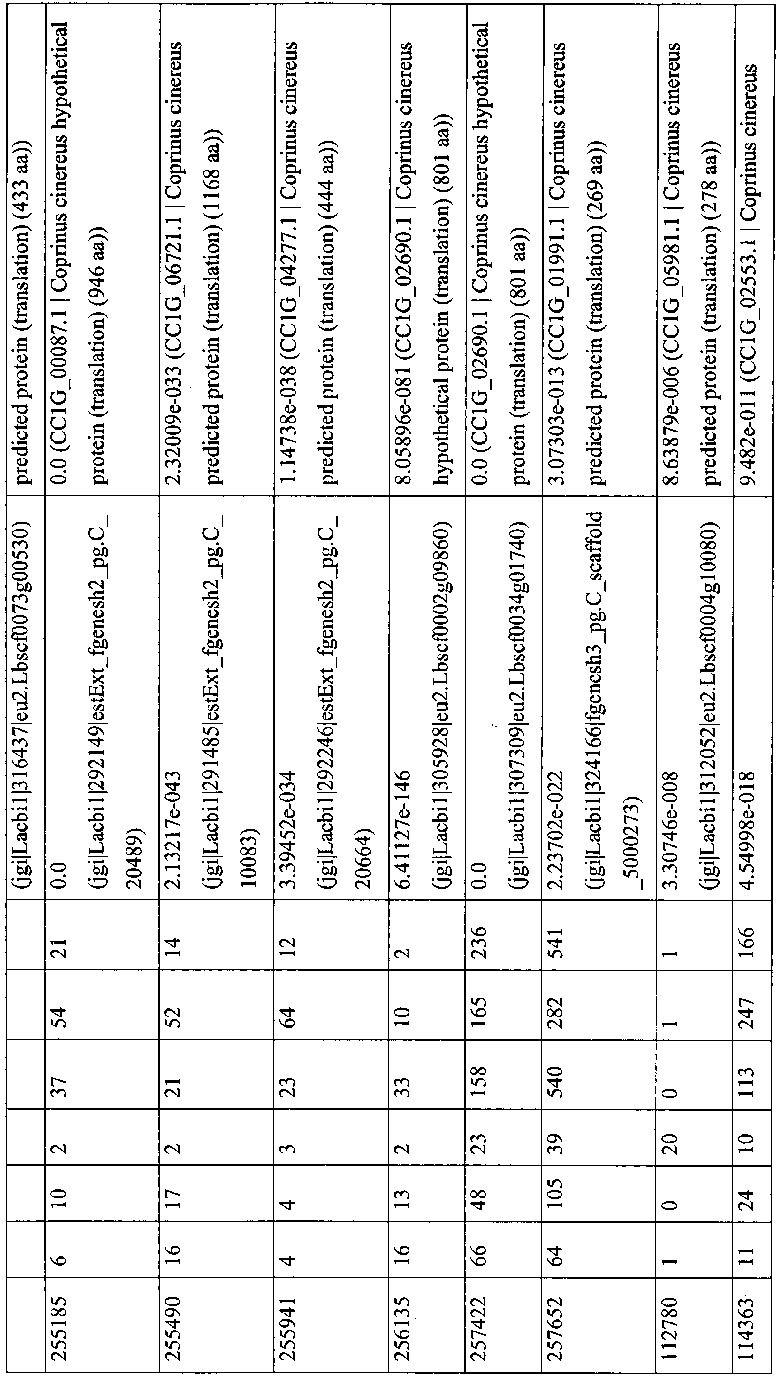

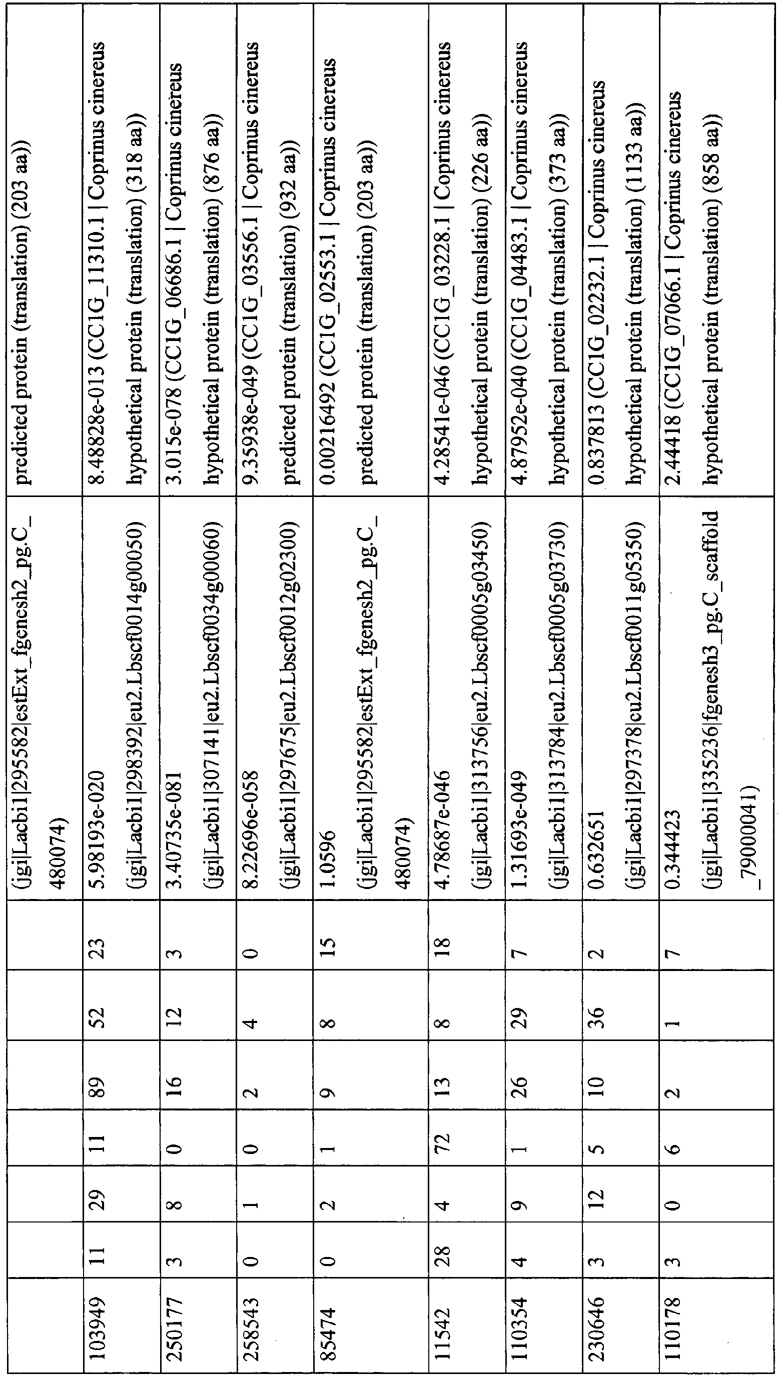

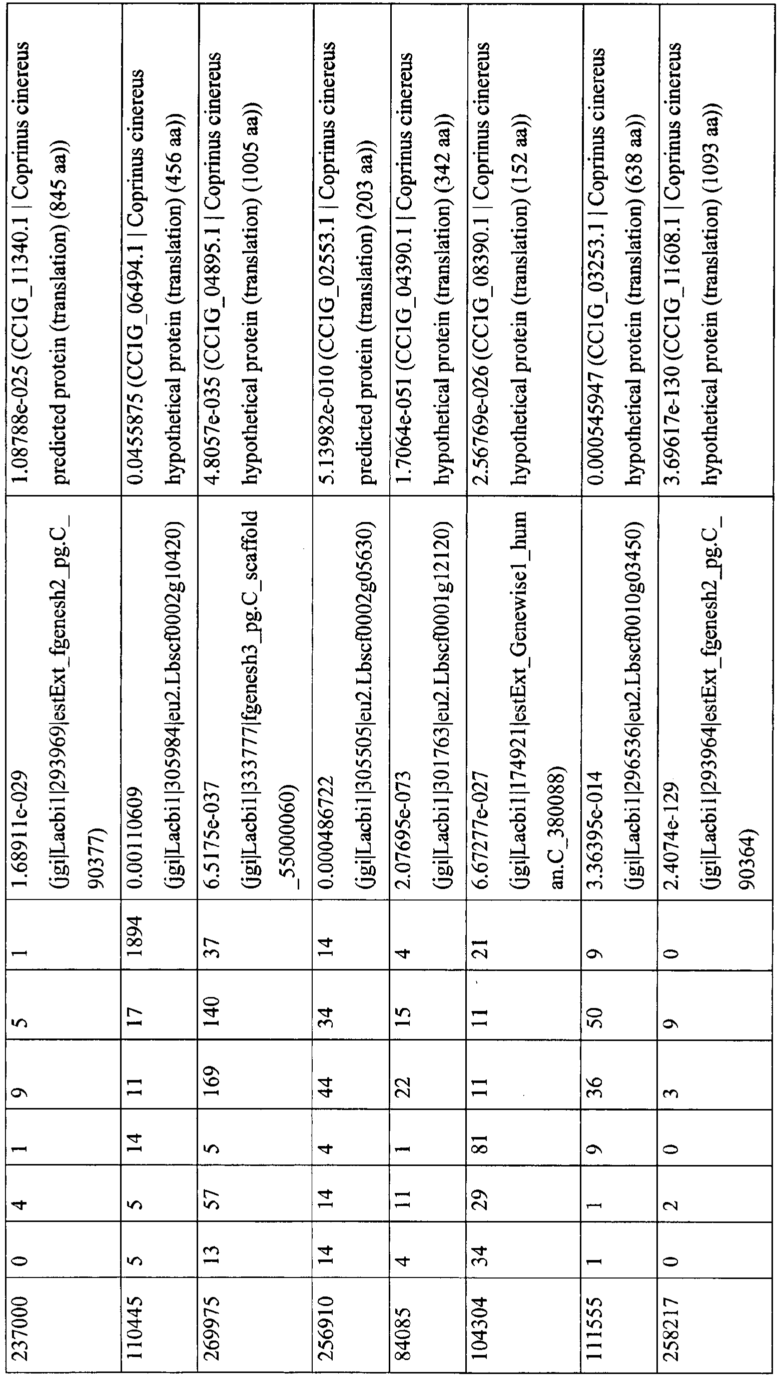

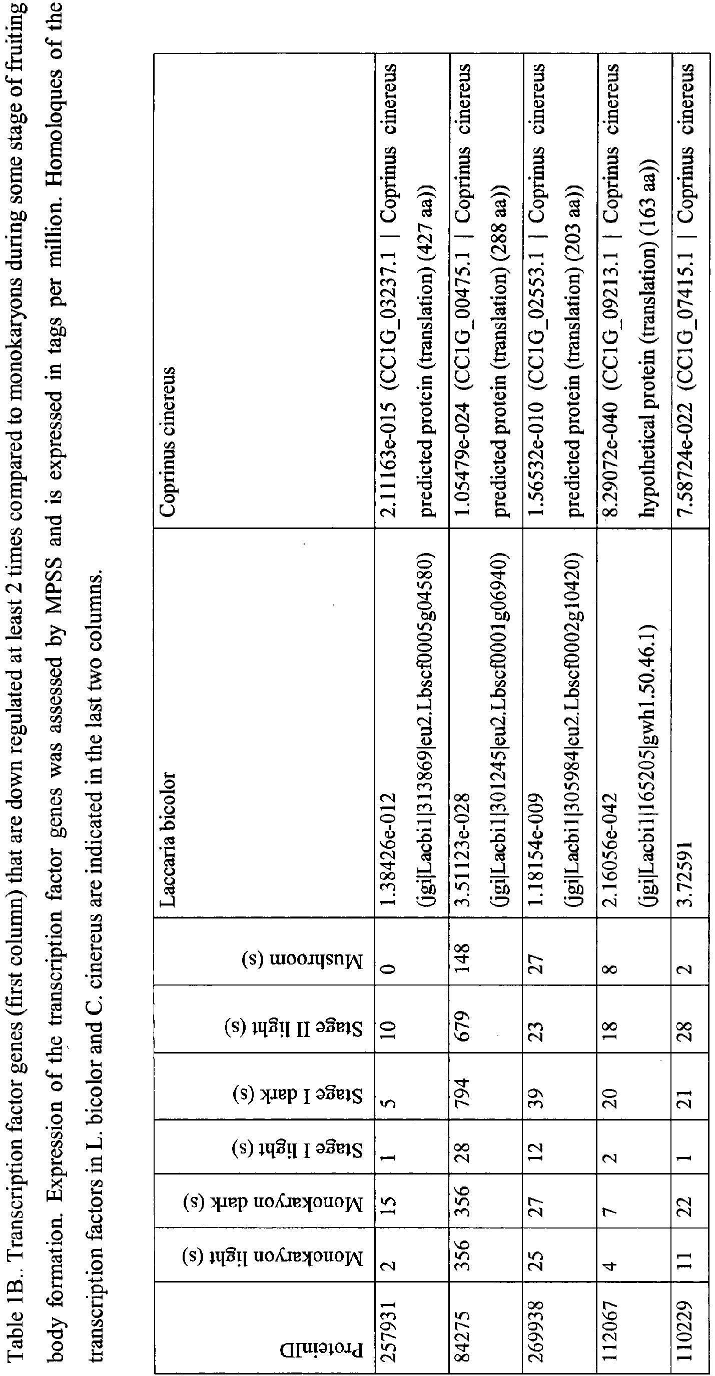

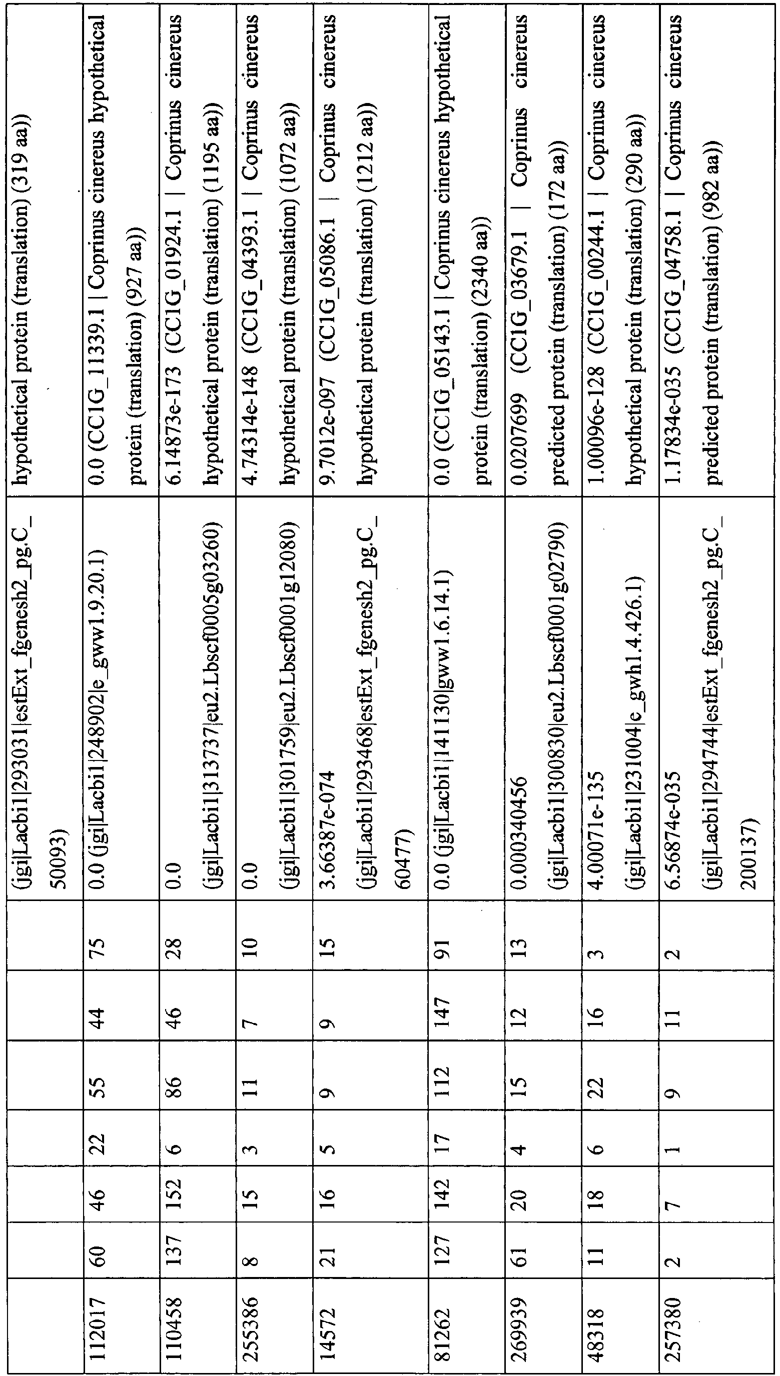

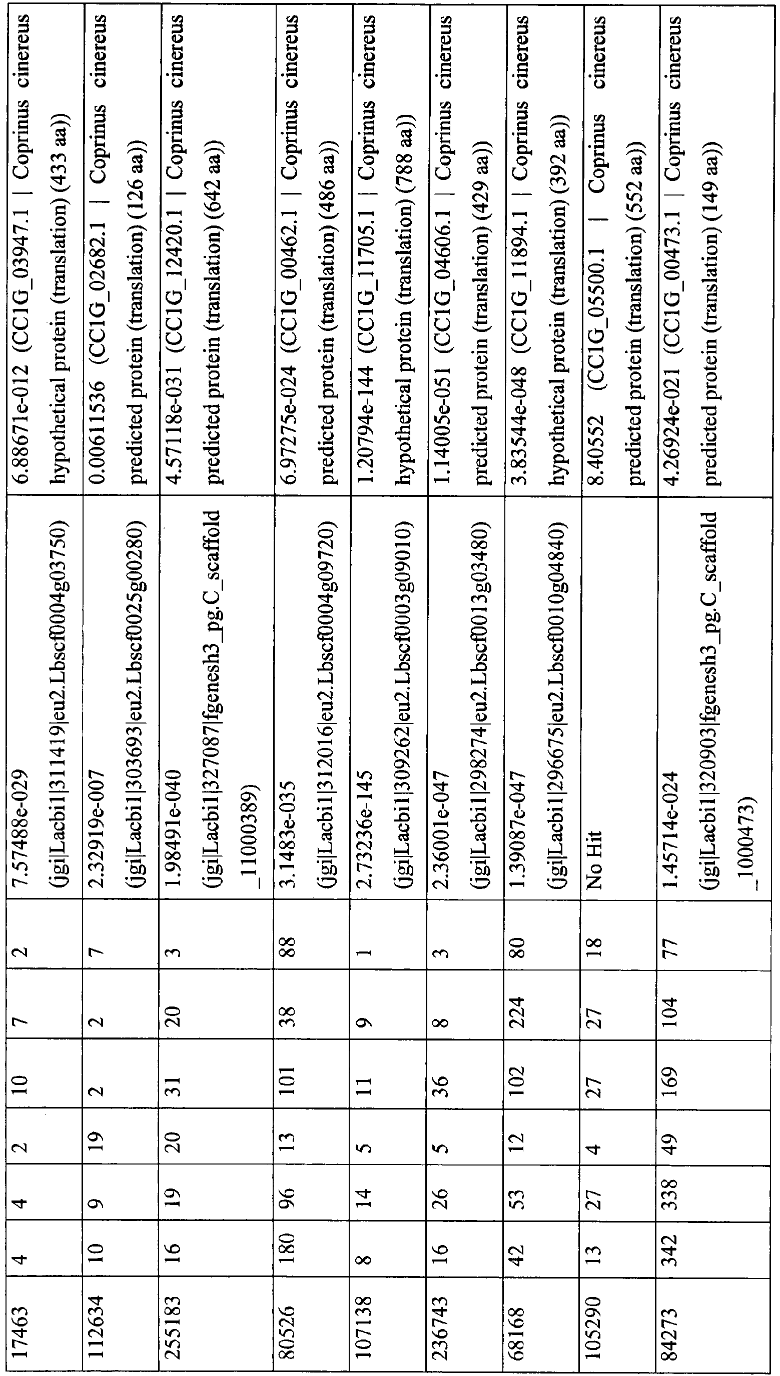

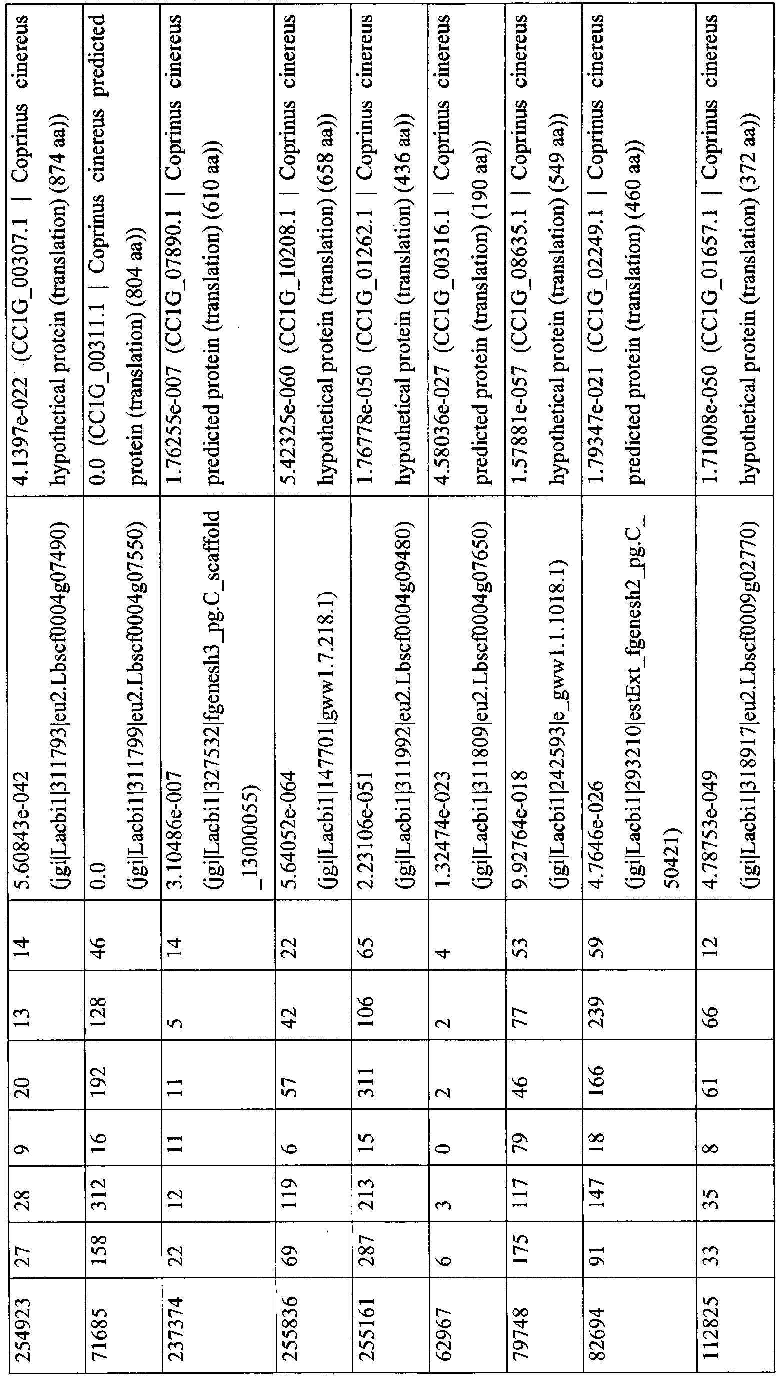

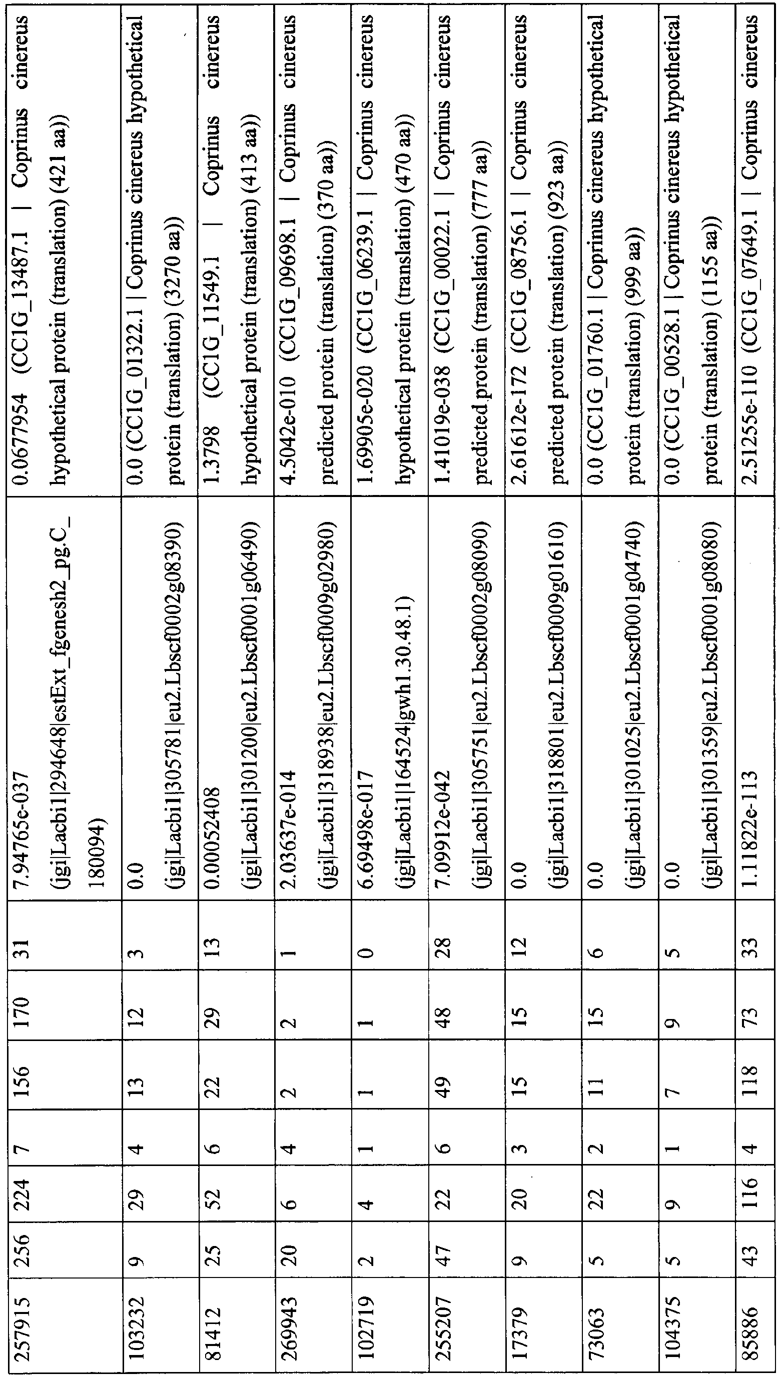

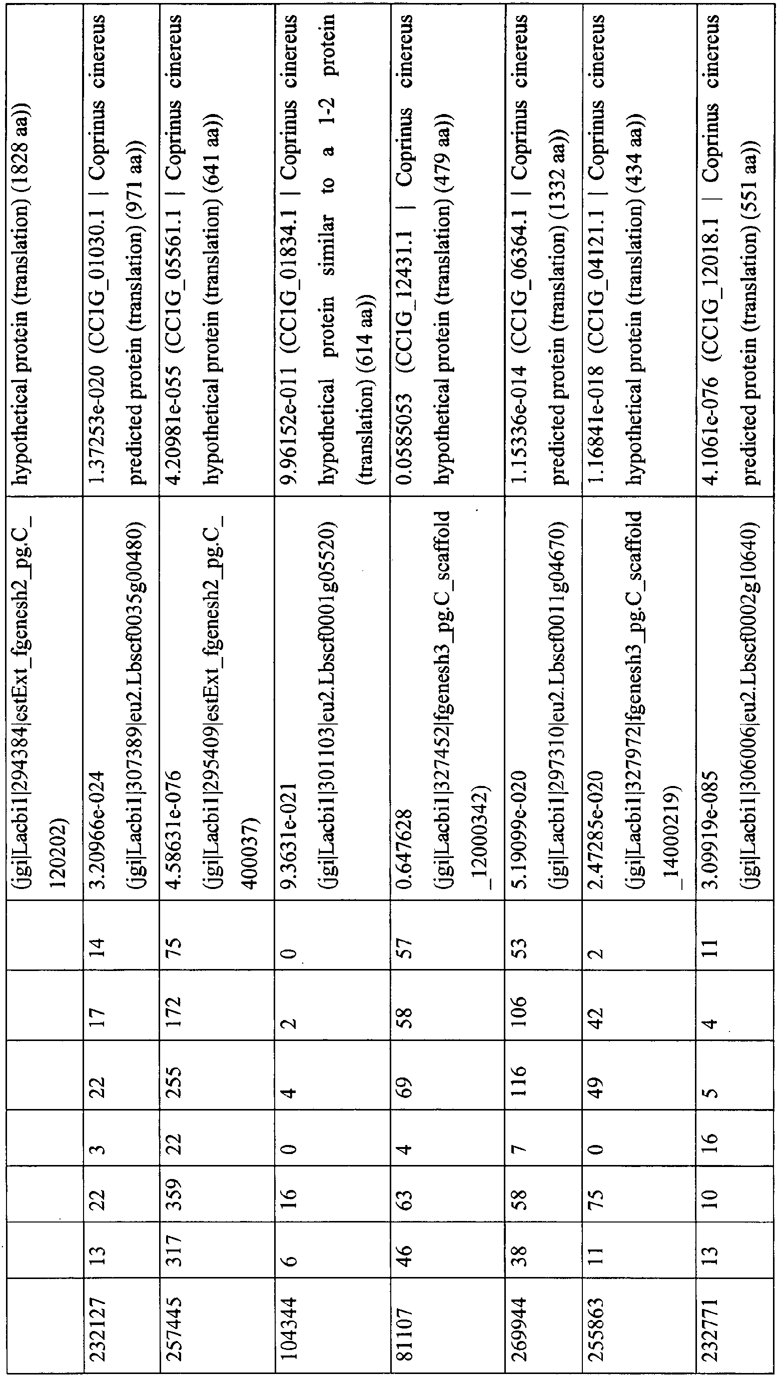

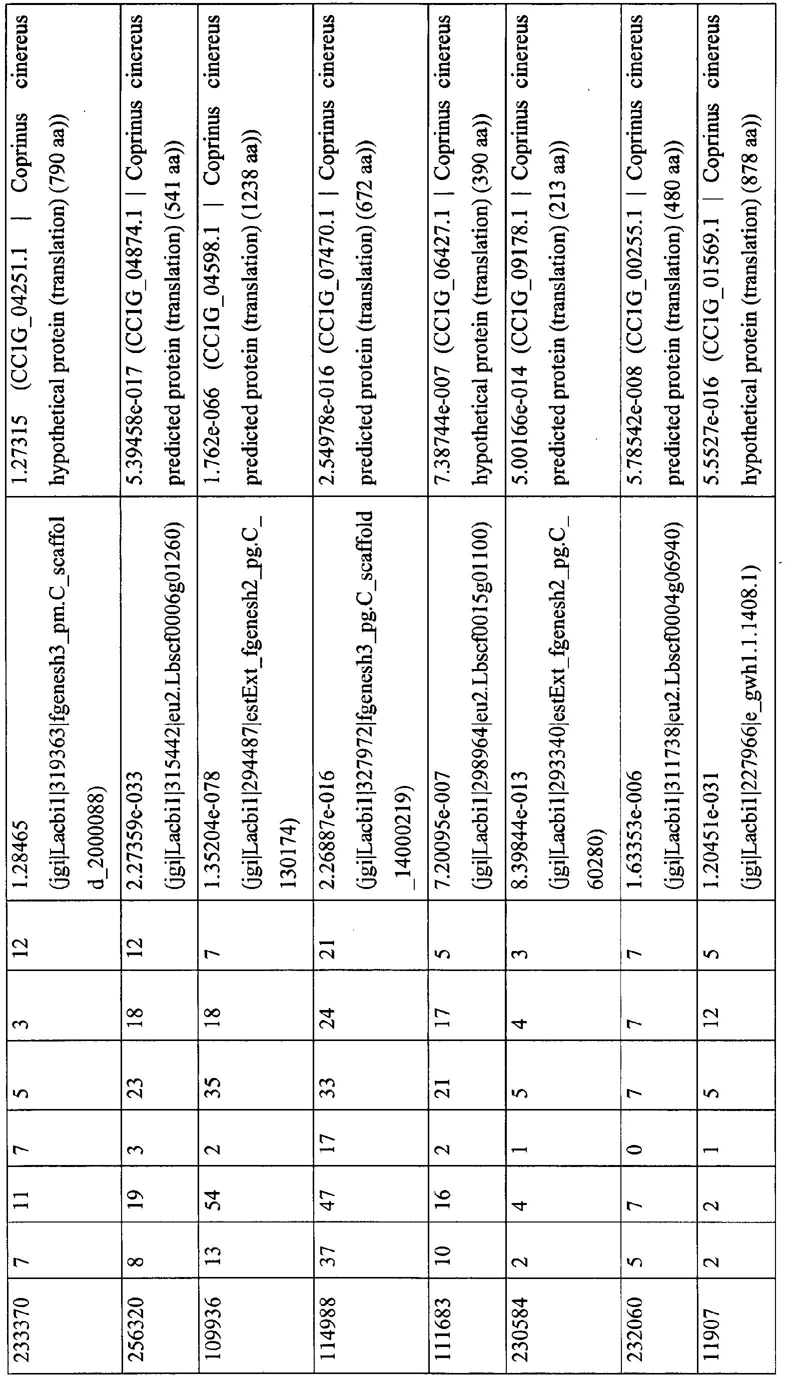

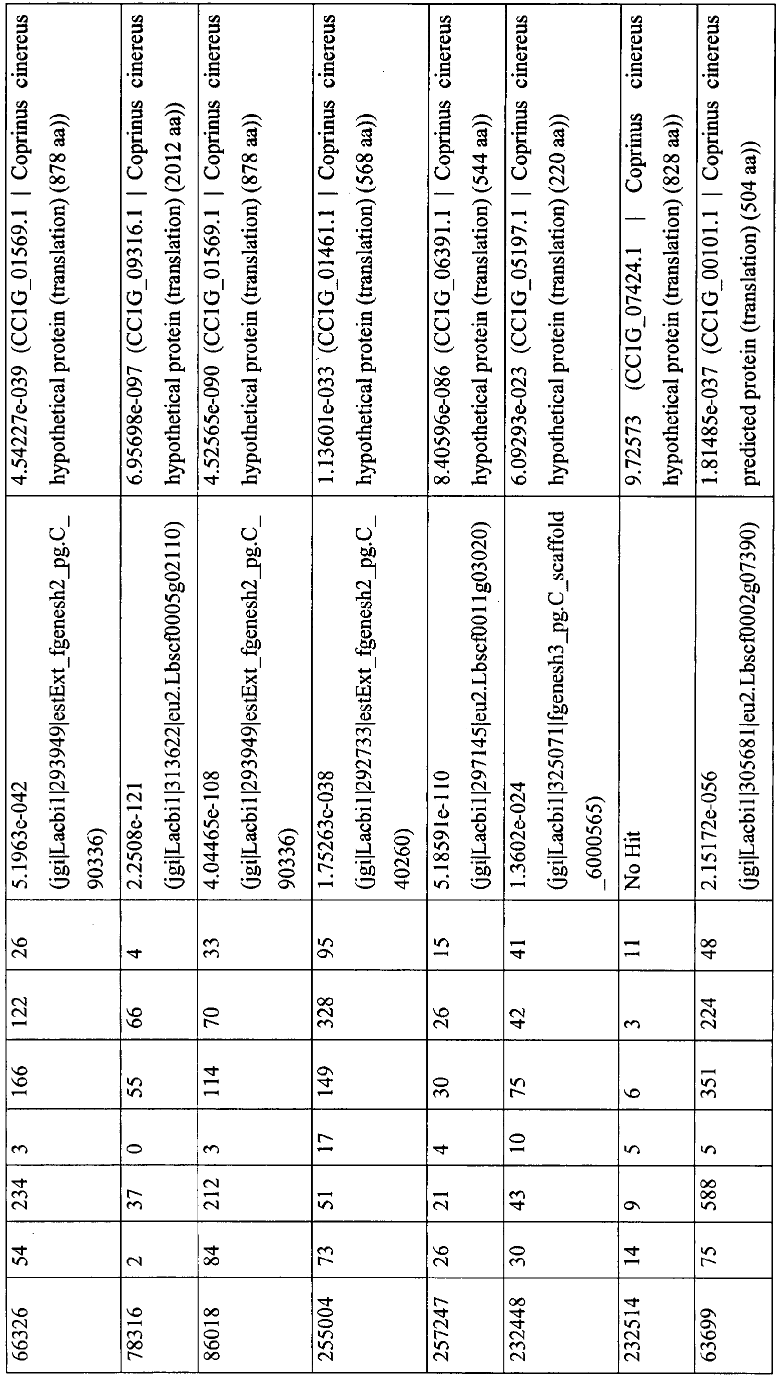

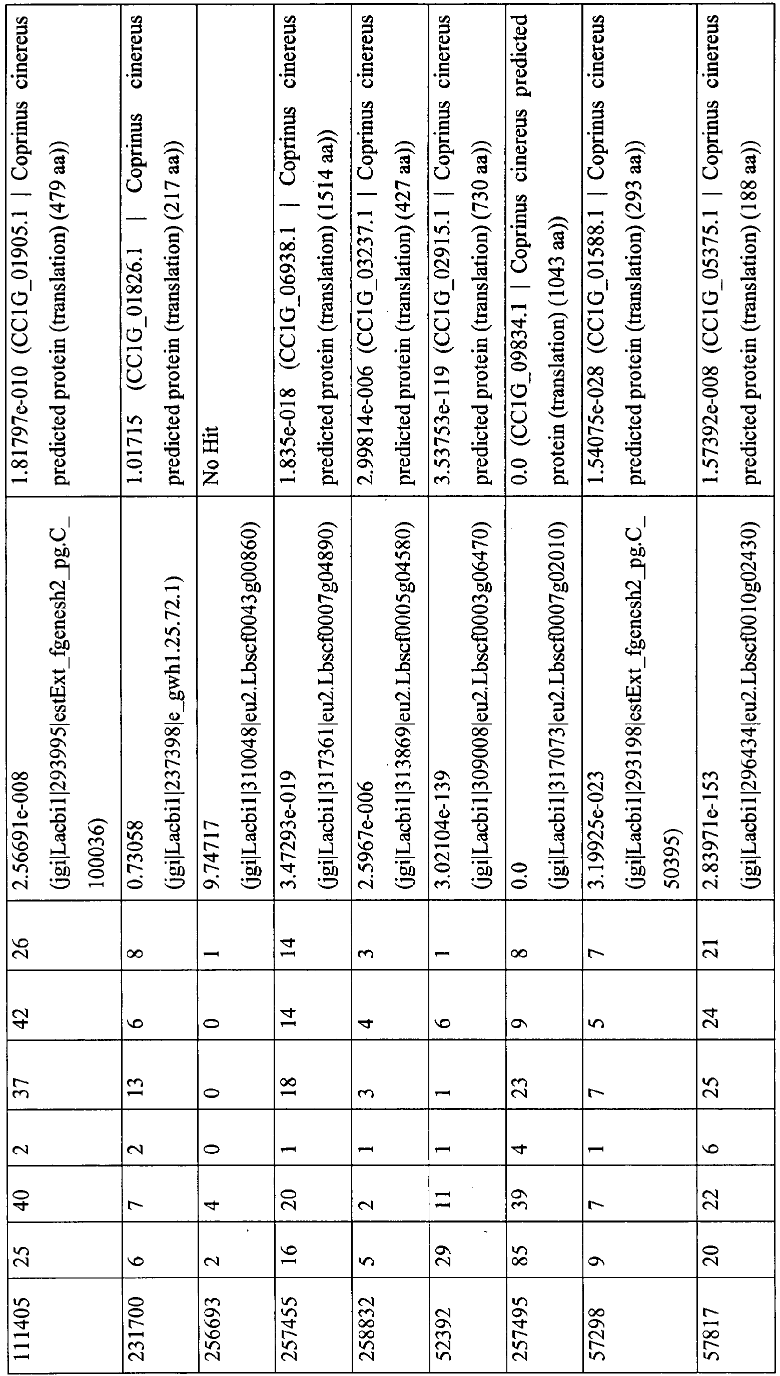

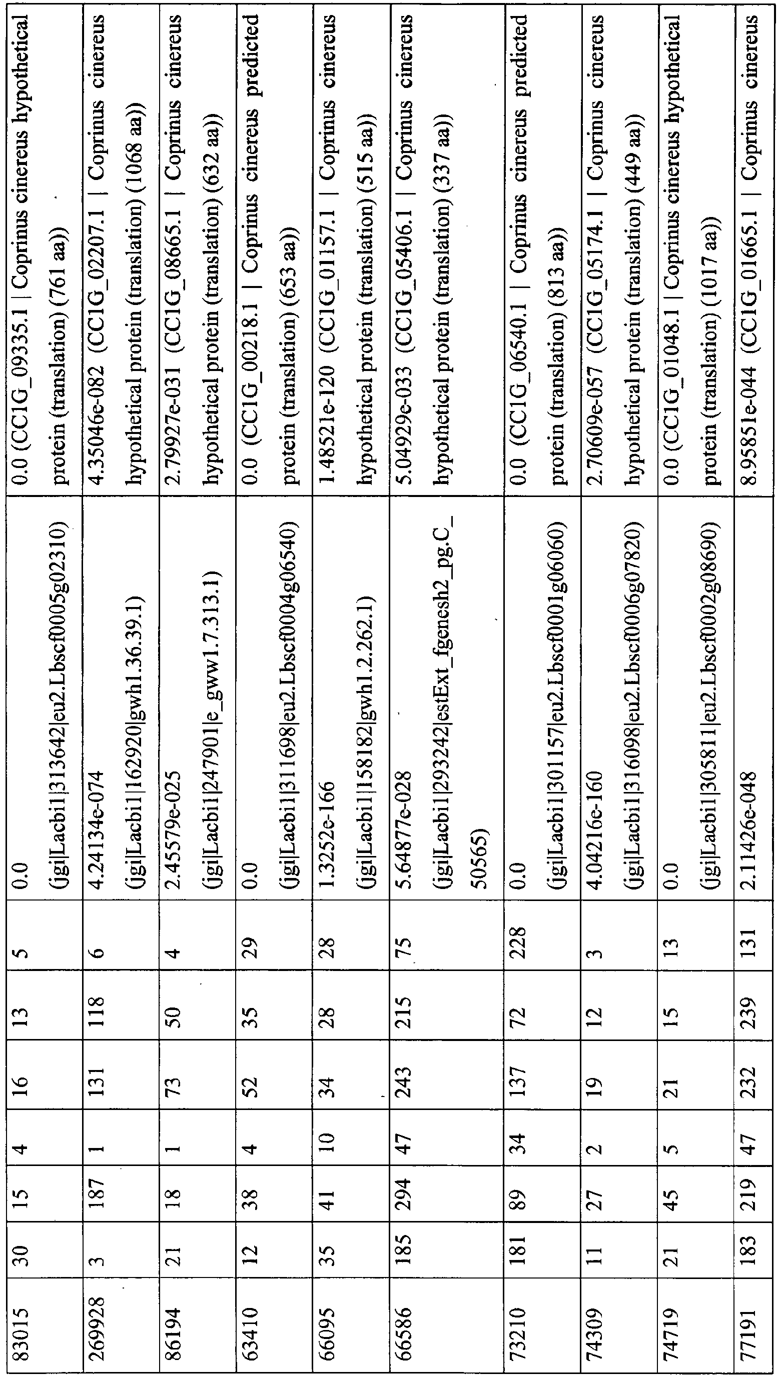

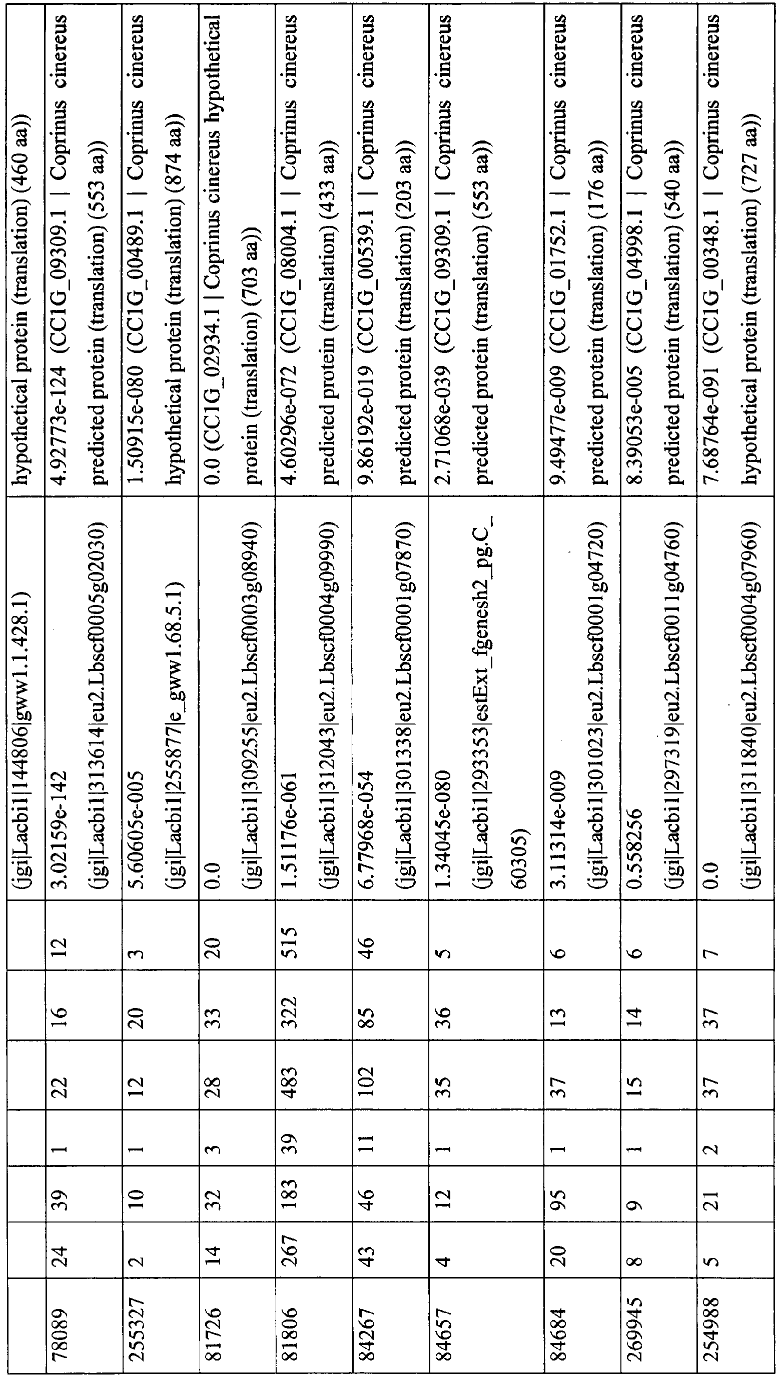

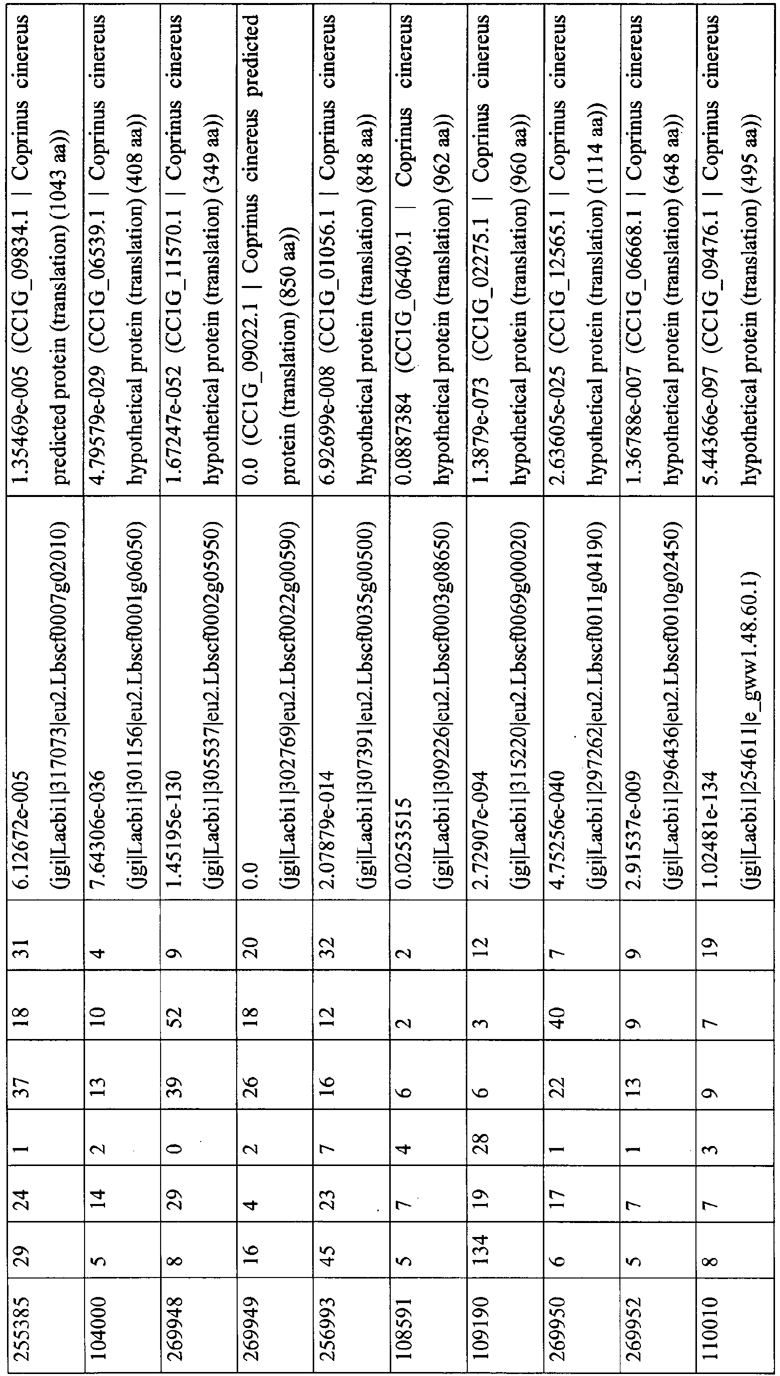

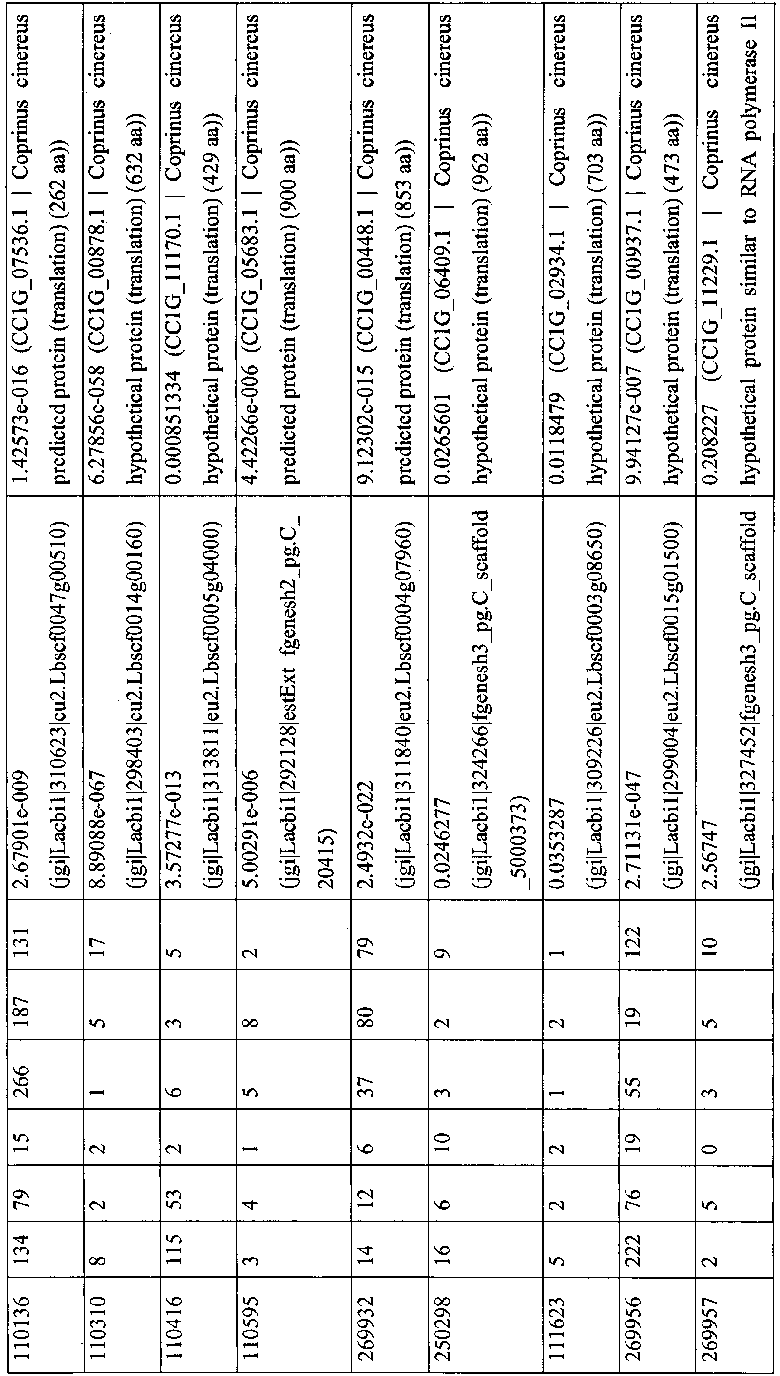

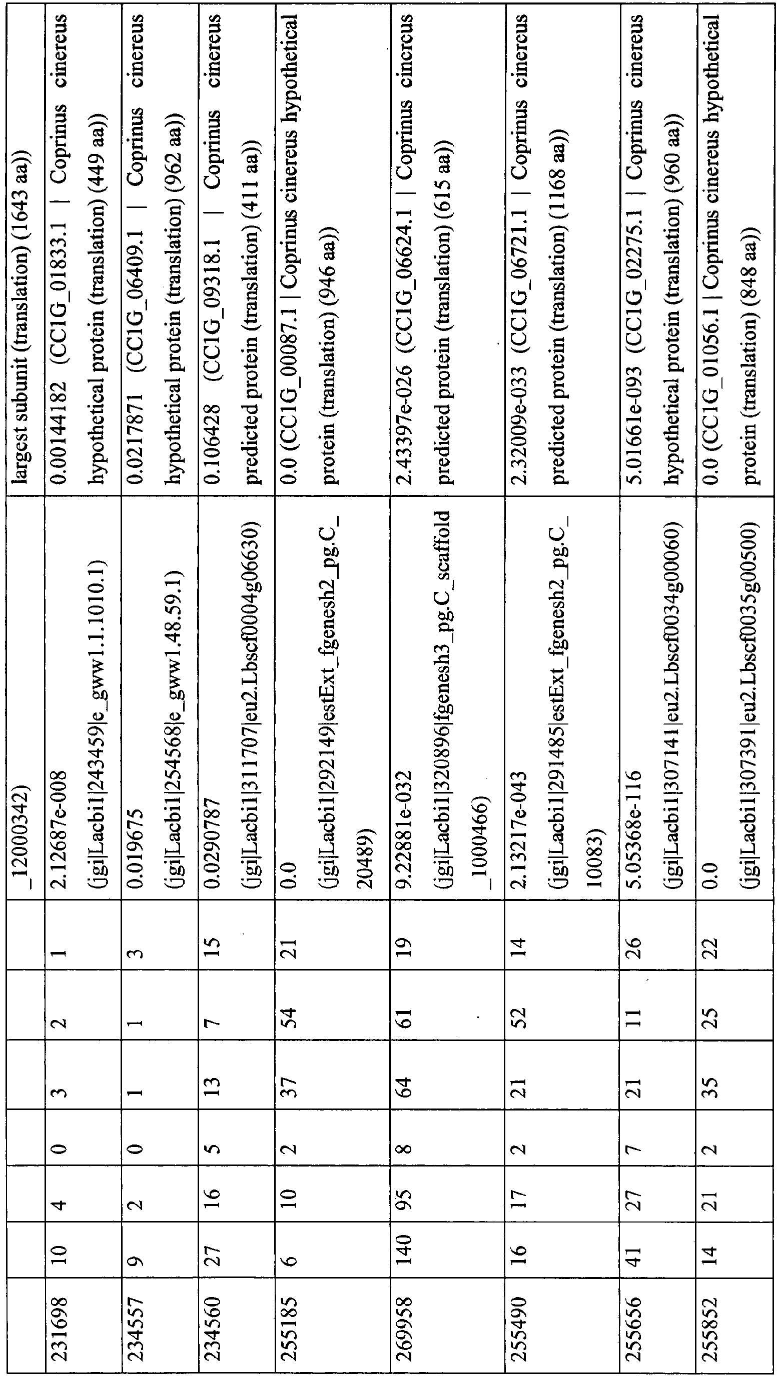

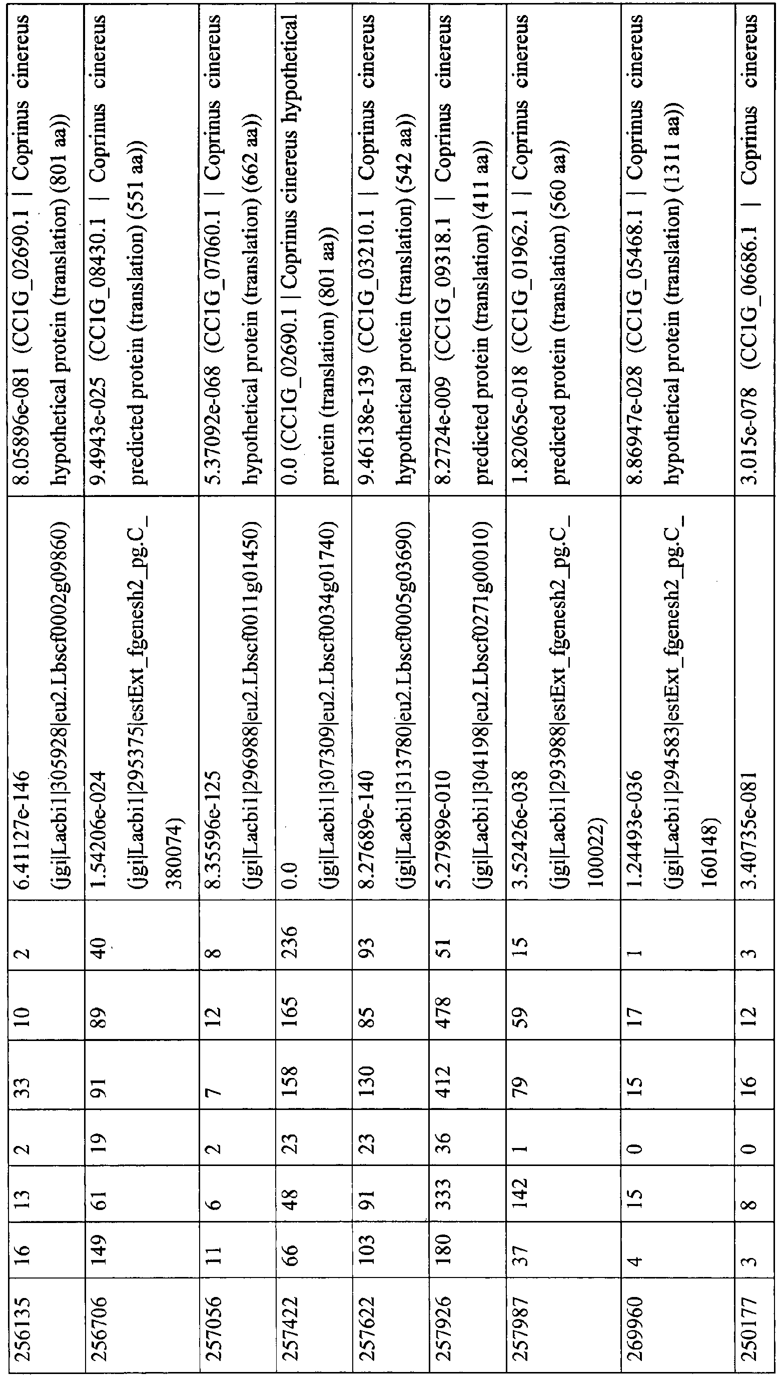

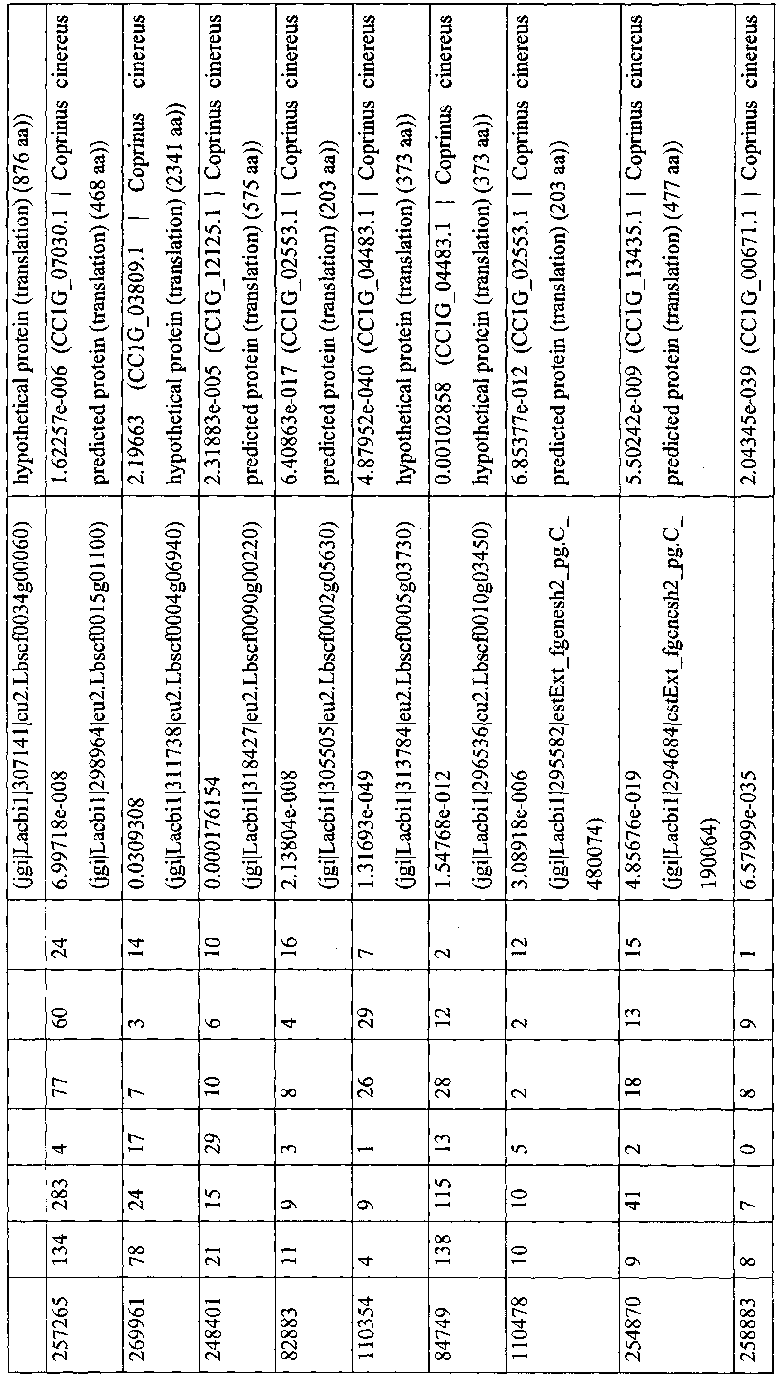

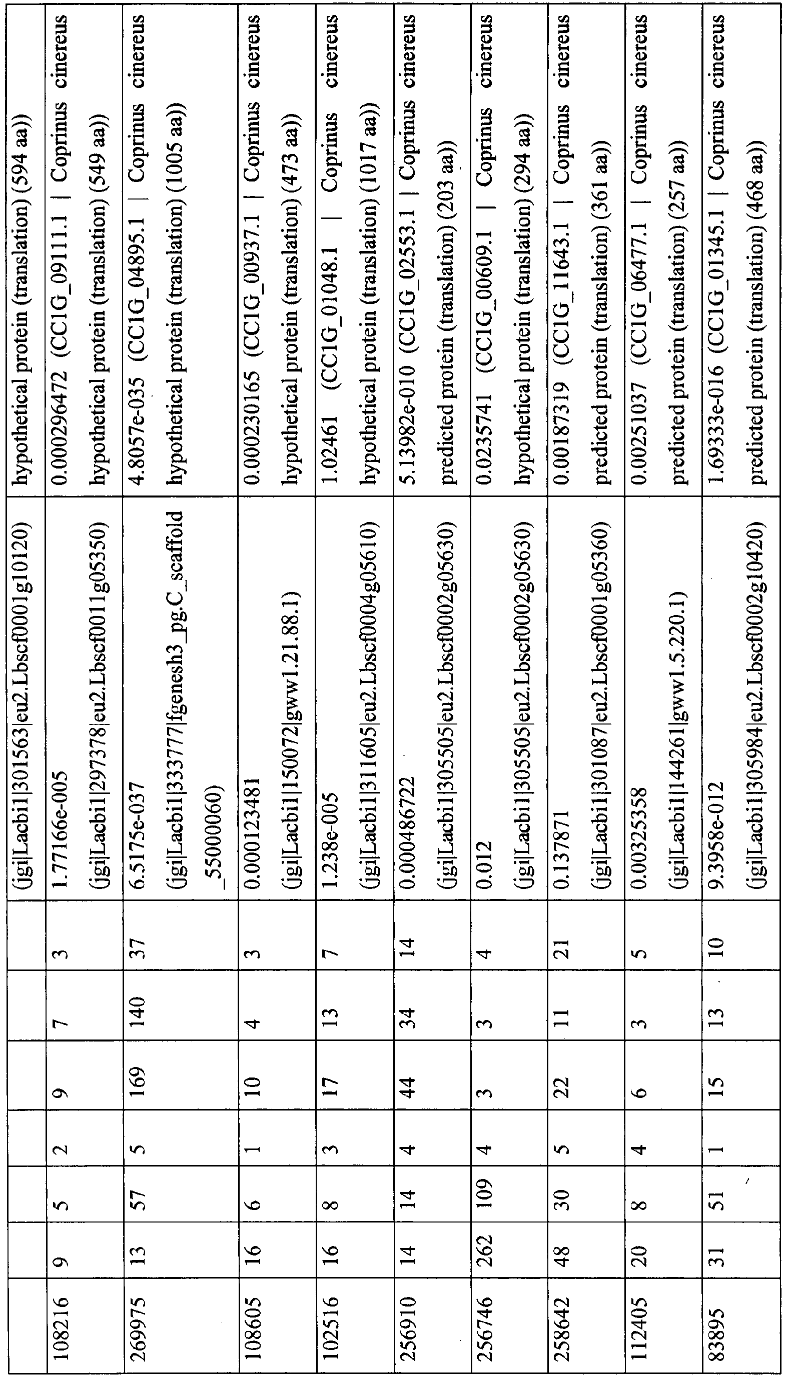

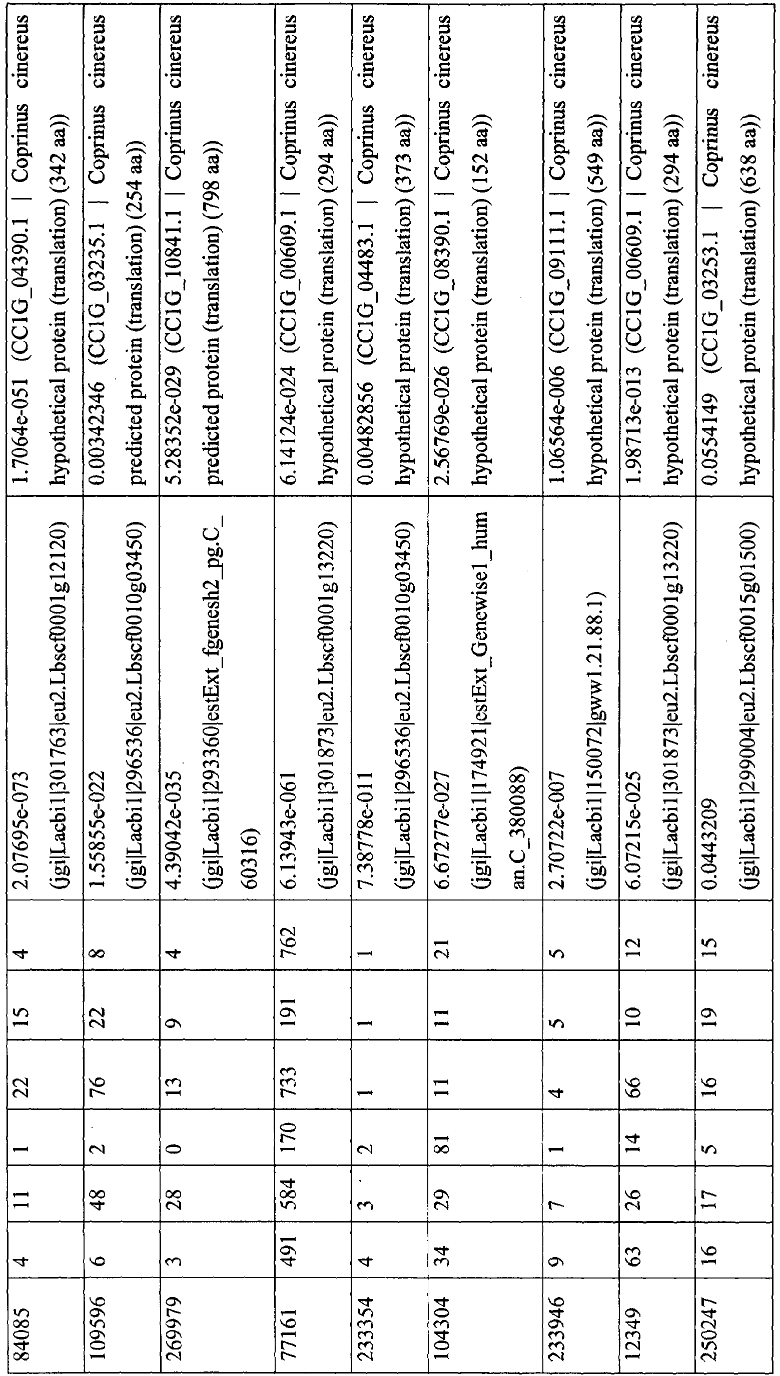

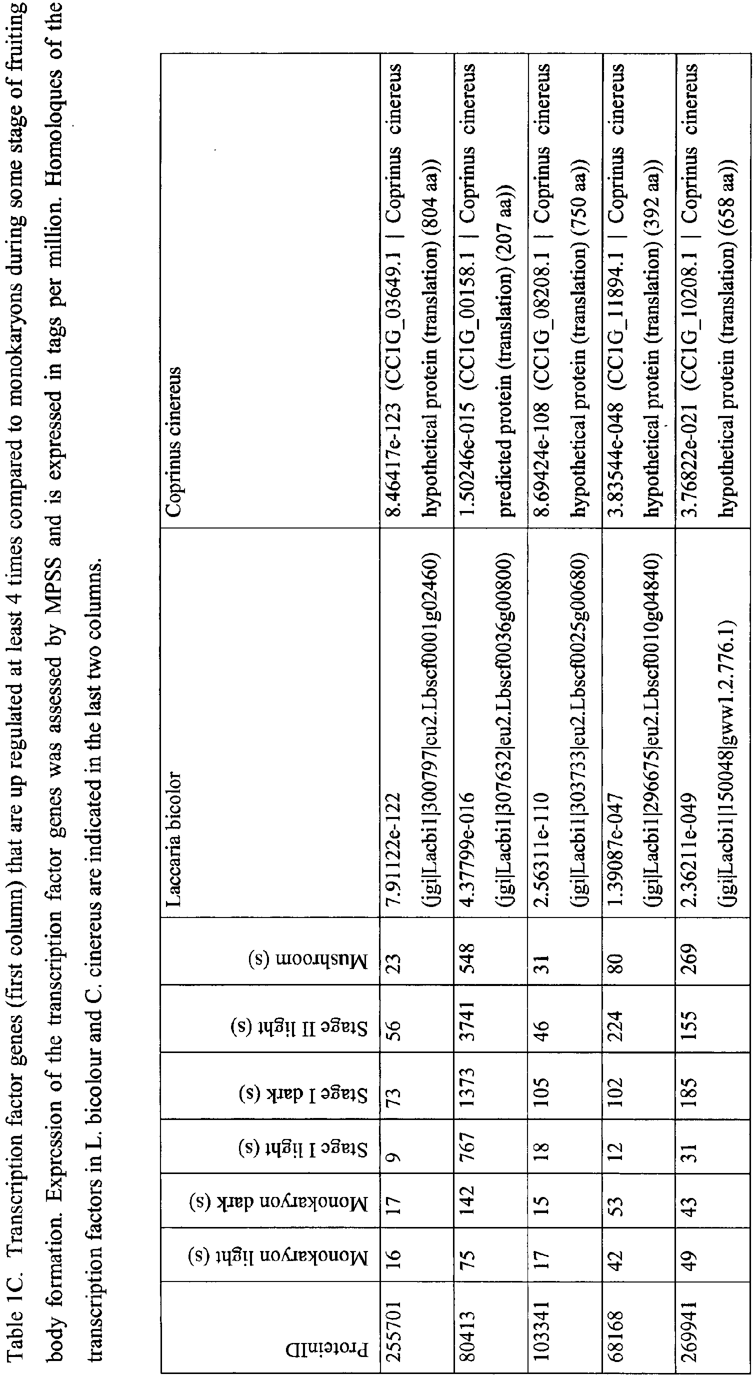

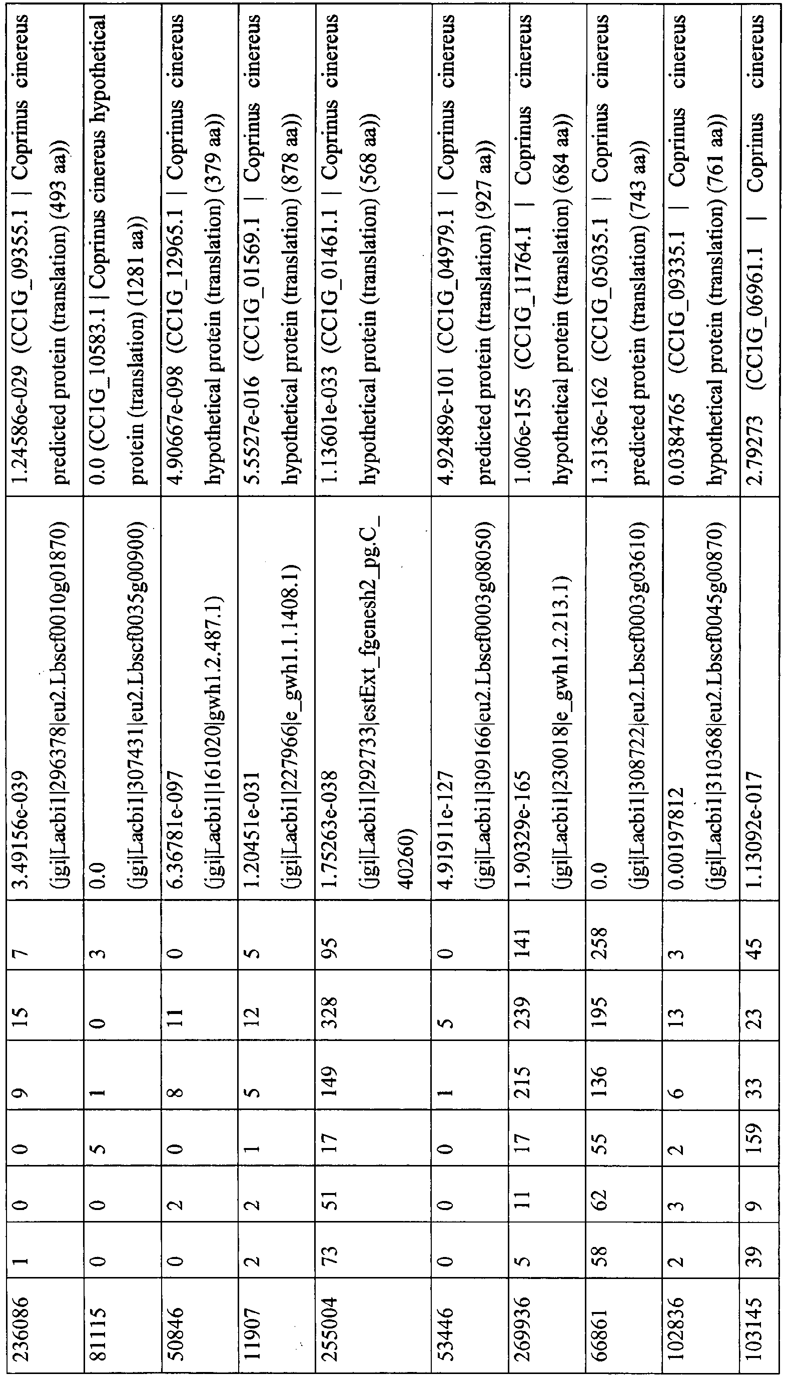

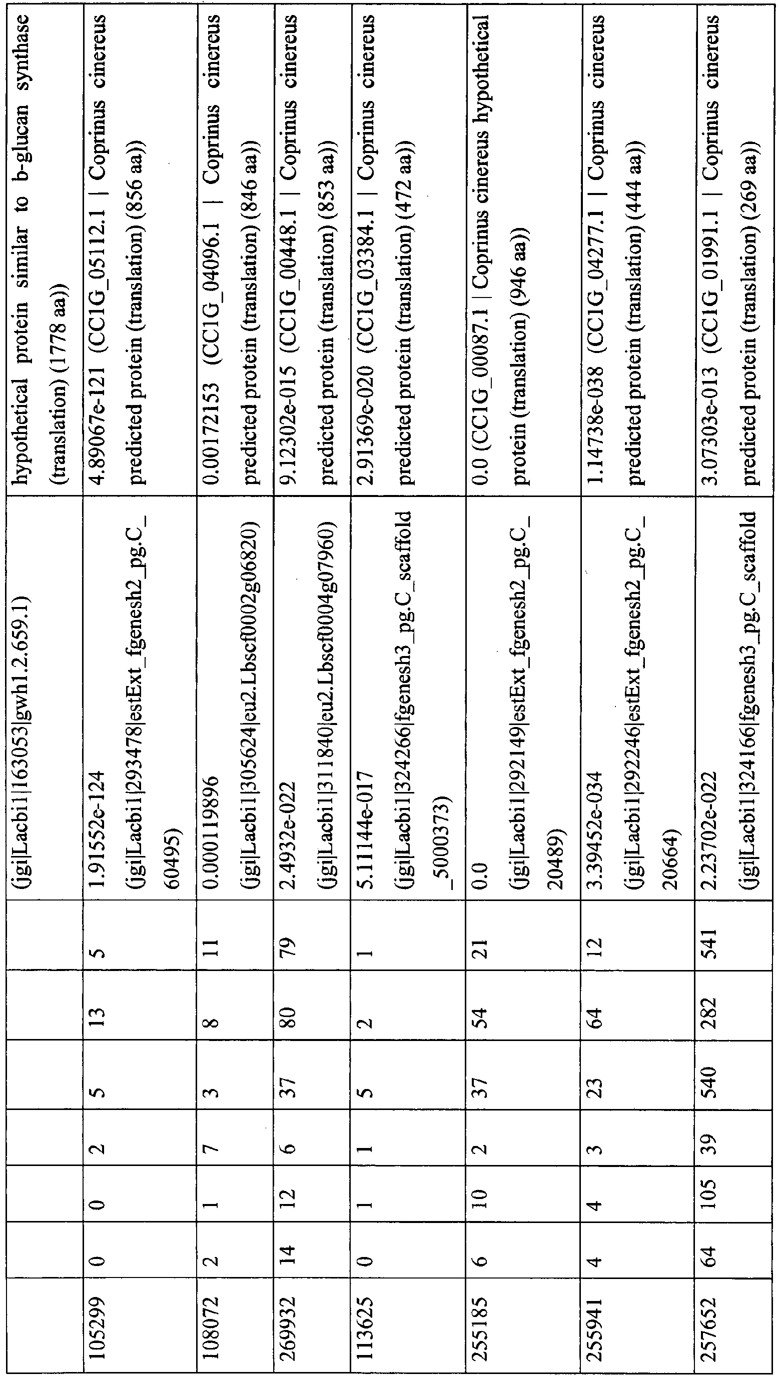

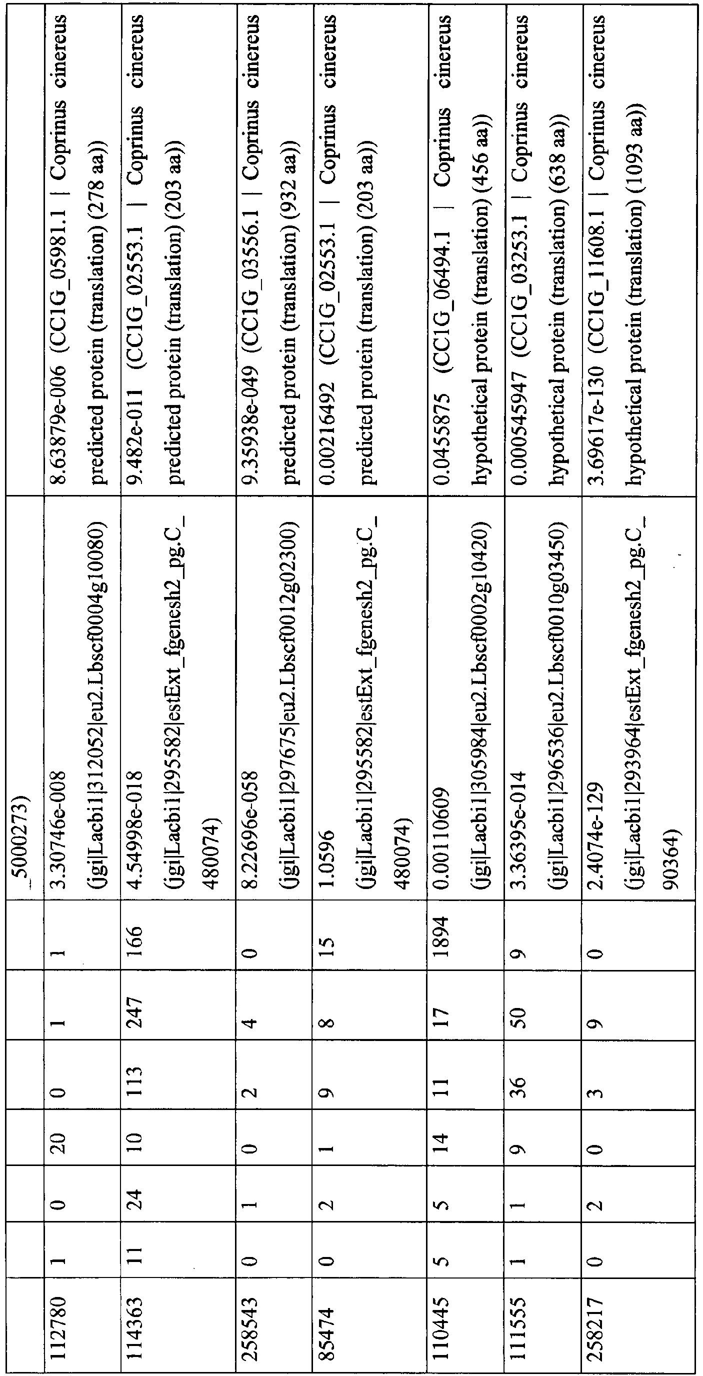

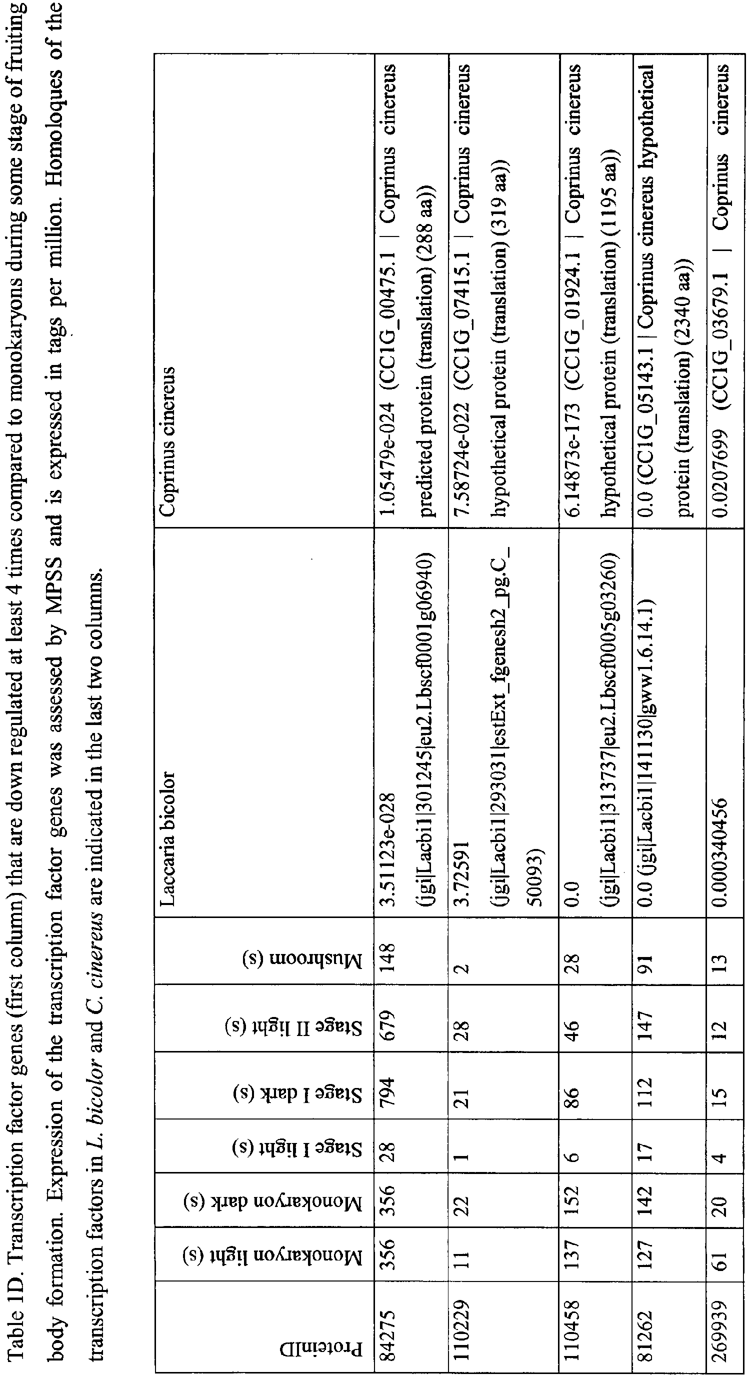

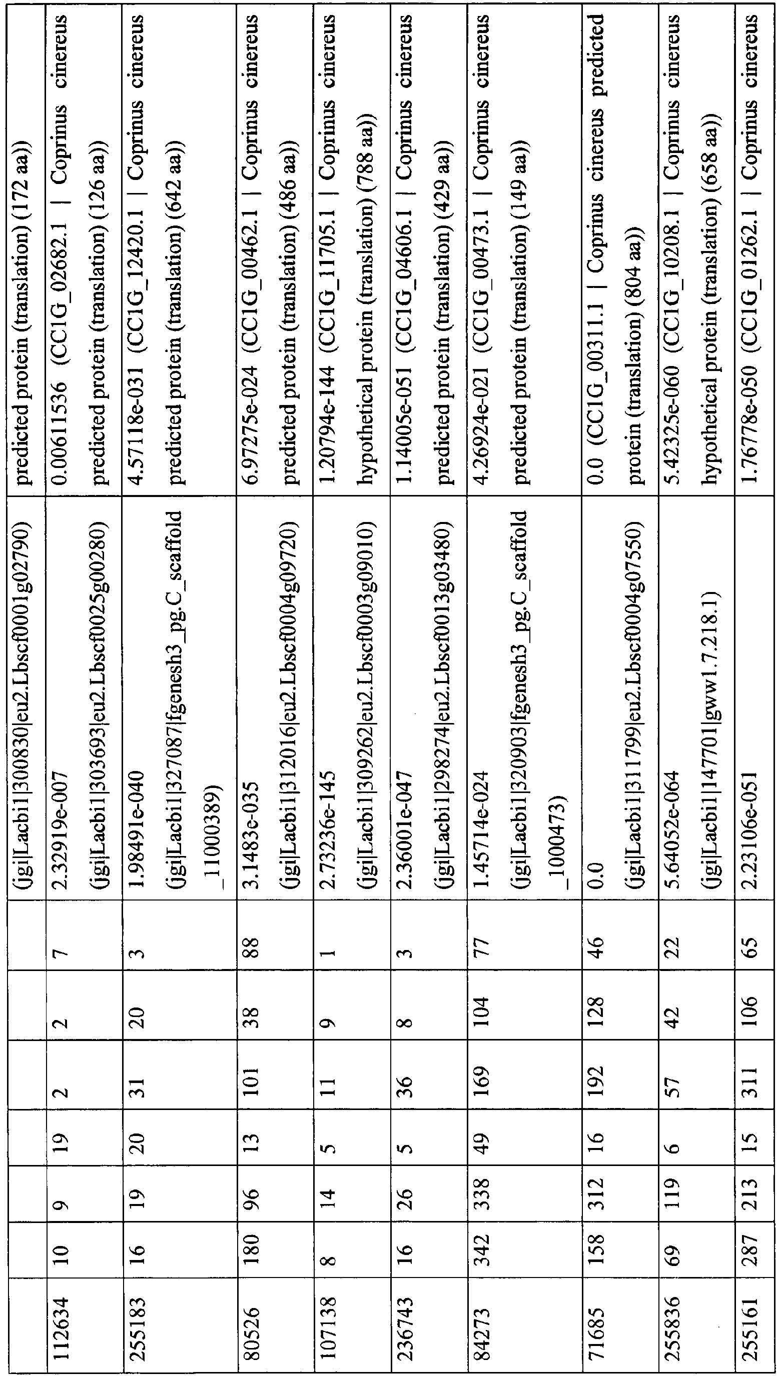

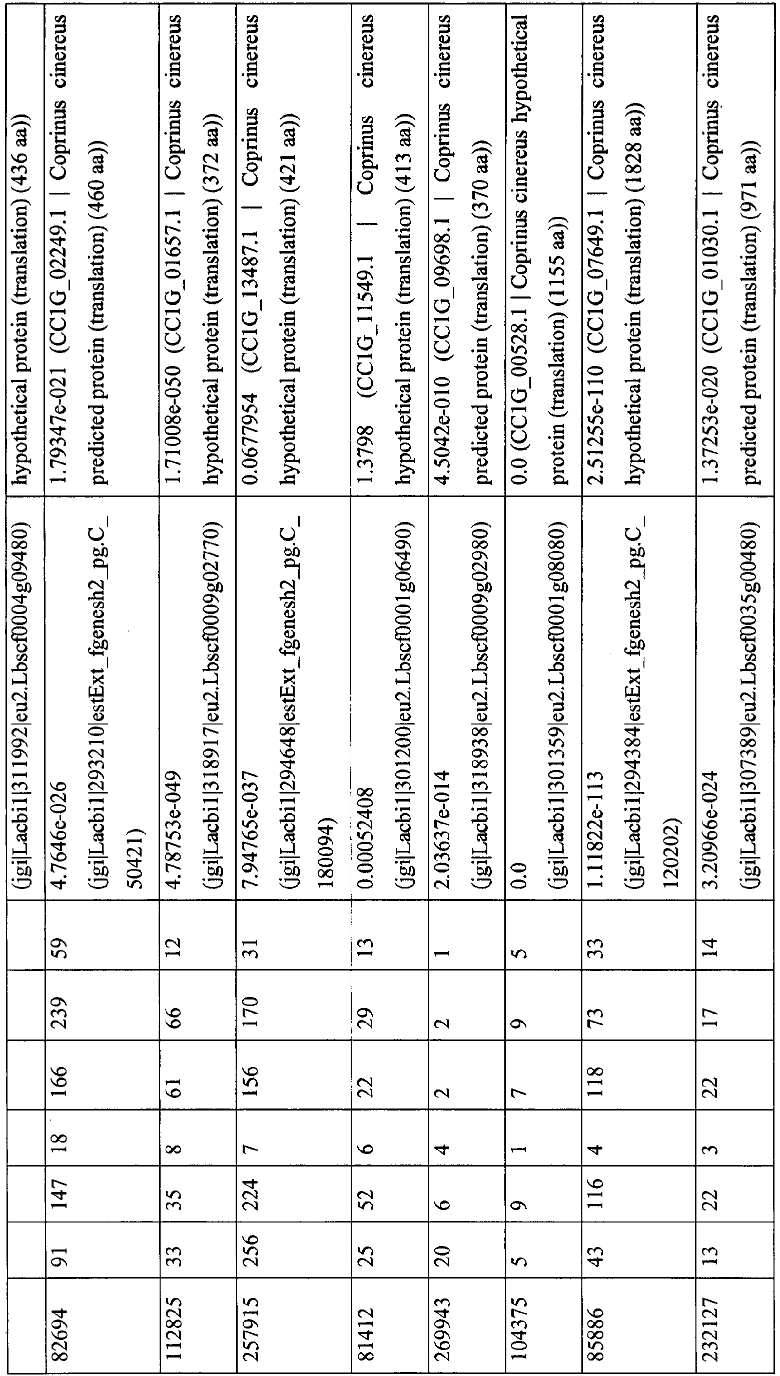

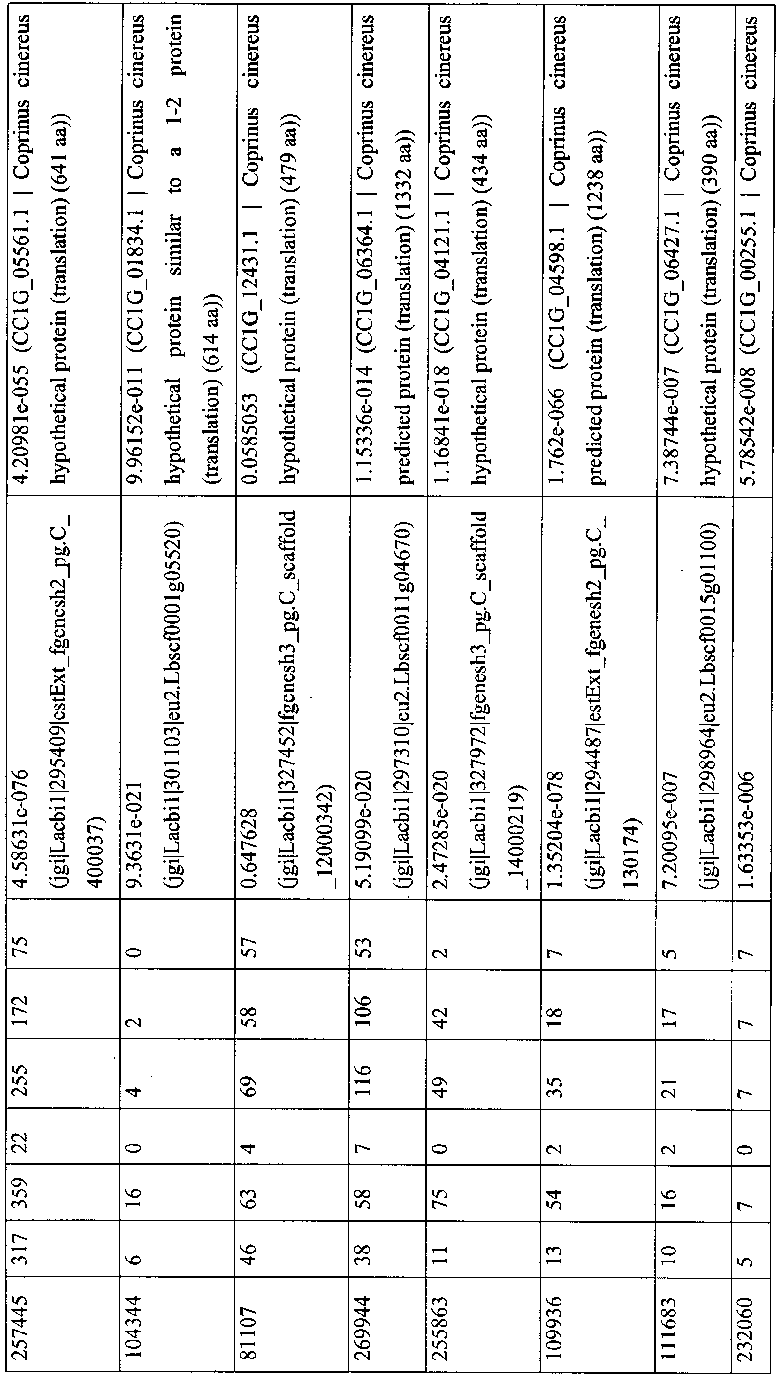

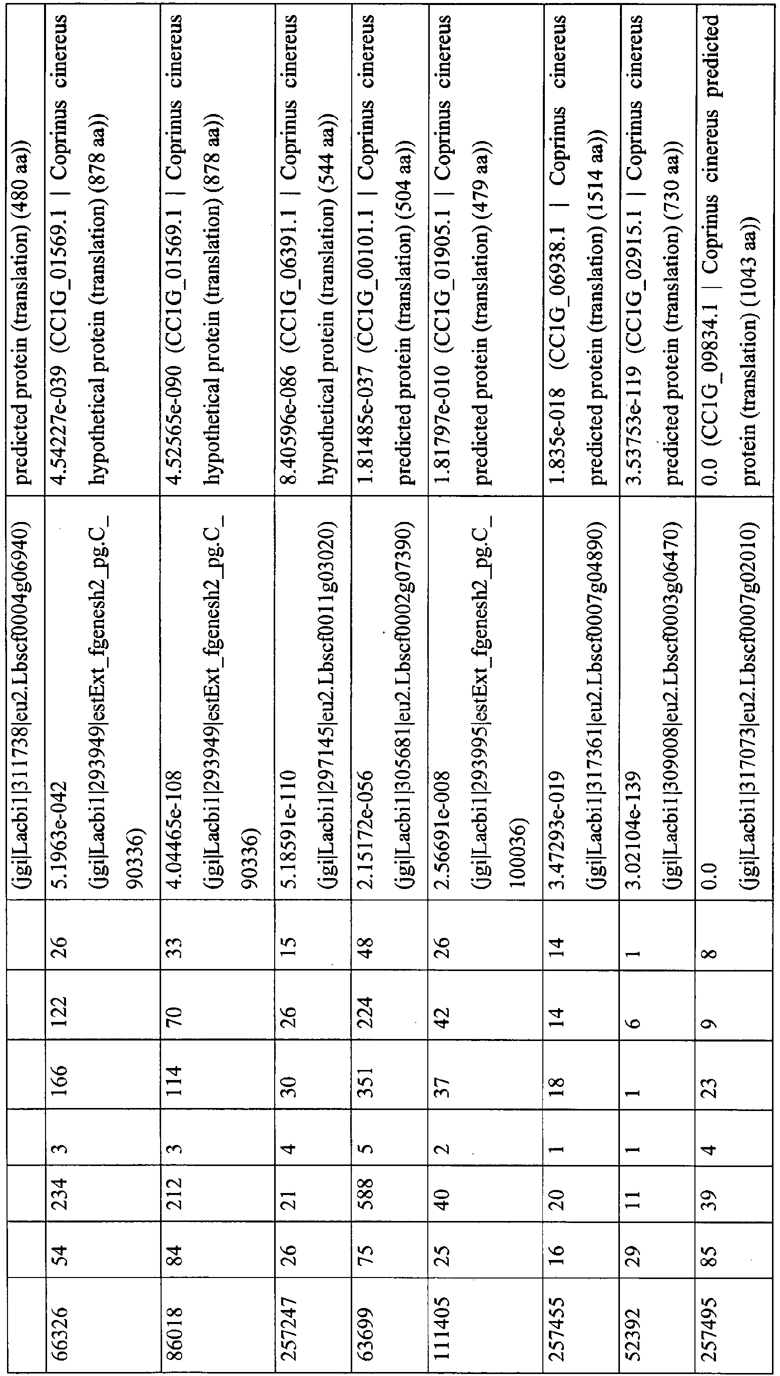

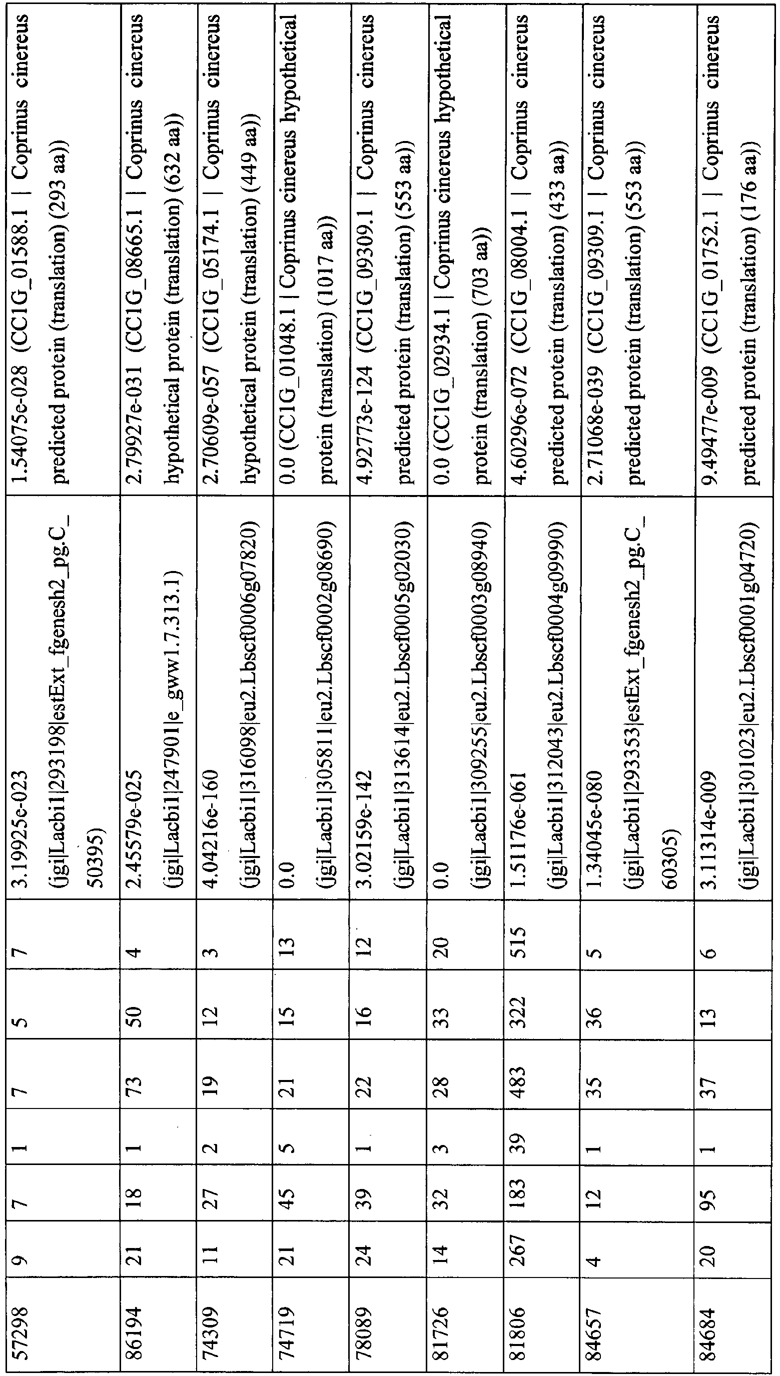

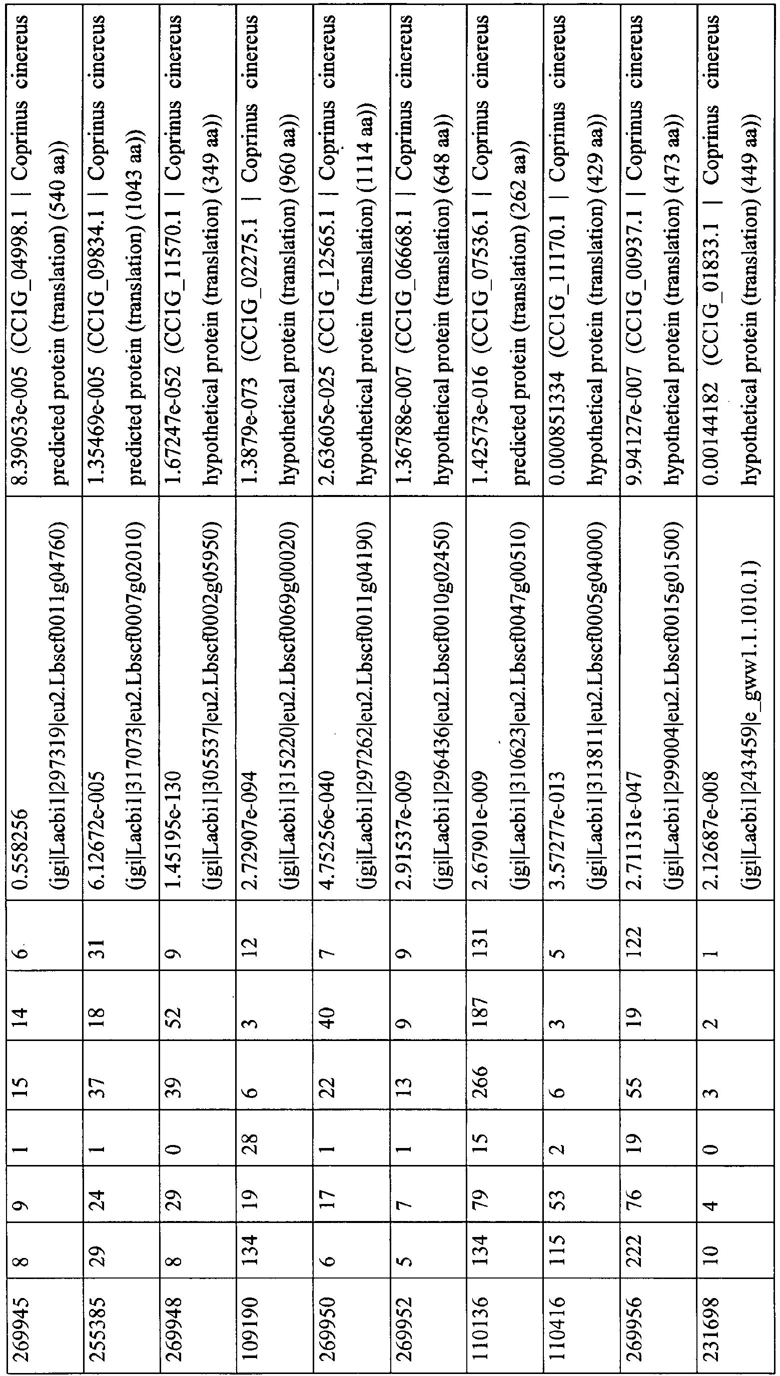

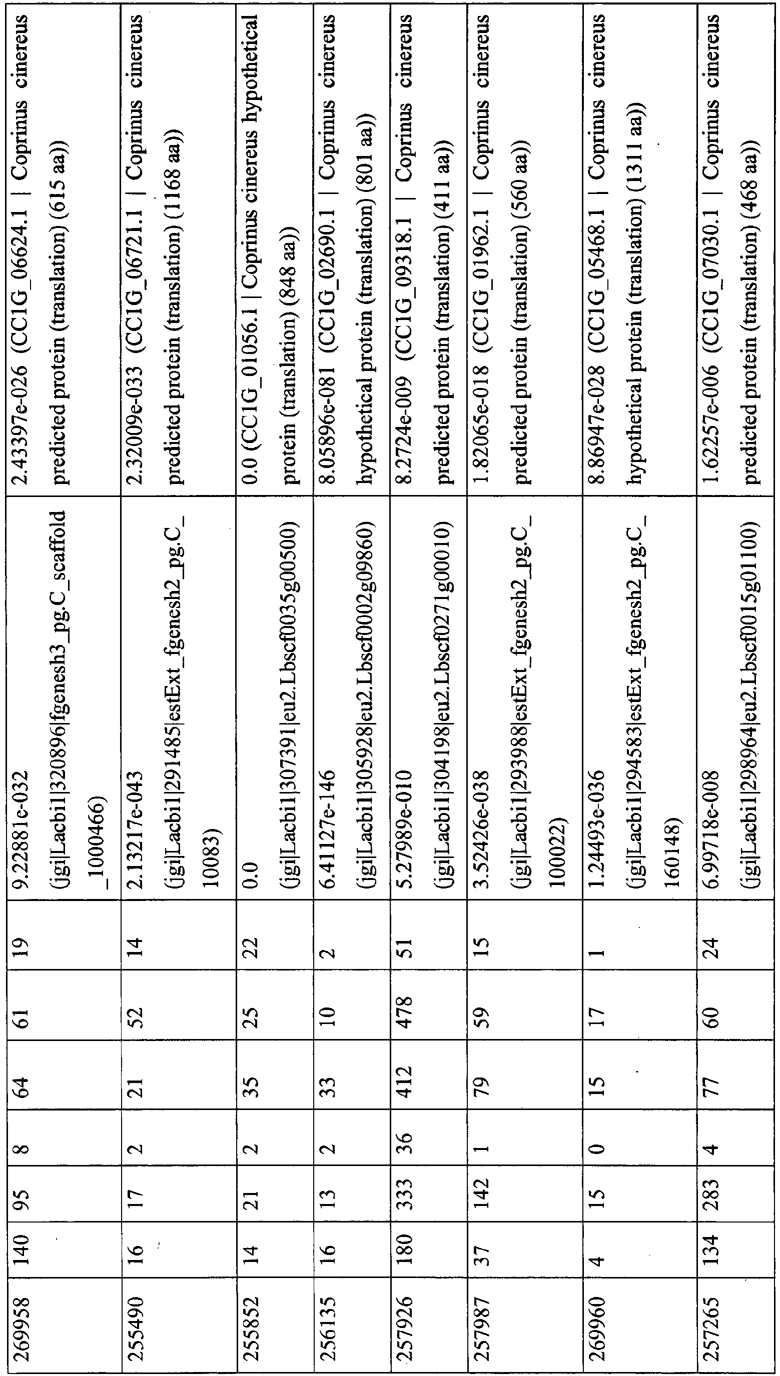

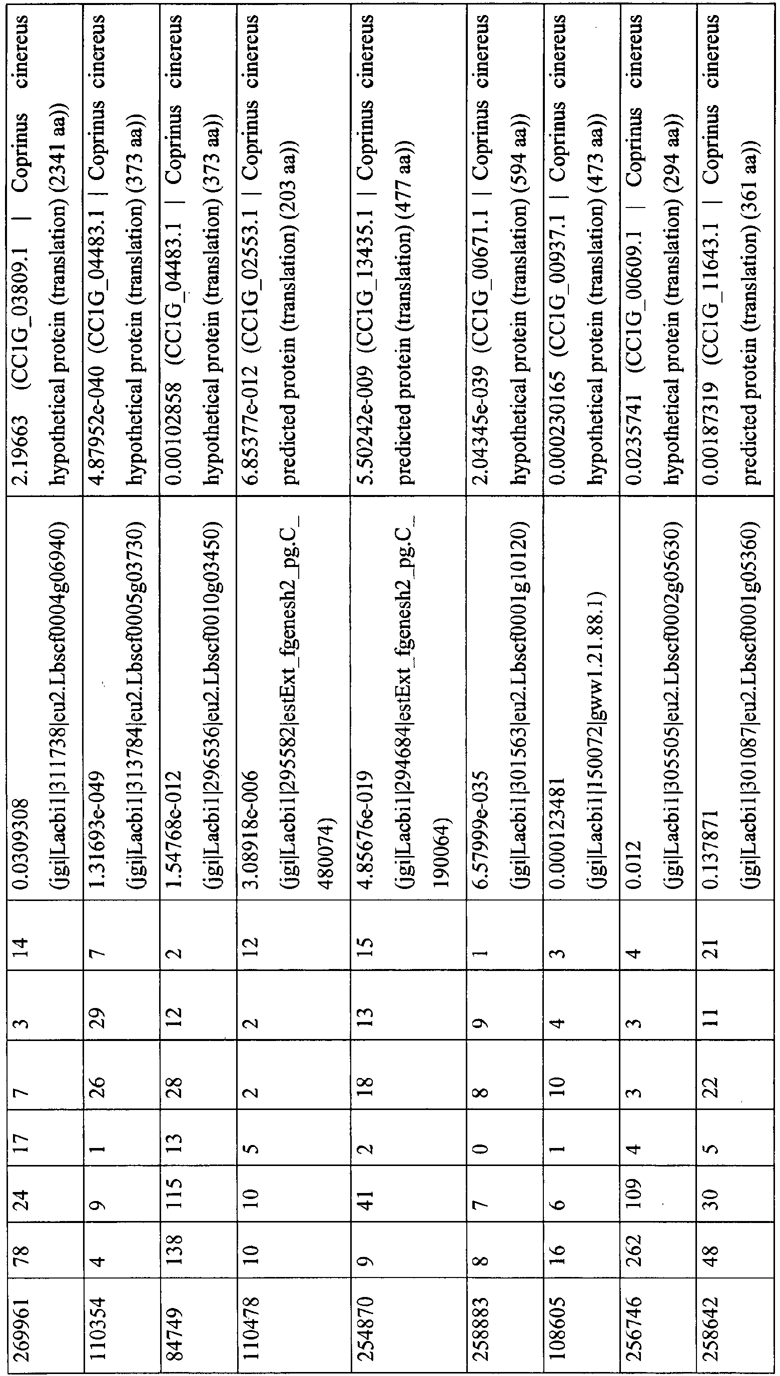

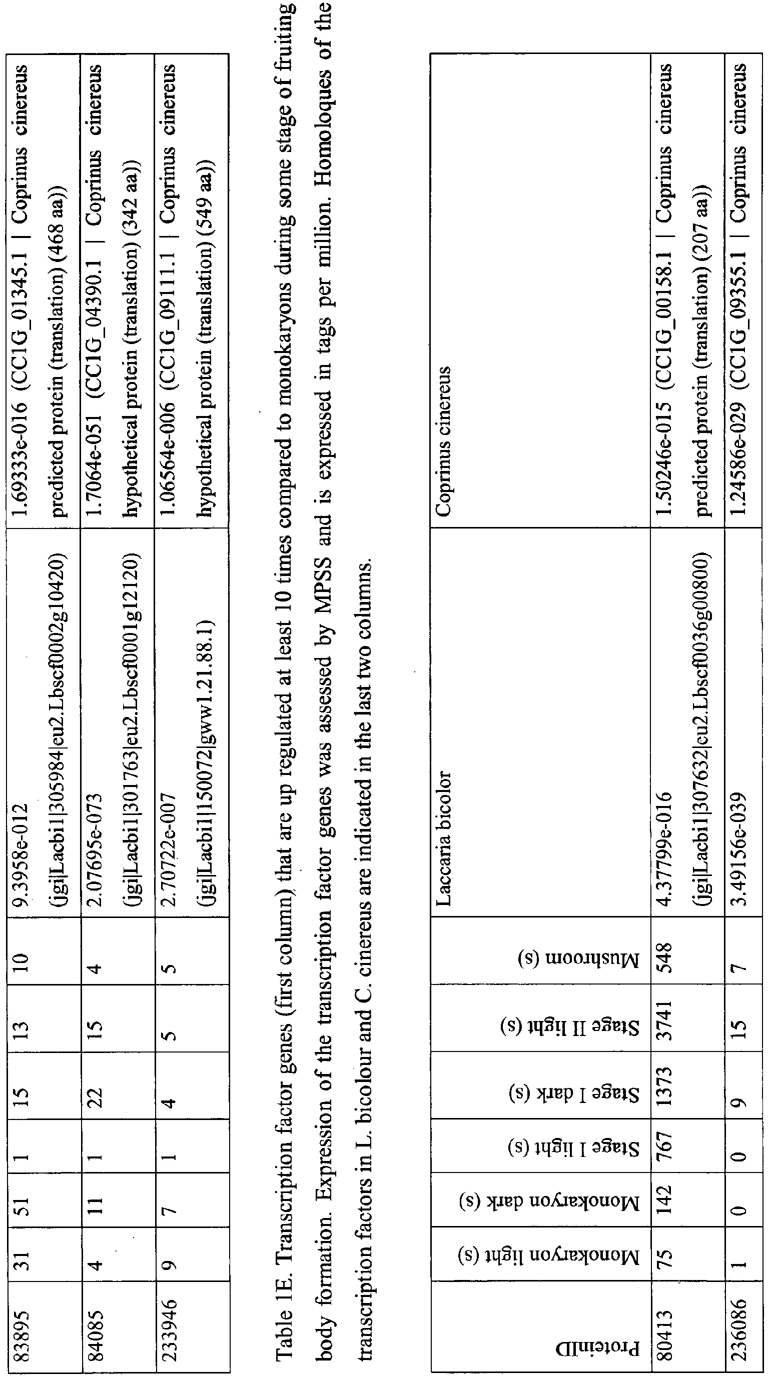

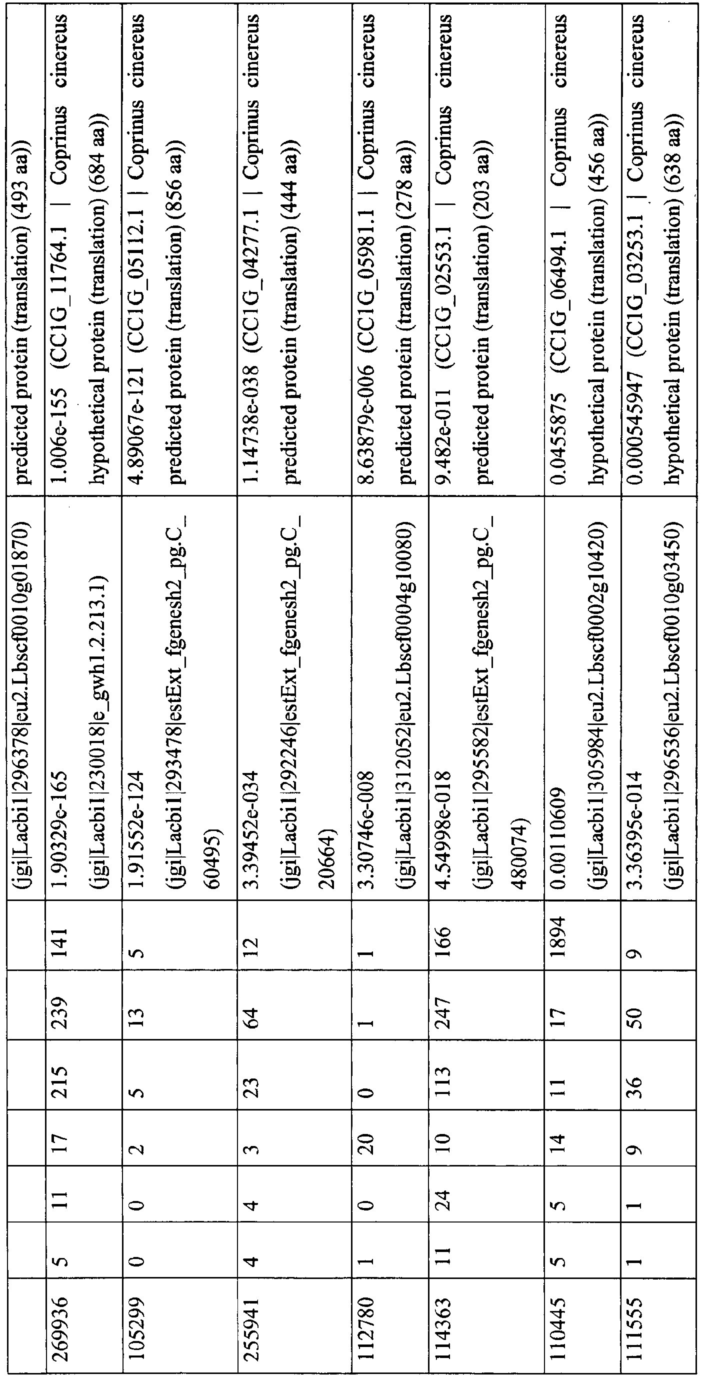

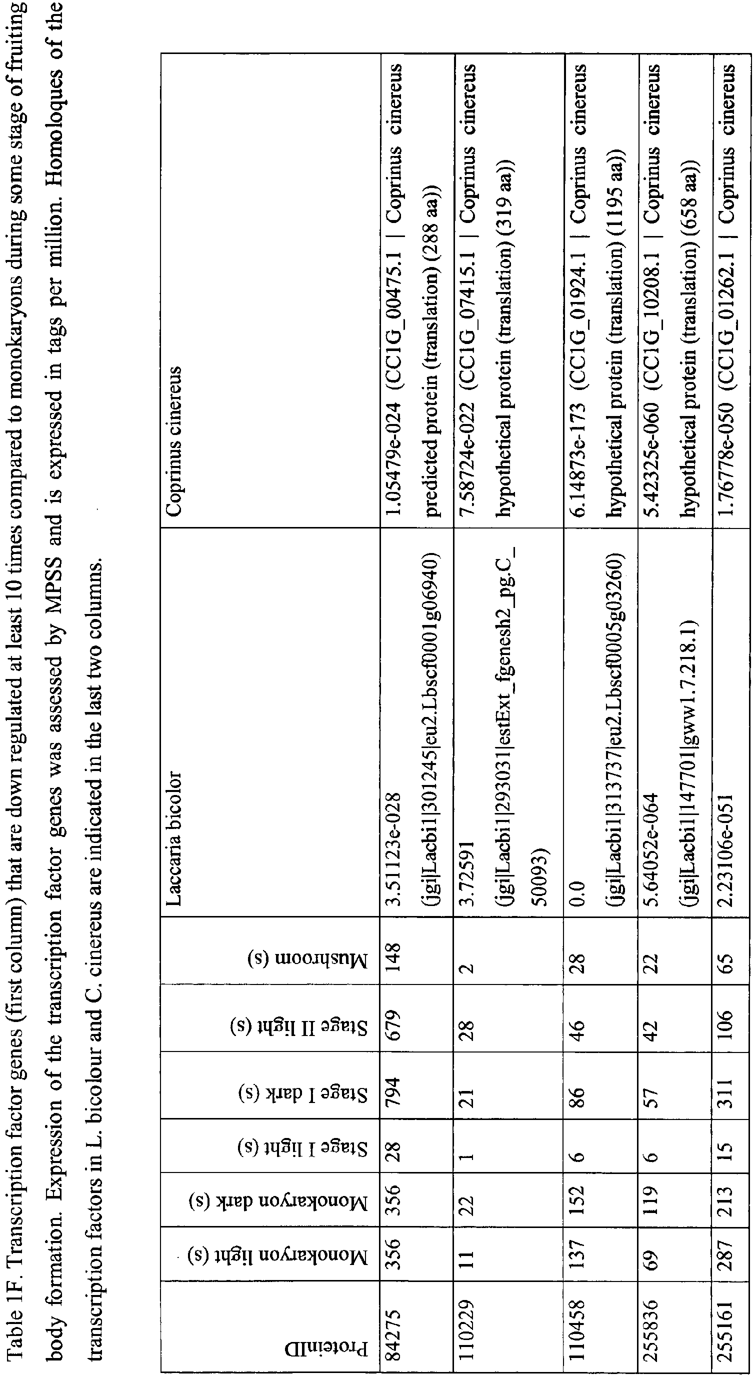

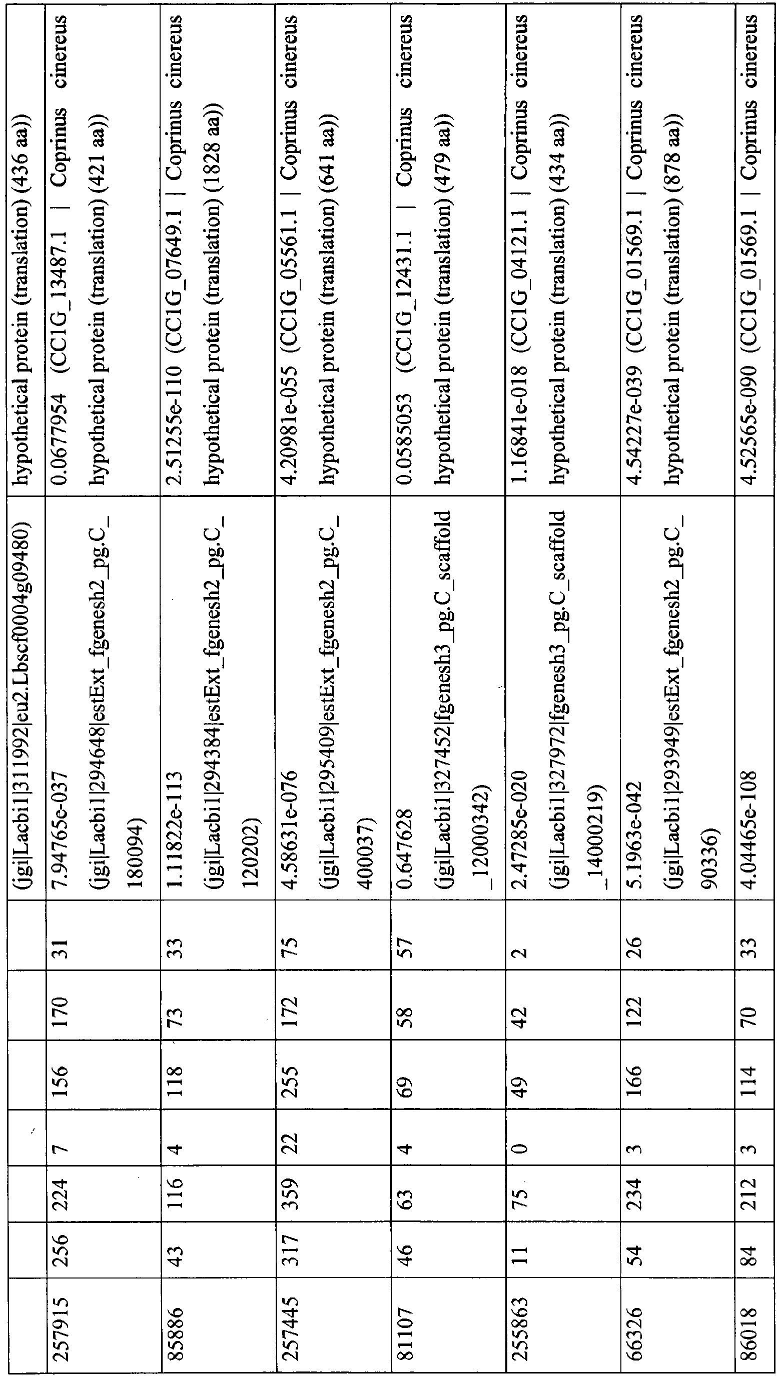

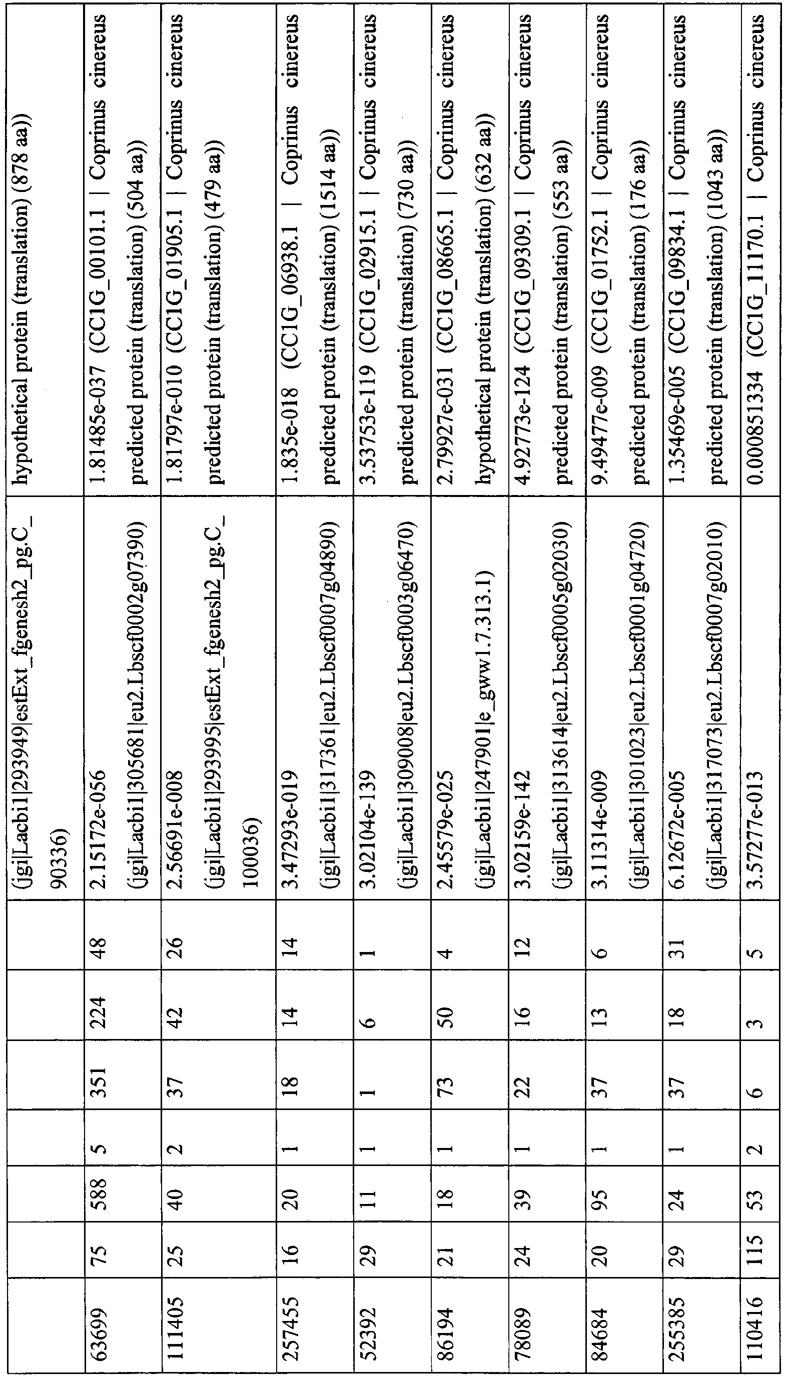

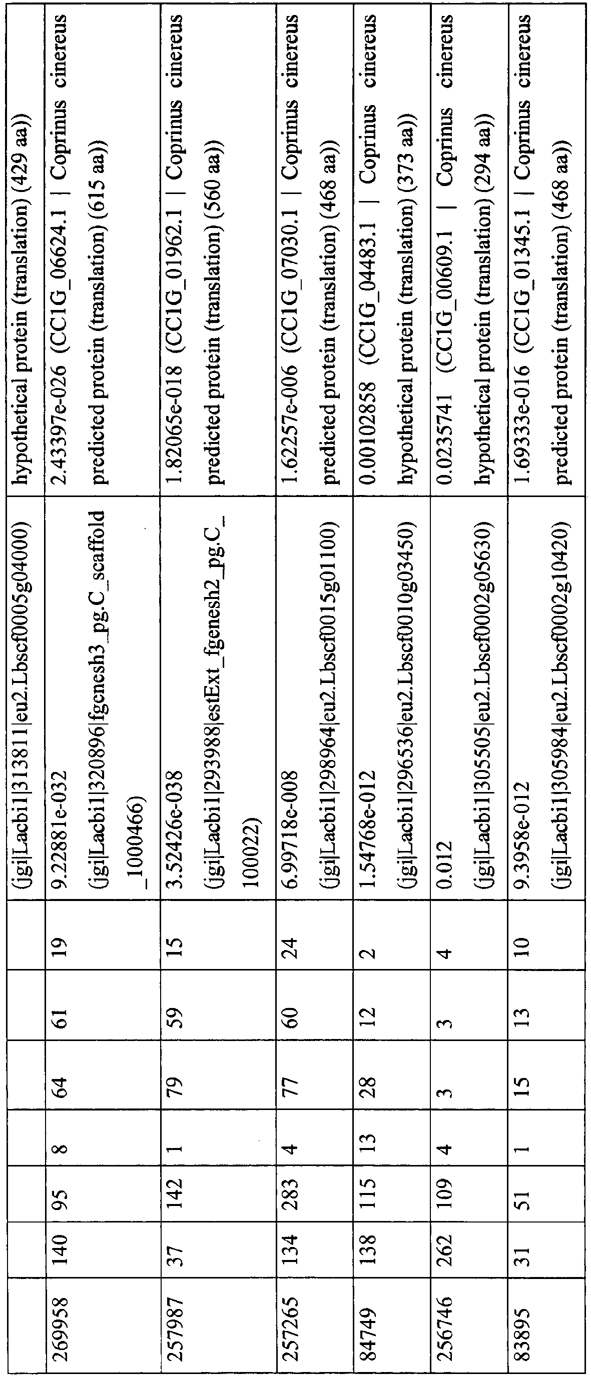

### Conservation of POLYPEPTIDEs in the fungal kingdom

In order to determine the level of conservation of the TFs throughout the fungal kingdom, protein sequences were blasted against the protein databases of *Laccaria bicolor, Coprinus cinereus, Phanerochaete chrysosporium, Cryptococcus neoformans* (H99), *Cryptococcus neoformans* (JEC21), *Ustilago maydis, Aspergillus niger* (DSM sequence), *Aspergillus niger* (Broad Institute), *Magnaporthe grisea* and *Neurospora crassa.*
The expect value and the name of the highest hit with the protein databases of the mushroom forming fungi *Coprinus cinereus* and *Laccaria bicolor* are indicated in Table 1.

### Example 2: Proof of principle: a knockout of fst3 (proteinID 257422) affects mushroom development

A knock-out was made of the putative transcription factor gene *fst3* (proteinID 257422). To this end, vector pDelcas was used as described in Ohm et al. (2010). Primers that were used to create the knock out construct are indicated in Table 2. This knock out construct called pRO097 consists of the flanking regions of the coding sequence of *fst3* in between which the nourseothricin resistance cassette is situated. The phleomycin resistance cassette is present elsewhere in the construct (for details see Ohm et al., 2010). Transformation of *S*. *commune* strain H4-8 was done as described (van Peer et al., 2009). Regeneration medium contained no antibiotic, whereas selection plates contained 20 µg ml⁻¹ nourseothricin. Deletion of the target gene was confirmed by PCR (for procedure see Ohm et al., 2010). Compatible monokaryons with a gene deletion were selected from spores originating from a cross of the mutant strains with wild-type strain 4-8.3 (L.G. Lugones, unpublished). Monokaryons with an inactivated *fst3* gene showed no phenotype when compared to the wild type. On the other hand, *Δfst3Δfst3* dikaryons showed clear differences in mushroom development compared to the wild-type. When grown from a point inoculum, mushrooms grew in the same location as in the wild type, but the number of mushrooms was increased and the size decreased (see Figure 1). When grown as a synchronized colony (by plating out homogenized mycelium), the number of mushrooms increased (data not shown). From these data we conclude that Fst3 inhibits formation of clusters of mushrooms. This regulation may be important in a natural environment to ensure sufficient energy is available for full fruiting body development.

### Example 3: Proof of principle: a knockout of fst4 (proteinID 66861) affects mushroom development

A knock-out was made of the putative transcription factor gene *fst4* (proteinID 66861). To this end, vector pDelcas was used as described in Ohm et al. (2010). Primers that were used to create the knock out construct are indicated in Table 2. The knock out construct called pRO191 consists of the flanking regions of the coding sequence of *fst4* in between which the nourseothricin resistance cassette is situated. The phleomycin resistance cassette is present elsewhere in the construct (for details see Ohm et al., 2010). Transformation of *S*. *commune* strain H4-8 was done as described (van Peer et al., 2009). Regeneration medium contained no antibiotic, whereas selection plates contained 20 µg ml⁻¹ nourseothricin. Deletion of the target gene was confirmed by PCR (for procedure see Ohm et al., 2010). Compatible monokaryons with a gene deletion were selected from spores originating from a cross of the mutant strains with wild-type strain 4-8.3. The *Δfst4* monokaryon showed no phenotypic differences when compared to the wild-type. In contrast, the *Δfst4Δfst4* dikaryon did not fruit but produced more aerial hyphae when compared to the wild-type (Figure 1). Apparently, Fst4 is involved in the switch between the vegetative phase and the reproductive phase.

**Table 2: primers used to inactivate putative transcription factors**

| **Gene (ProteinID)** | **Deletion construct (pROxxxx) with primers used to amplify flanking sequences** |
|---|---|
| ***c2h2*** (114363) | **pRO103** |
| dC2H2UpFw | GGCCTAATAGGCCCGGATGCTTTCTCGGAGAGG |
| dC2H2UpRev | GGCCTCGCAGGCCGAGCAGATGCTTCGCTCCGG |
| dC2H2DwFw | GGCCTGCGAGGCCCCAGTCGACCTCAATTAGCC |
| dC2H2DwRev | GGCCTATTAGGCCGCCCCTCACCCGTGTACCCG |
| | |

| ***gat1*** (255004) | **pRO190** |
|---|---|
| dGATA1UpFw | GGCCTAATAGGCCTGGTCAAGGCATCCCGCAG |
| dGATA1UpRev | GGCCTCGCAGGCCCTTCTTCTCAAGCCCAAATG |
| dGATA1DwFw | GGCCTGCGAGGCCTACTCTCATGCGAGACCCAC |
| dGATA1DwRev | GGCCTATTAGGCCCGTGGGTTGTTGAACTTACC |
| | |

| ***fst4*** (66861) | **pRO191** |
|---|---|
| dFst4UpFw | GGCCTAATAGGCCACAAGCAGCAGAGGCTTGG |
| dFst4UpRev | GGCCTCGCAGGCCGATTCGGACAGTCGAG |
| dFst4DwFw | GGCCTGCGAGGCCGACTATAGGATGGTGAGCG |
| dFst4DwRev | GGCCTATTAGGCCCAAACGGTGTCGGGAACGC |
| | |

| ***fst3*** (257422) | **pRO097** |
|---|---|
| dFst3UpFw | GGCCTAATAGGCCCGTTTCCTAGTACACCTGTC |
| dFst3UpRev | GGCCTCGCAGGCCGGAGAACGGGGTCCAGCAGG |
| dFst3DwFw | GGCCTGCGAGGCCAGACCACCGAAGGATAGTTG |
| dFst3DwRev | GGCCTATTAGGCCTCGTTGCTATCAGGAGCGGC |
| | |

| ***wc2*** (13988) | **pRO192** |
|---|---|
| dWC2UpFw2 | GGCCTAATAGGCCACCGTCACGTCCATGTTCG |
| dWC2UpRev2 | GGCCTCGCAGGCCCGAAACAACAATGATTG |
| dWC2DwFw | GGCCTGCGAGGCCCTAGATGTTCGGTAATTGCC |
| dWC2DwRev | GGCCTATTAGGCCCAGCCACCCATCTCGACTTG |
| | |

| ***hom2*** (257987) | **pRO189** |
|---|---|
| dHom2UpFw | GGCCTAATAGGCCTTGAGATGTTGCCTTGTCG |
| dHom2UpRev | GGCCTCGCAGGCCCAAGAGCAAGCGTTGAG |
| dHom2DwFw | GGCCTGCGAGGCCCACGATCTACCCAAACAG |
| dHom2DwRev | GGCCTATTAGGCCAGATCCAACGTGAGAGCCAG |
| | |

| ***hom1*** (257652) | **pRO093** |
|---|---|
| dHom1UpFw2 | GGCCTAATAGGCCAGTGCTGGTGAGACTCACG |
| dHom1UpRev2 | GGCCTCGCAGGCCCGATTGGTACGAGCTGGATG |
| dHom1DwFw | GGCCTGCGAGGCCCATTCTCATATGCCTCAAAC |
| dHom1DwRev | GGCCTATTAGGCCTCGTCTCTATTCACAACCGC |
| | |

| ***bri1*** (255701) | **pDelcas-BRIGHT** |
|---|---|
| dBrightUpFw | GGCCGAATGGGCCGTATGAAGGAAG |
| dBrighUpRev | GGCCCCGCTGGCCCTGCAAACGAAC |
| dBrightDwFw | GGCCAGCGAGGCCAGGTCCGTGATCCTTTGTG |
| dBrightDwRev | GGCCTATTAGGCCTGAAGGGCGGTAATGCTG |

### Example 4: Proof of principle: knockouts of other putative transcription factors

In addition to *fst3* and *fst4,* 6 other putative transcription factor genes have been inactivated. Inactivation followed the procedures described above using primers indicated in Table 2. These deletions also affected mushroom formation (see Table 3)

**Table 3 Transcription factors of S. commune that have been inactivated.**

| **Name** | **Protein ID** | **Phenotype knock-out** |
|---|---|---|
| *hom2* | 257987 | No mushrooms, radial colony growth |
| *wc2* | 13988 | No mushrooms, radial colony growth |
| *fst4* | 66861 | No mushrooms, normal colony morphology |
| *c2h2* | 114363 | Development stops in stage I |
| *fst3* | 257422 | More but smaller mushrooms |
| *gat1* | 255004 | More but smaller mushrooms |
| *hom1* | 257652 | More but smaller mushrooms |
| *bri1* | 255701 | No mushrooms formed |

These results clearly show that with the above described method we have identified transcription factors that are involved in fruiting body development in *S. commune.*

### Example 5

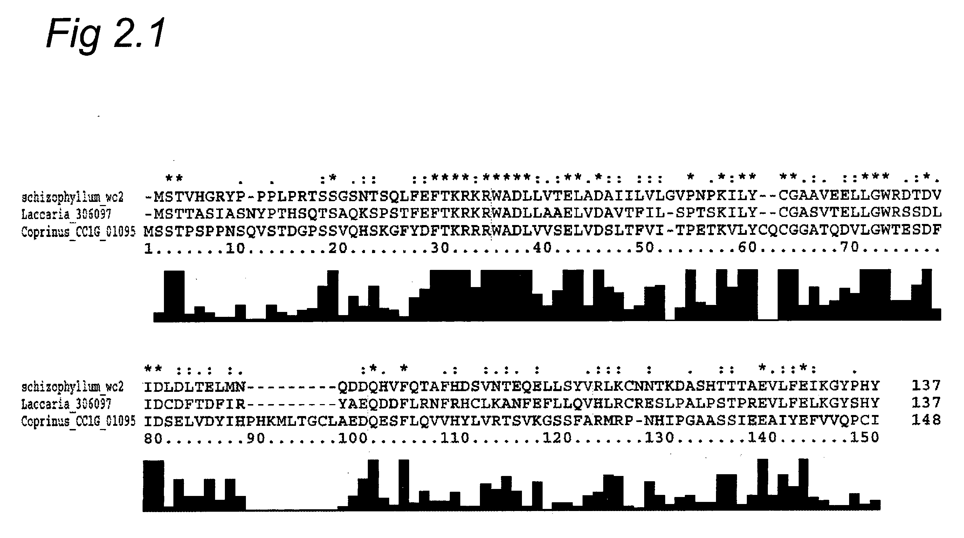

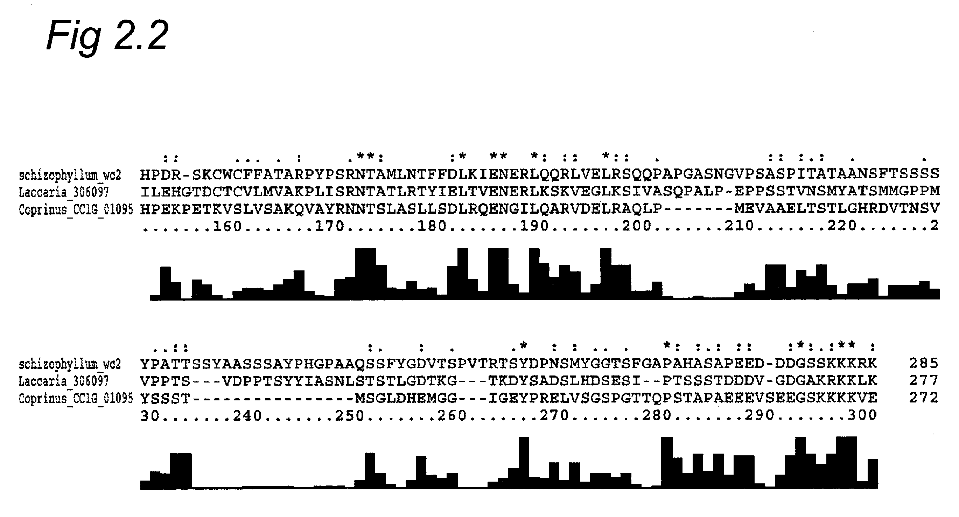

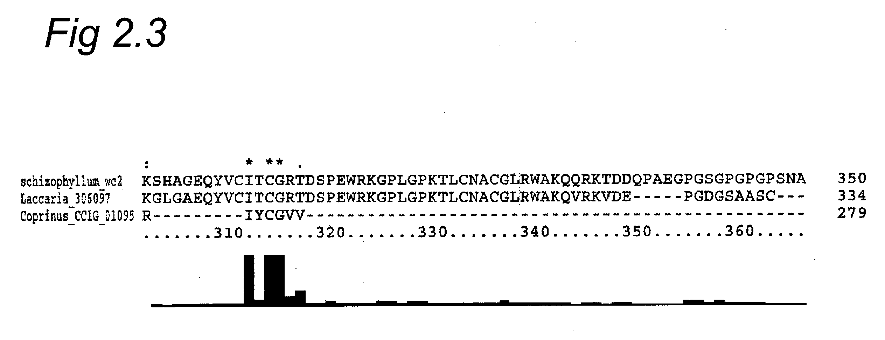

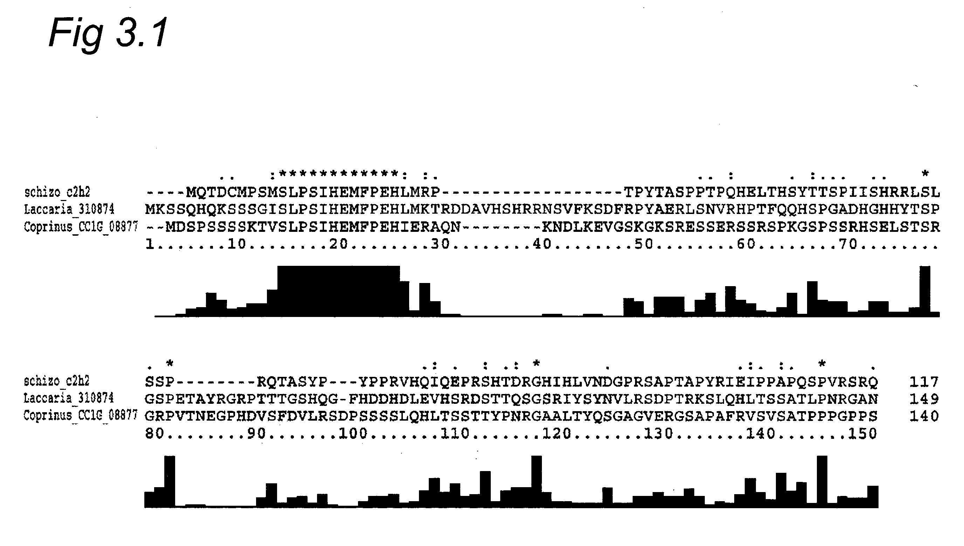

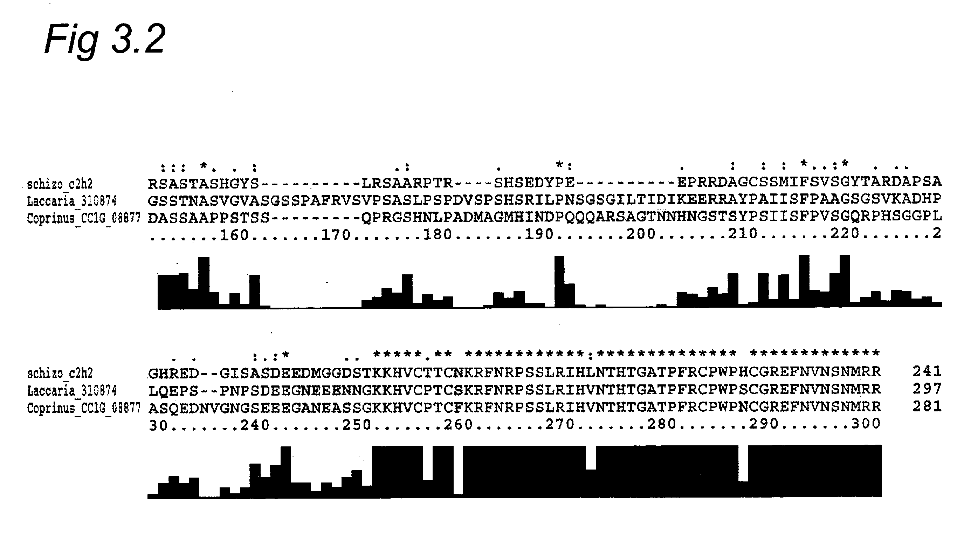

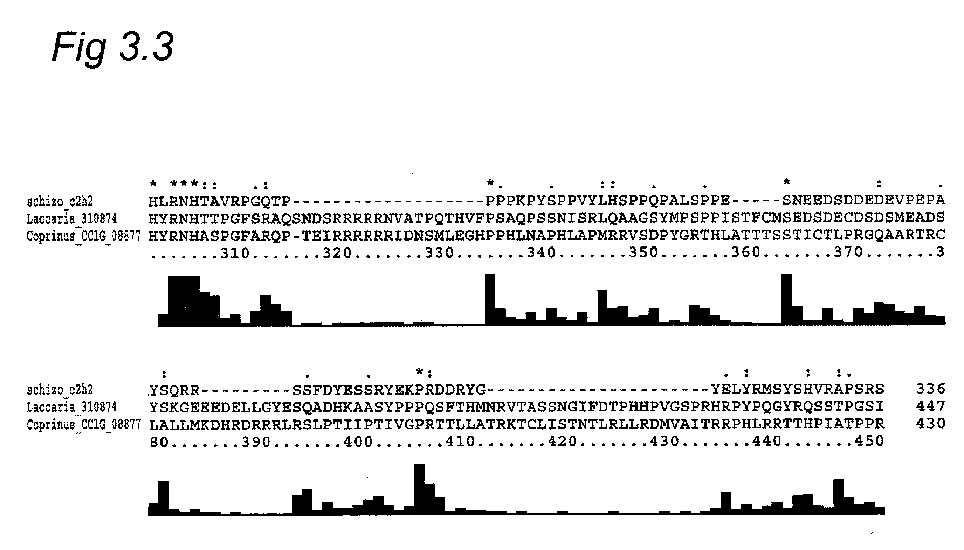

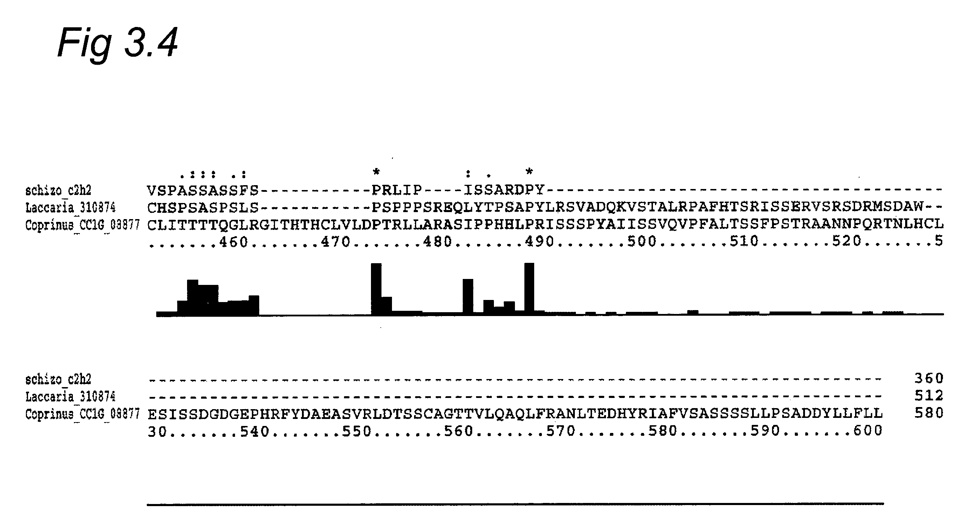

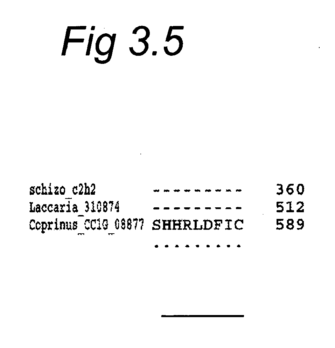

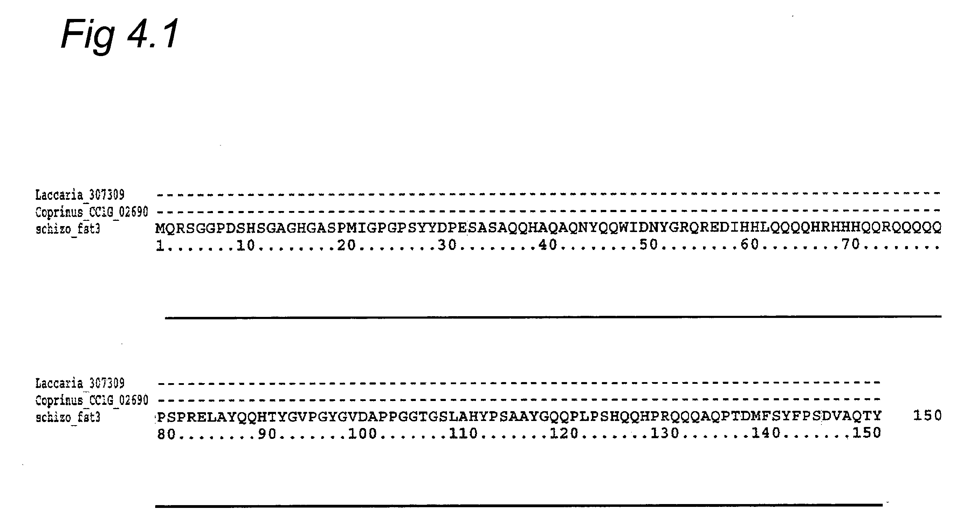

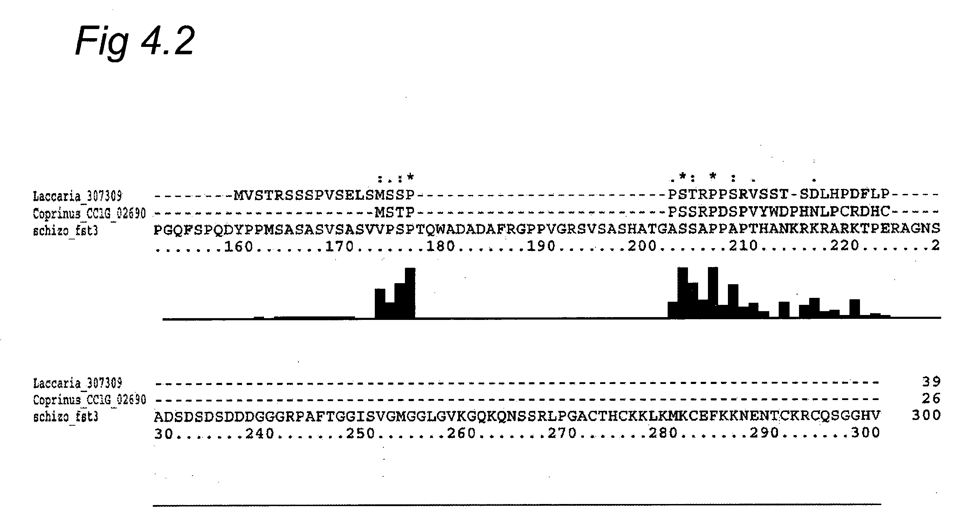

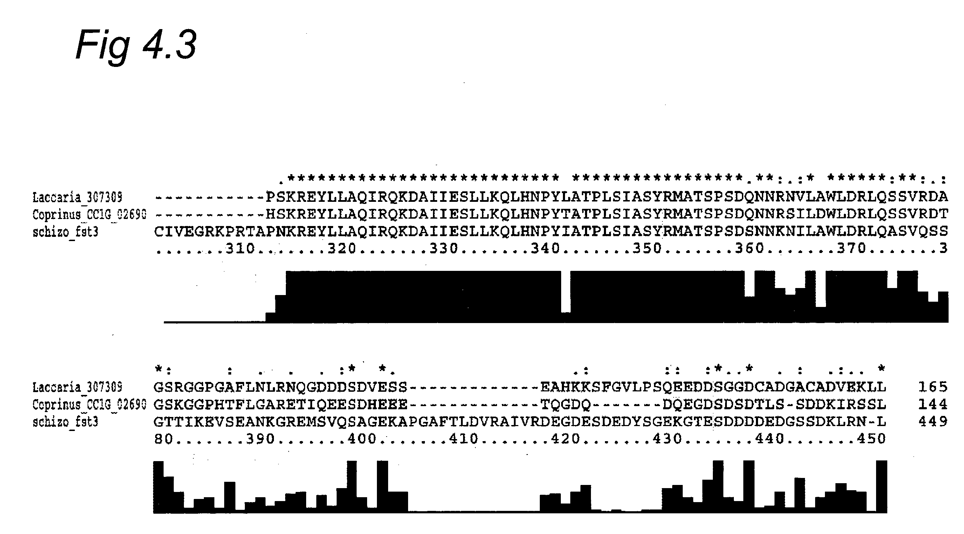

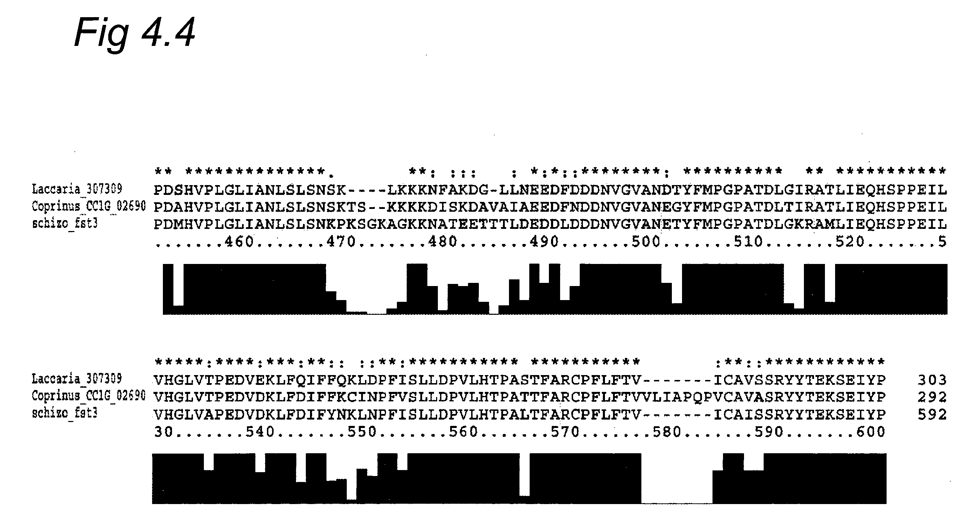

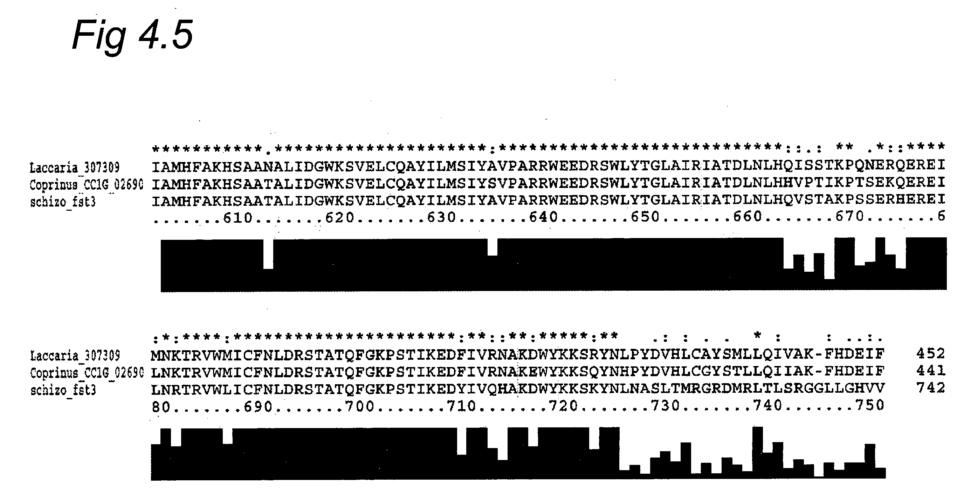

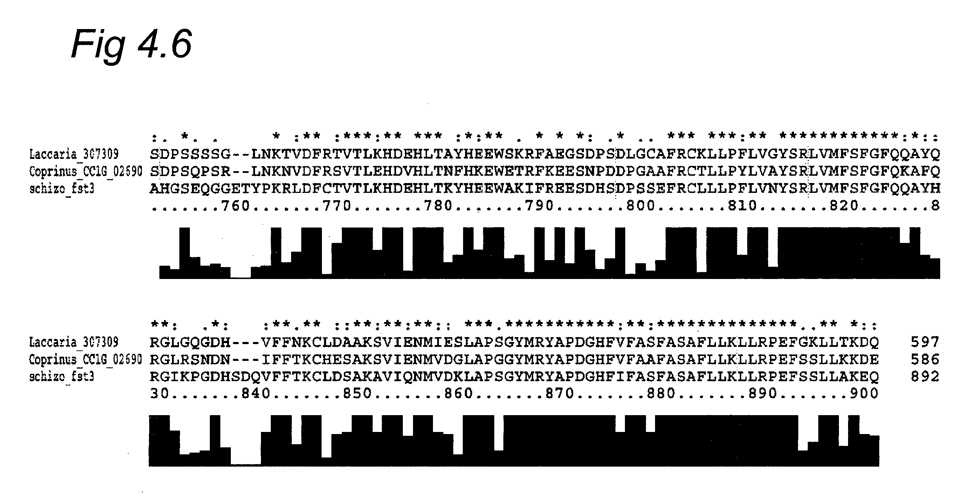

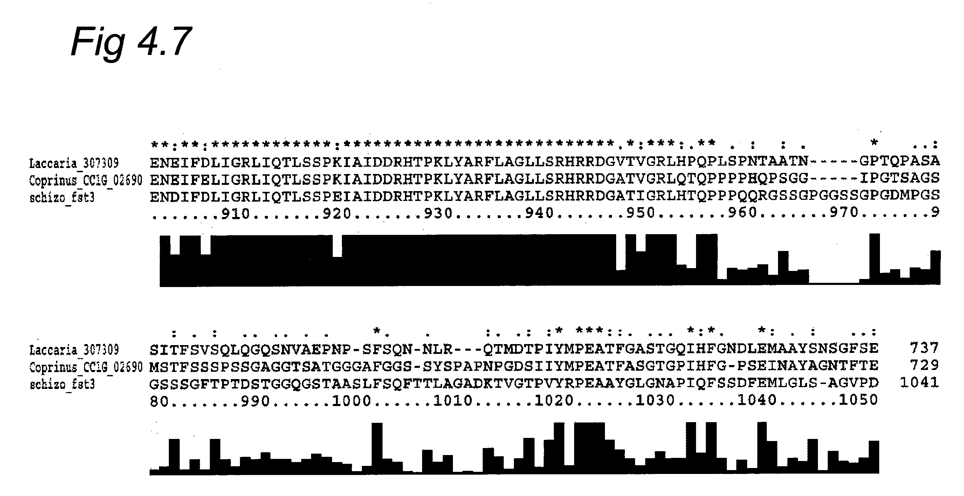

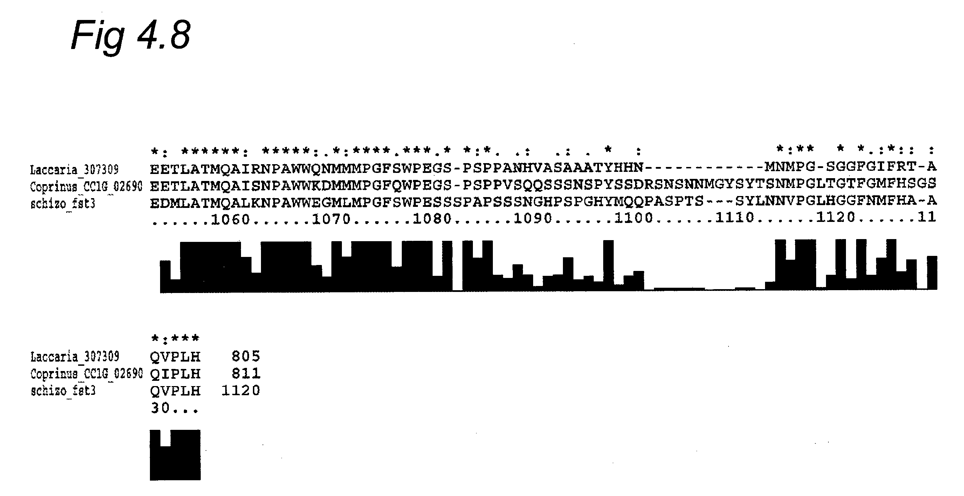

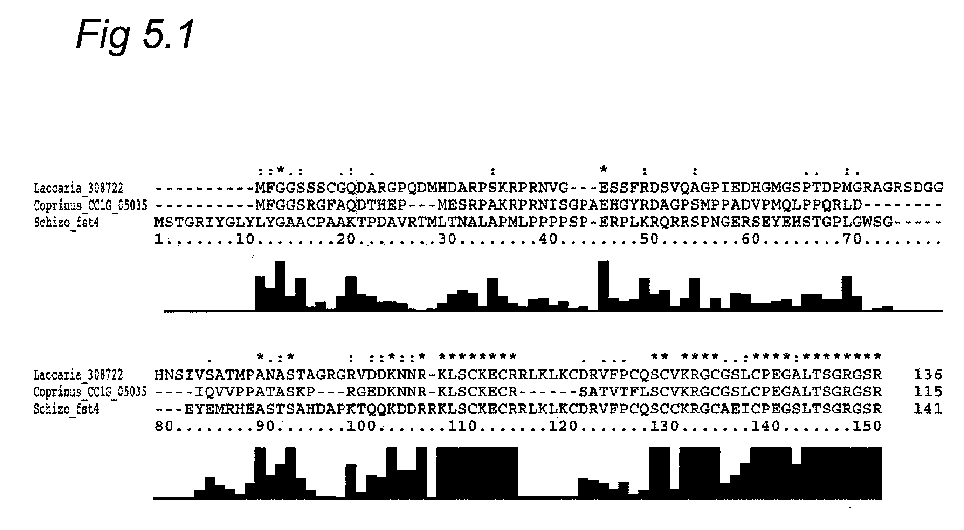

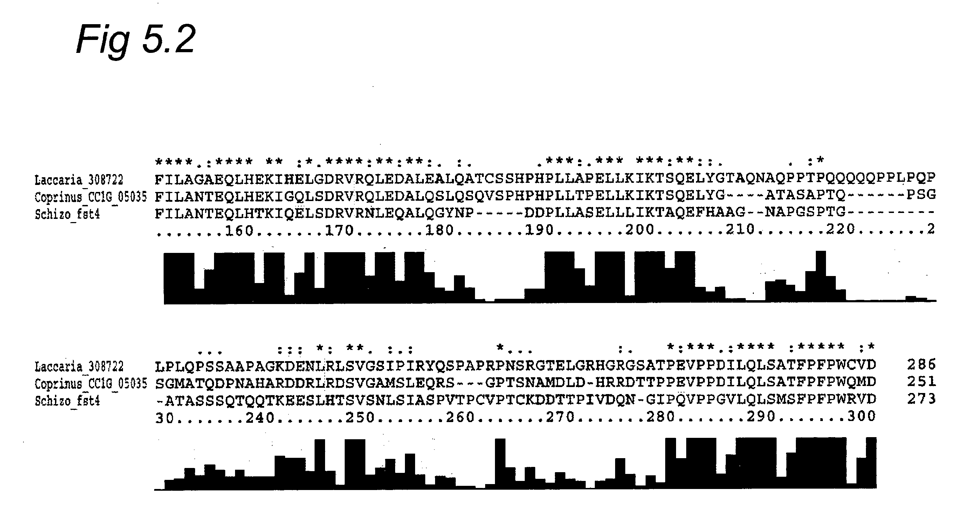

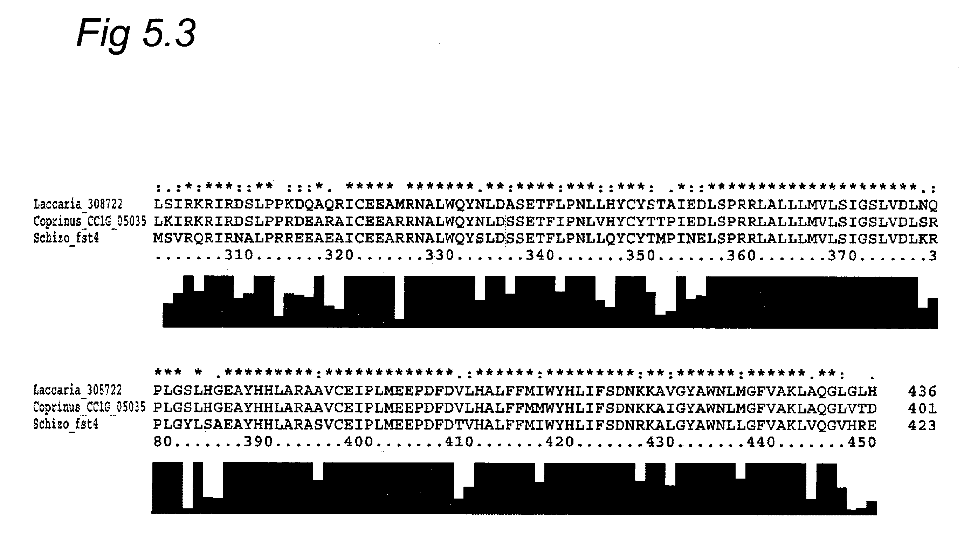

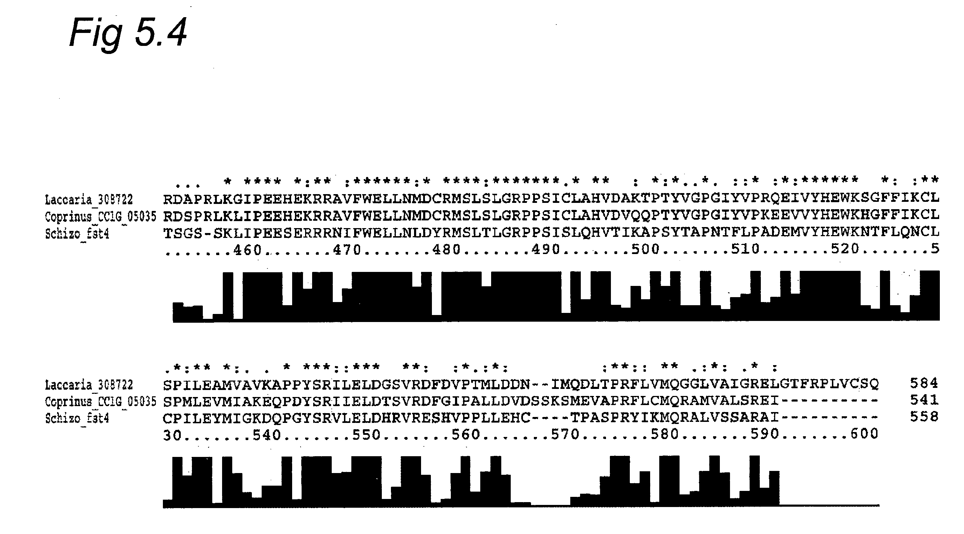

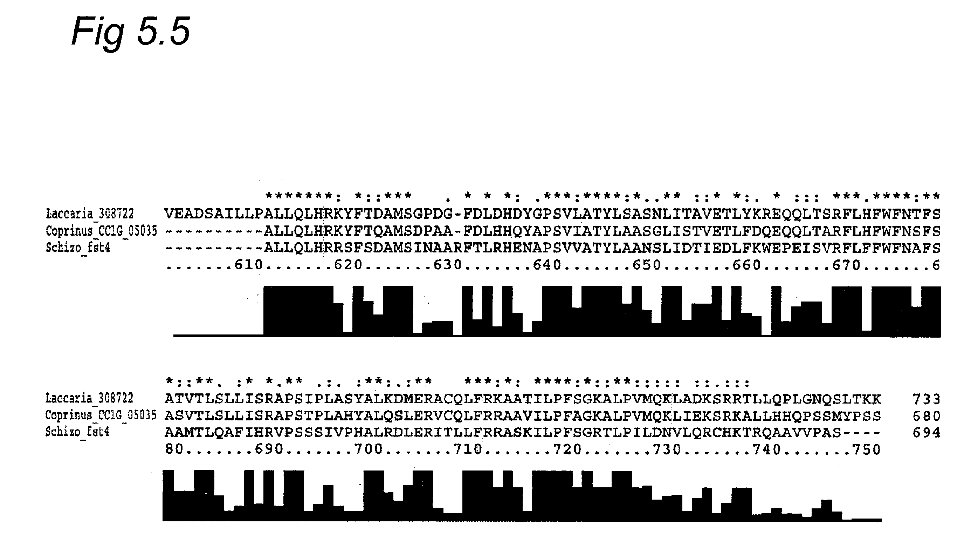

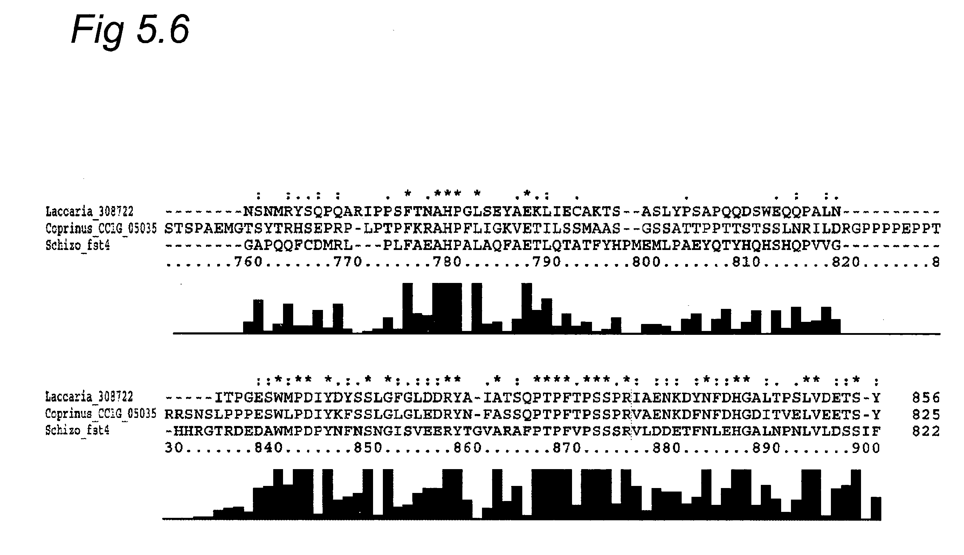

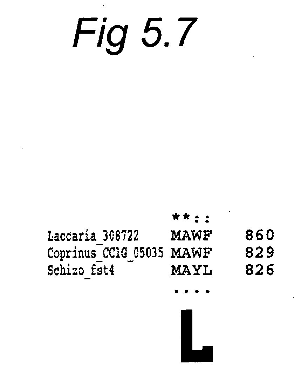

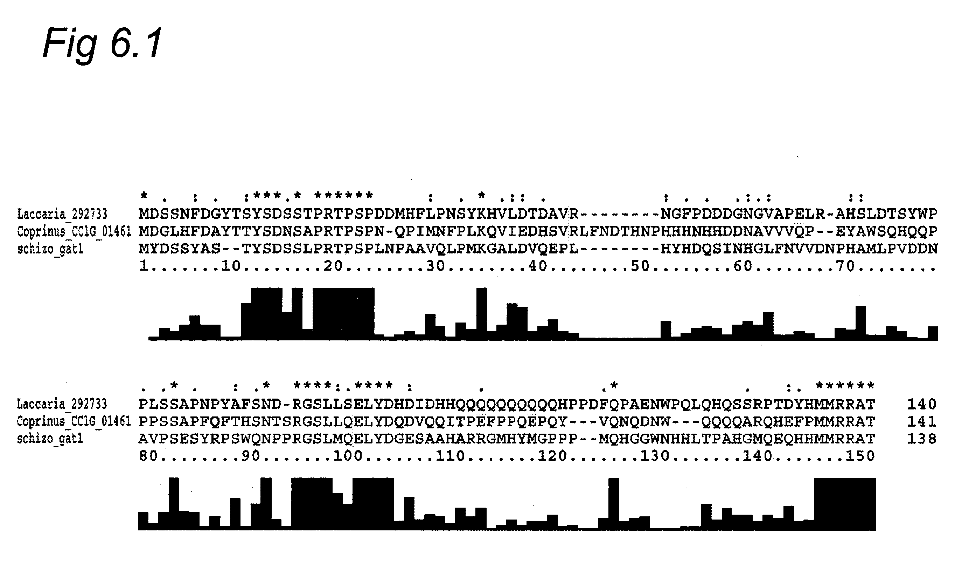

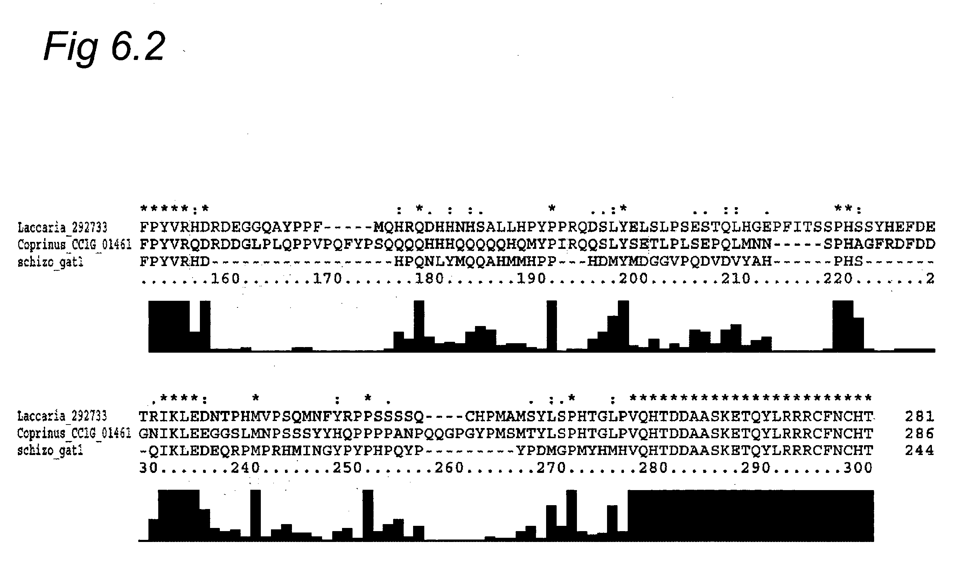

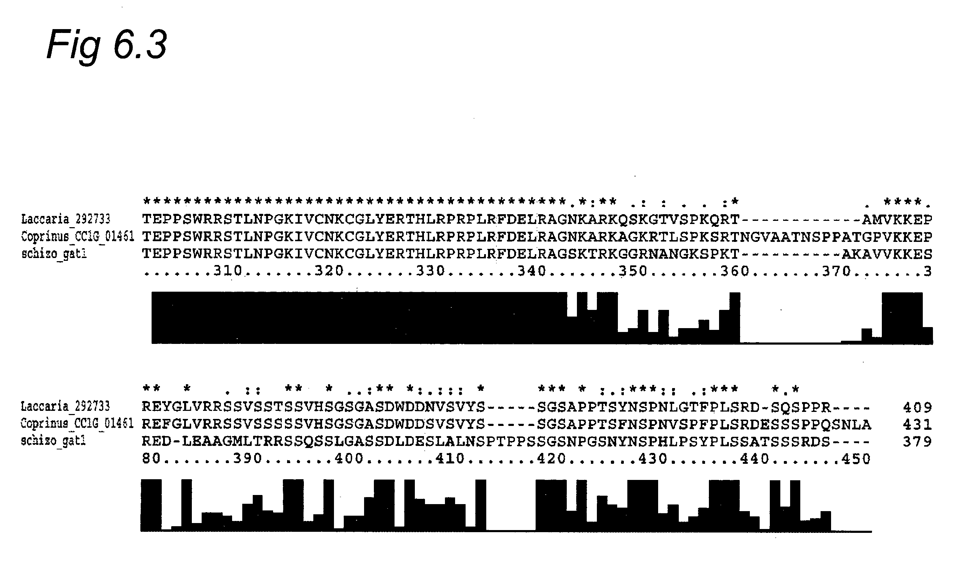

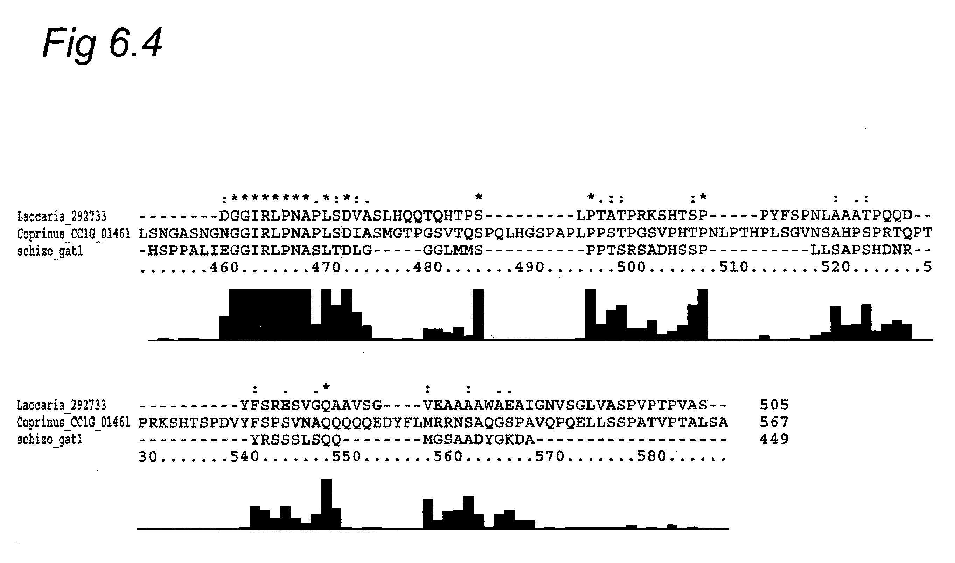

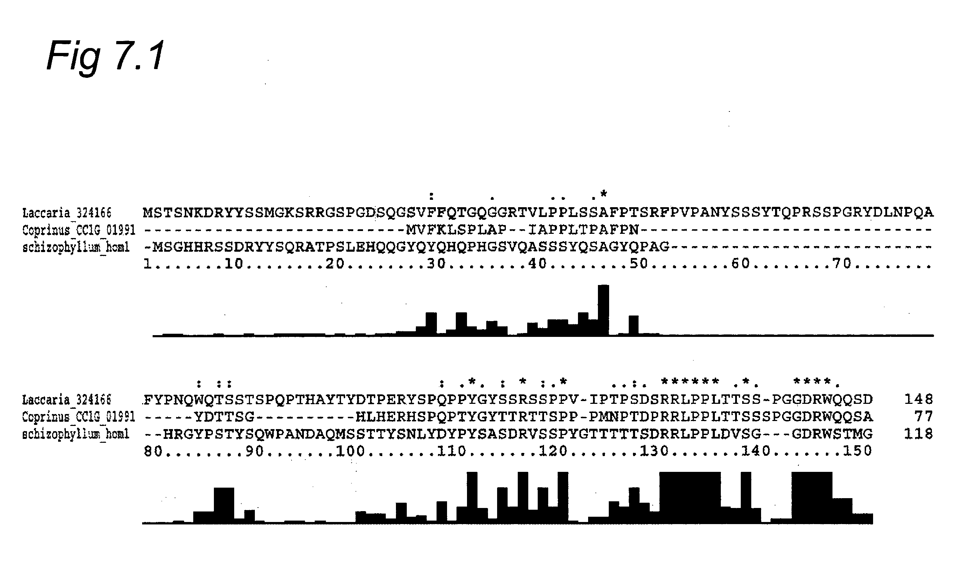

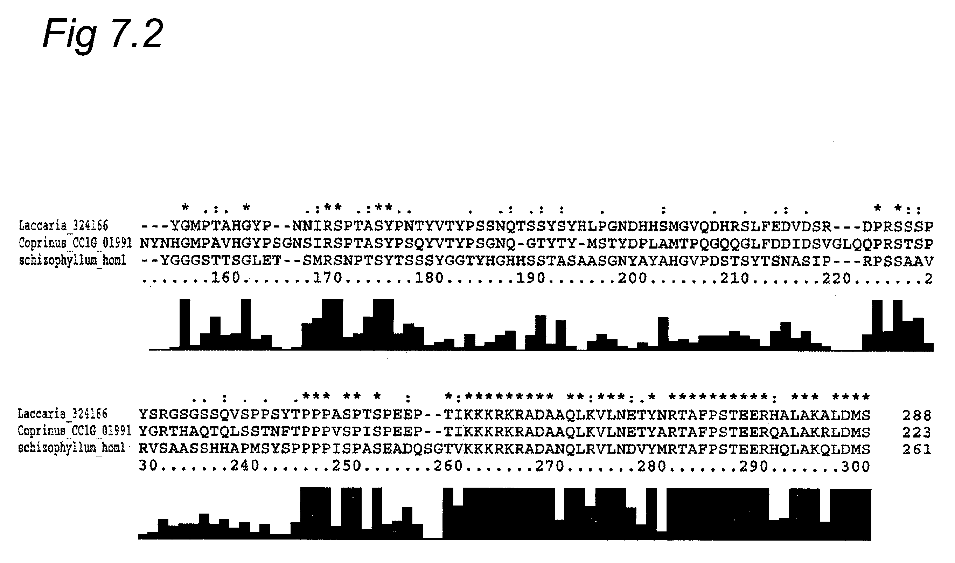

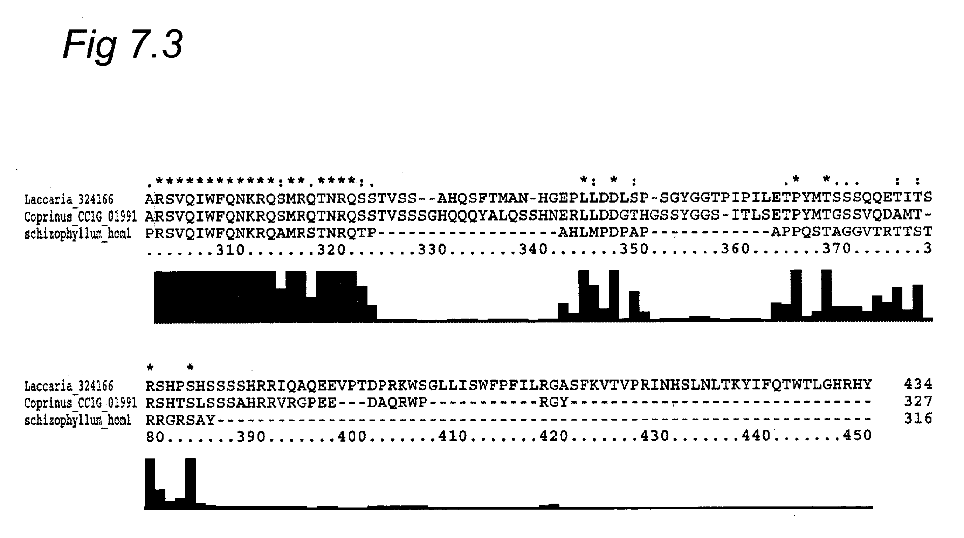

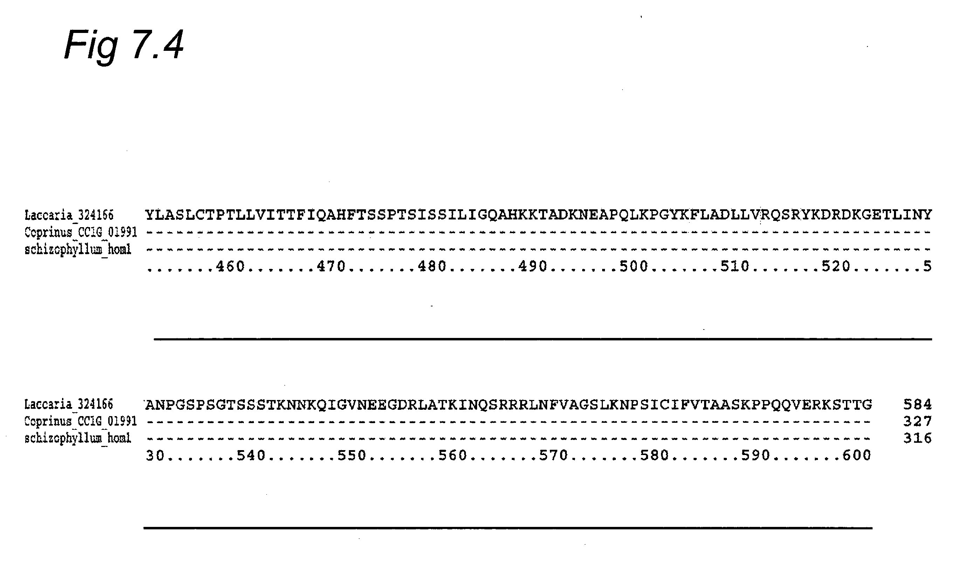

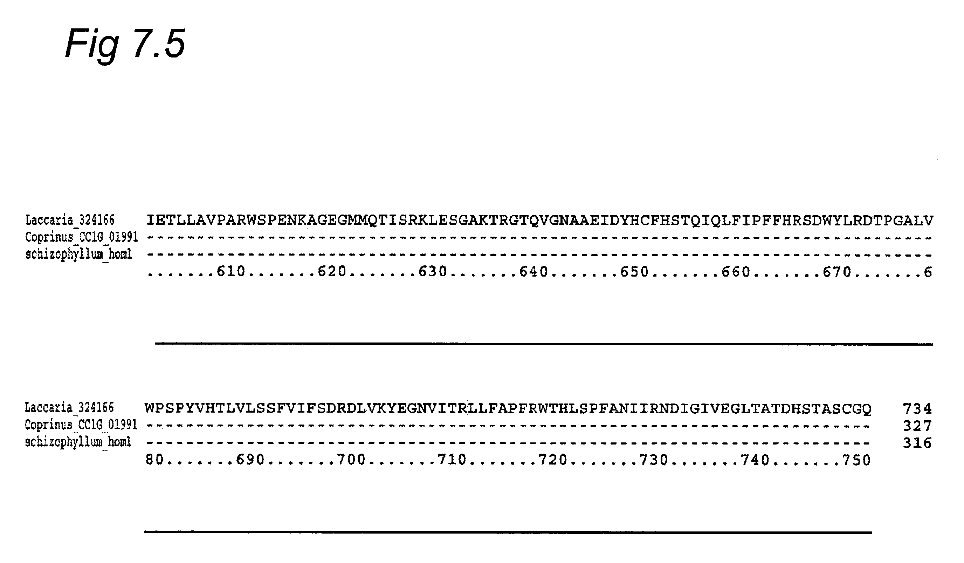

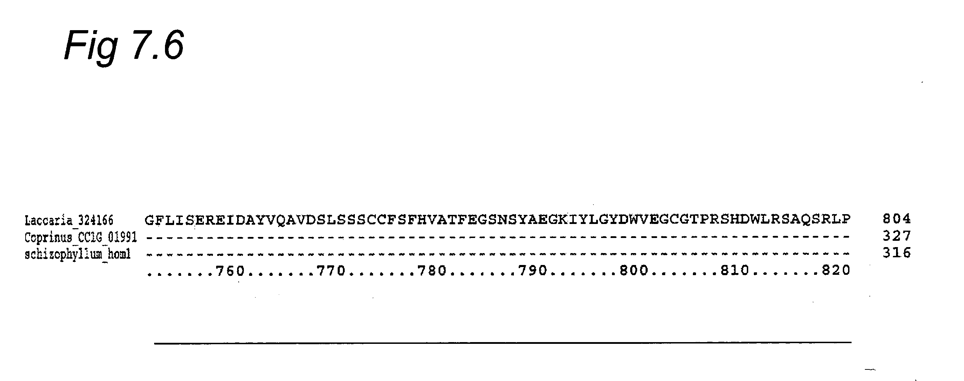

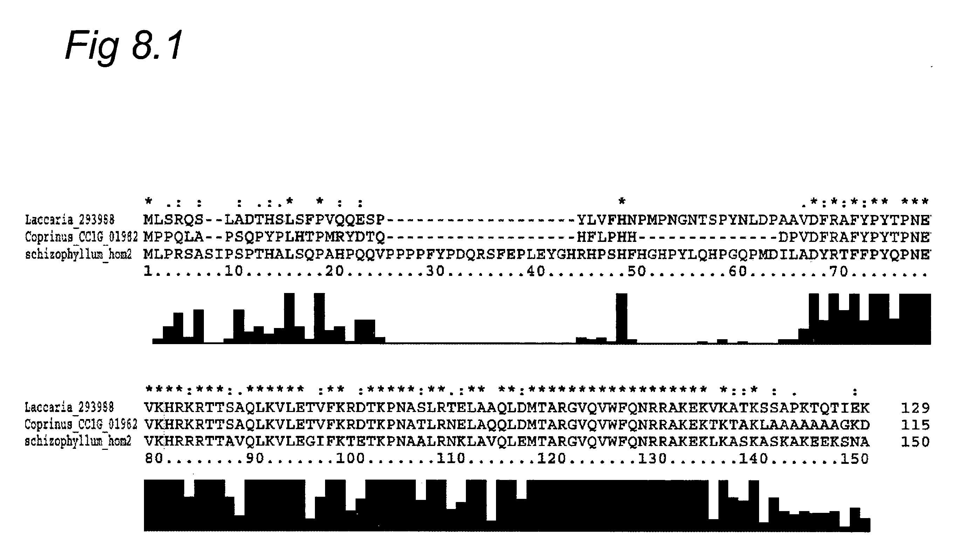

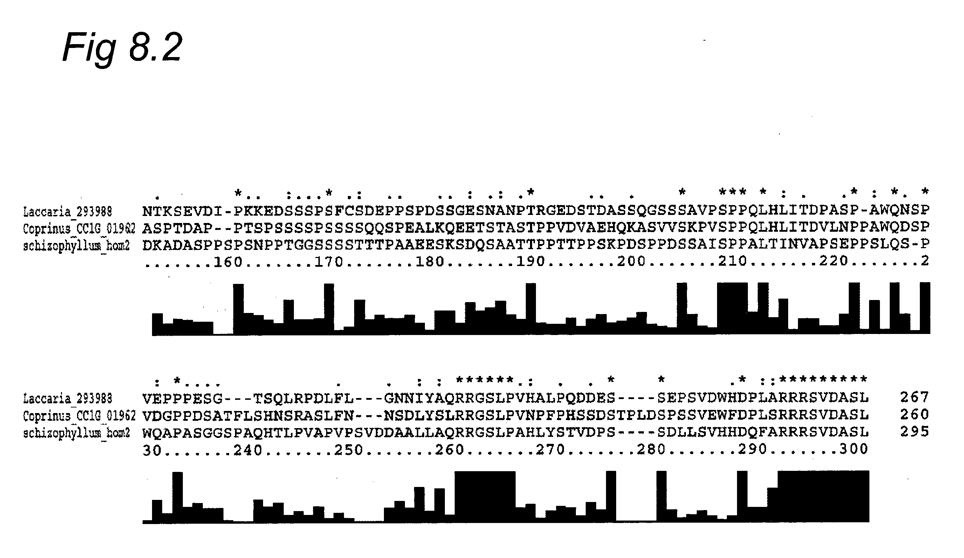

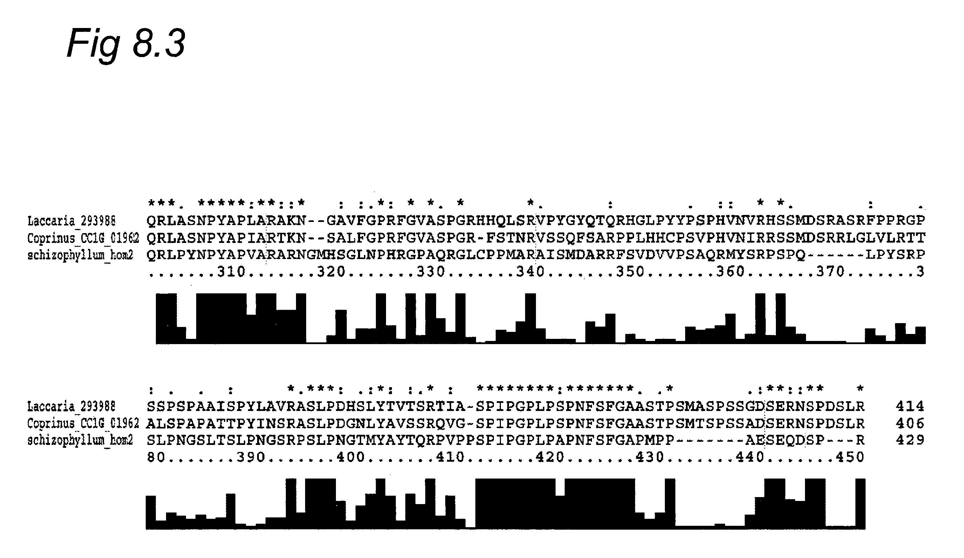

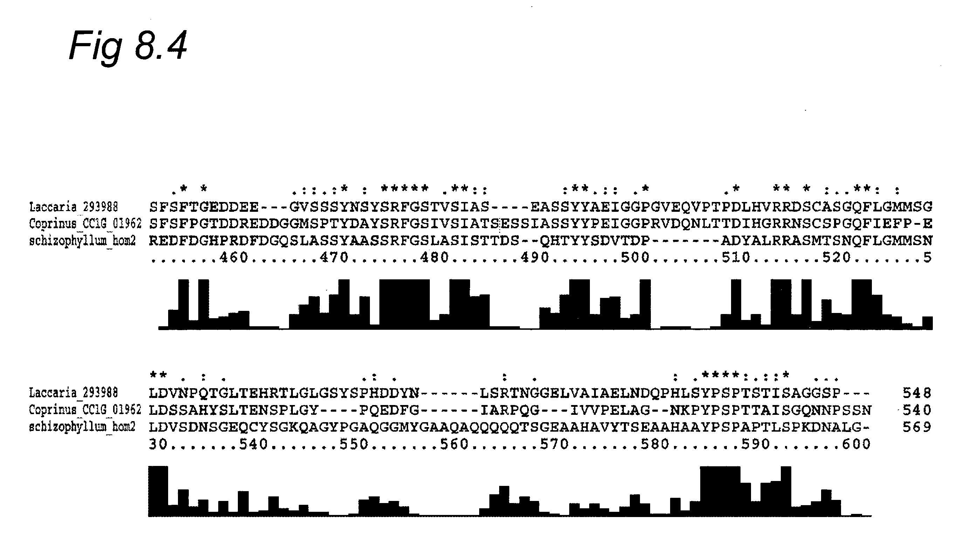

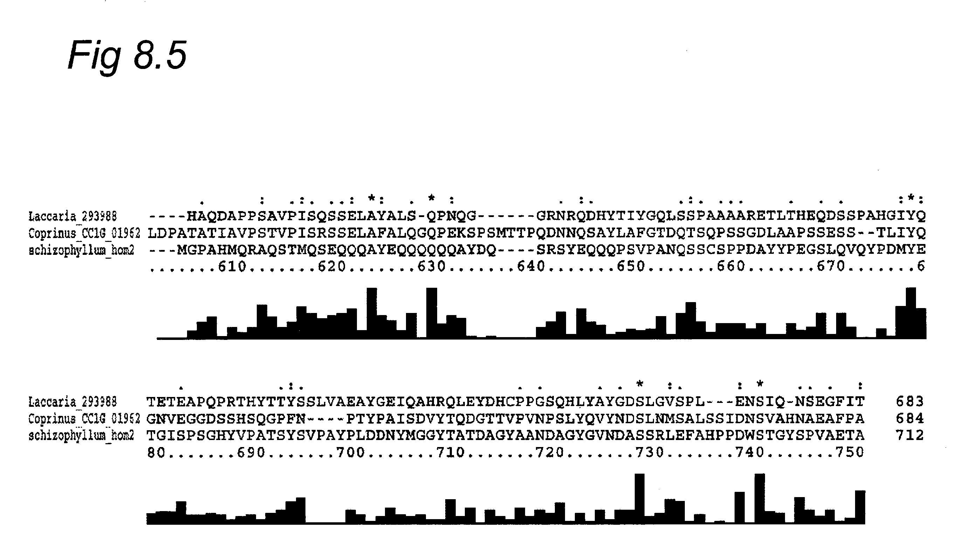

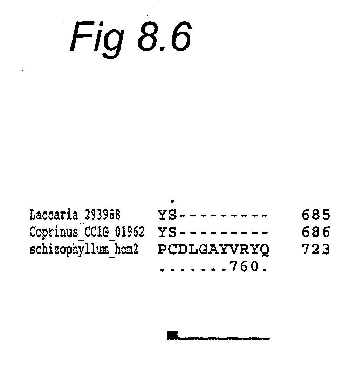

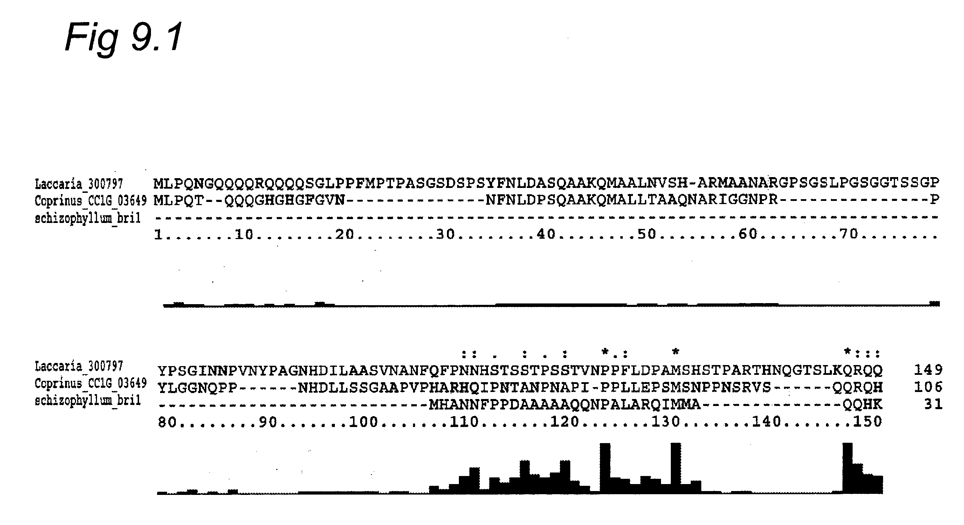

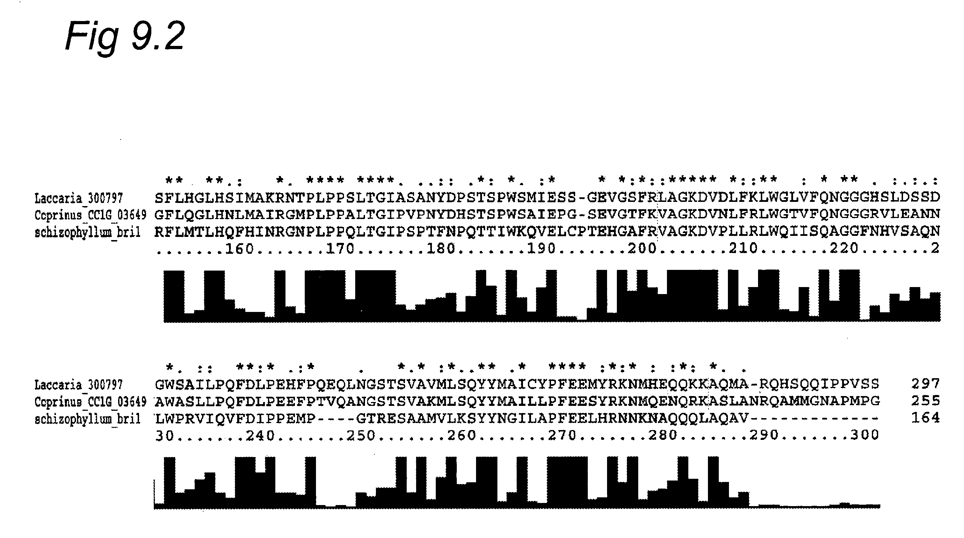

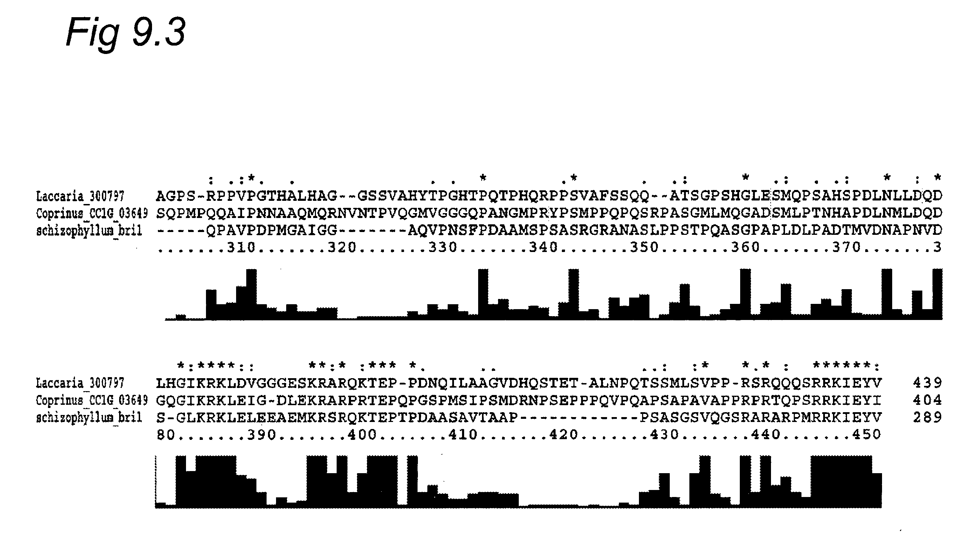

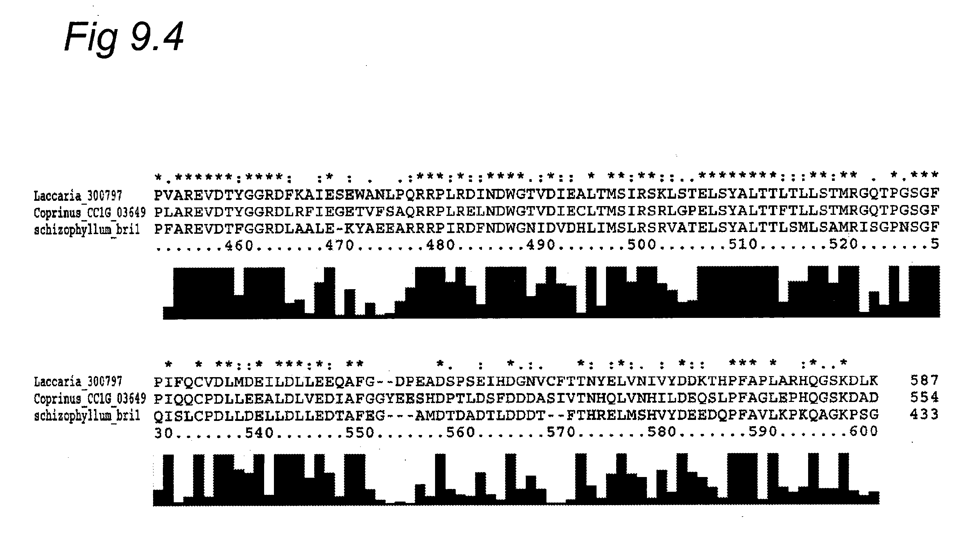

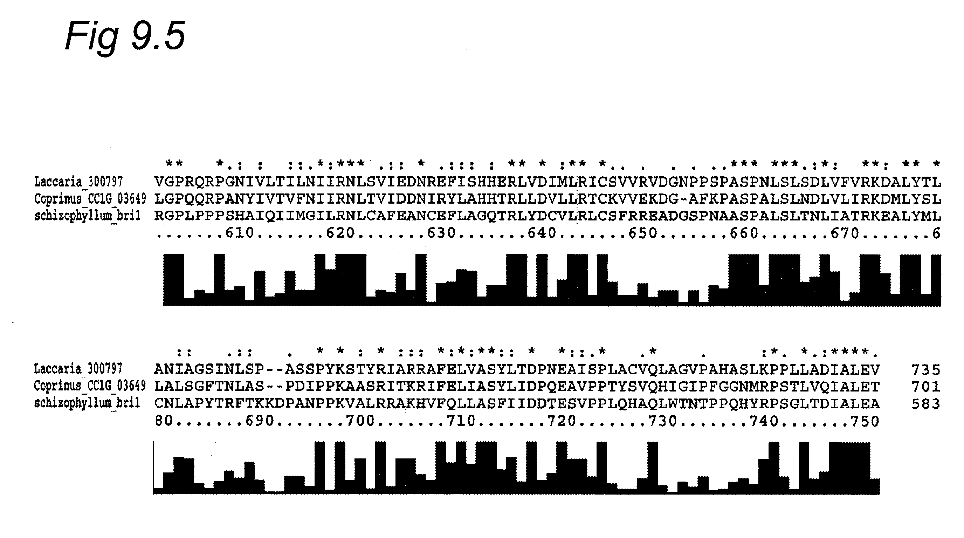

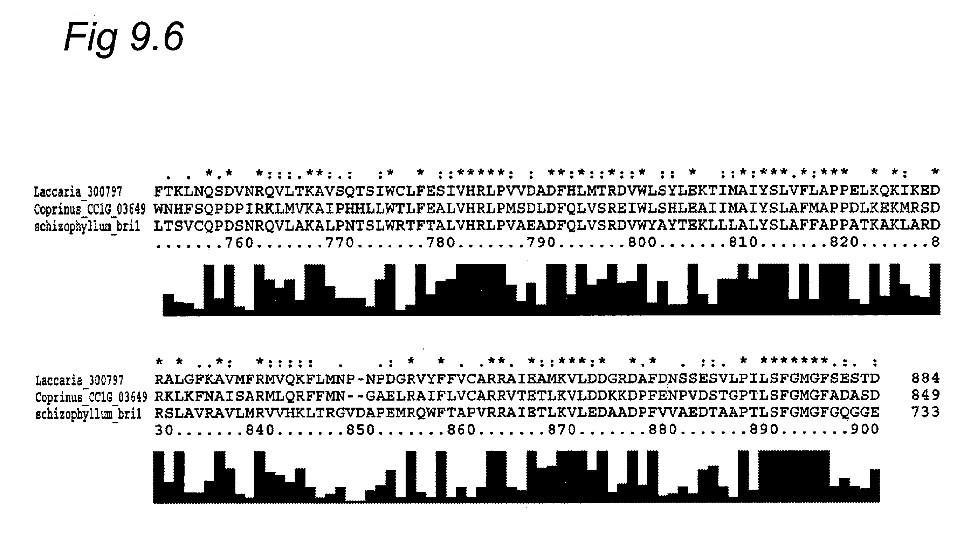

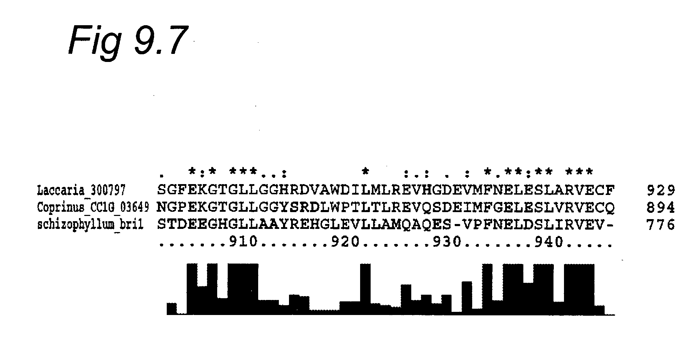

The public protein databases of *Coprinopsis cinerea* and *Laccaria bicolor* were examined for homologs of the 8 transcription factors (Table 3) using the blastp algorithm (version 2.2.23+, using default settings). The protein sequence of the best hit was extracted from the database. Alignments were made with ClustalX (version 2.0.12, using default settings) in order to identify conserved domains in the protein sequences (Table 4; figures 2 - 9).

**Table 4:Domains of S. commune transcription factors that are conserved in C. cinerea and L. bicolor.**

| Transcription factor | Domain start | Domain stop | Domain sequence |
|---|---|---|---|
| Bri1 | 283 | 358 | RRKIEYVPFAREVDTFGGRDLAALEKYAE EARRRPIRDFNDWGNIDVDHLIMSLRSRVA |
| | | | TELSYALTTLSMLSAMR |
| C2h2 | 192 | 247 | KKHVCTTCNKRFNRPSSLRIHLNTHTGATP FRCPWPHCGREFNVNSNMRRHLRNHT |
| Fst3 | 593 | 716 | IAMHFAKHSAATALIDGWKSVELCQAYIL MSIYAVPARRWEEDRSWLYTGLAIRIATDL NLHQVSTAKPSSERHEREILNRTRVWLICF NLDRSTATQFGKPSTIKEDYIVQHAKDWY KKSKYN |
| Fst4 | 329 | 463 | LSPRRLALLLMVLSIGSLVDLKRPLGYLSA EAYHHLARASVCEIPLMEEPDFDTVHALFF MIWYHLIFSDNRKALGYAWNLLGFVAKL VQGVHRETSGSSKLIPEESERRRNIFWELLN LDYRMSLTLGRPPSIS |
| Gat1 | 221 | 291 | VQHTDDAASKETQYLRRRCFNCHTTEPPS WRRSTLNPGKIVCNKCGLYERTHLRPRPLR FDELRAGSKTRK |
| Hom1 | 222 | 228 | KKKRKRADANQLRVLNDVYMRTAFPSTE ERHQLAKQLDMSPRSVQIWFQNKRQAMR STNRQ |
| Hom2 | 67 | 139 | DYRTFFPYQPNEVKHRRRTTAVQLKVLEGI FKTETKPNAALRNKLAVQLEMTARGVQV WFQNRRAKEKLKASK |
| Wc2 | 27 | 83 | FTKRKRWADLLVTELADAIILVLGVPNPKI LYCGAAVEELLGWRDTDVIDLDLTELM |

**Table 5. Relationship Protein ID (see (http://jgi.doe.gov/Scommune) and Protein Domain (see Table 4) and SEQ ID NO's as used herein.**

| ProteinID | SEQ ID NO: | ProteinID | SEQ ID NO: | ProteinID | SEQ ID NO: |
|---|---|---|---|---|---|
| 84275 | 1 | 102836 | 33 | 237000 | 65 |
| 112067 | 2 | 102971 | 34 | 110445 | 66 |
| 75142 | 3 | 103145 | 35 | 269975 | 67 |
| 255701 | 4 | 105299 | 36 | 256910 | 68 |
| 17463 | 5 | 108072 | 37 | 84085 | 69 |
| 80413 | 6 | 269950 | 38 | 104304 | 70 |
| 269940 | 7 | 110010 | 39 | 111555 | 71 |
| 68168 | 8 | 110310 | 40 | 258217 | 72 |
| 80935 | 9 | 110595 | 41 | 257931 | 73 |
| 65208 | 10 | 269932 | 42 | 269938 | 74 |
| 269941 | 11 | 111234 | 43 | 110229 | 75 |
| 81364 | 12 | 269957 | 44 | 112017 | 76 |
| 236086 | 13 | 113625 | 45 | 110458 | 77 |
| 258244 | 14 | 114874 | 46 | 255386 | 78 |
| 233954 | 15 | 230844 | 47 | 14572 | 79 |
| 81115 | 16 | 233513 | 48 | 81262 | 80 |
| 50846 | 17 | 236631 | 49 | 269939 | 81 |
| 269944 | 18 | 255185 | 50 | 48318 | 82 |
| 11907 | 19 | 255490 | 51 | 257380 | 83 |
| 13988 | 20 | 255941 | 52 | 112634 | 84 |
| 255004 | 21 | 256135 | 53 | 255183 | 85 |
| 85539 | 22 | 257422 | 54 | 80526 | 86 |
| 83110 | 23 | 257652 | 55 | 107138 | 87 |
| 16376 | 24 | 114363 | 56 | 236743 | 88 |
| 53446 | 25 | 103949 | 57 | 105290 | 89 |
| 67562 | 26 | 250177 | 58 | 84273 | 90 |
| 54452 | 27 | 258543 | 59 | 254923 | 91 |
| 86194 | 28 | 85474 | 60 | 71685 | 92 |
| 66861 | 29 | 11542 | 61 | 237374 | 93 |
| 255327 | 30 | 110354 | 62 | 255836 | 94 |
| 84267 | 31 | 230646 | 63 | 255161 | 95 |
| 84657 | 32 | 110178 | 64 | 62967 | 96 |
| 79748 | 97 | 231700 | 131 | 111623 | 165 |
| 82694 | 98 | 256693 | 132 | 269956 | 166 |
| 112825 | 99 | 257455 | 133 | 231698 | 167 |
| 257915 | 100 | 258832 | 134 | 234557 | 168 |
| 103232 | 101 | 52392 | 135 | 234560 | 169 |
| 81412 | 102 | 257495 | 136 | 269958 | 170 |
| 269943 | 103 | 57298 | 137 | 255656 | 171 |
| 102719 | 104 | 57817 | 138 | 255852 | 172 |
| 255207 | 105 | 83015 | 139 | 256706 | 173 |
| 17379 | 106 | 269928 | 140 | 257056 | 174 |
| 73063 | 107 | 63410 | 141 | 257622 | 175 |
| 104375 | 108 | 66095 | 142 | 257926 | 176 |
| 85886 | 109 | 66586 | 143 | 257987 | 177 |
| 232127 | 110 | 73210 | 144 | 269960 | 178 |
| 257445 | 111 | 74309 | 145 | 257265 | 179 |
| 104344 | 112 | 74719 | 146 | 269961 | 180 |
| 81107 | 113 | 77191 | 147 | 248401 | 181 |
| 255863 | 114 | 78089 | 148 | 82883 | 182 |
| 232771 | 115 | 81726 | 149 | 84749 | 183 |
| 233370 | 116 | 81806 | 150 | 110478 | 184 |
| 256320 | 117 | 84684 | 151 | 254870 | 185 |
| 109936 | 118 | 269945 | 152 | 258883 | 186 |
| 114988 | 119 | 254988 | 153 | 108216 | 187 |
| 111683 | 120 | 255385 | 154 | 108605 | 188 |
| 230584 | 121 | 104000 | 155 | 102516 | 189 |
| 232060 | 122 | 269948 | 156 | 256746 | 190 |
| 66326 | 123 | 269949 | 157 | 258642 | 191 |
| 78316 | 124 | 256993 | 158 | 112405 | 192 |
| 86018 | 125 | 108591 | 159 | 83895 | 193 |
| 257247 | 126 | 109190 | 160 | 109596 | 194 |
| 232448 | 127 | 269952 | 161 | 269979 | 195 |
| 232514 | 128 | 110136 | 162 | 77161 | 196 |
| 63699 | 129 | 110416 | 163 | 233354 | 197 |
| 111405 | 130 | 250298 | 164 | 233946 | 198 |

| ProteinID | SEQ ID NO: | | | | |
|---|---|---|---|---|---|
| 12349 | 199 | | | | |
| 250247 | 200 | | | | |

| Protein domain | SEQ ID NO: | | | | |
|---|---|---|---|---|---|
| Bri1 domain | 201 | | | | |
| C2h2 domain | 202 | | | | |
| Fst3 domain | 203 | | | | |
| Fst4 domain | 204 | | | | |
| Gat1 domain | 205 | | | | |
| Hom1 domain | 206 | | | | |
| Hom2 domain | 207 | | | | |
| Wc2 domain | 208 | | | | |

### REFERENCES

Altschul SF et al. (1990). J. Mol. Biol. 215:403-410.
Ashburner M et al. (2000) Nat. Genet. 25, 25-29.
Alves, AMCR et al. (2004) Appl. Environm. Microbiol. 70, 6379-6384.
Brenner S et al. (2000) Nat. Biotechnol. 18, 630-634.
Bromberg SK and Schwalb MN (1977). Can. J. Genet. Cytol. 19,477-481.
de Jong JF et al (2006). Appl Environ Microbiol 72, 1267-1269.
Devereux J et al. (1984). Nucleic Acids Research 12: 387,
Finn, RD et al. (2008) Nucleic Acids Res. Database Issue 36,D281-D288.
Fleer R et al., 1991, Biotechnology 9:968-975
Harmsen MC et al. (1992). Curr Genet. 22, 447-454.
Hentikoff and Hentikoff (1992) Proc. Natl. Acad. Sci. USA. 89:10915-10919
Horton JS, Palmer GE, Smith WJ (1999). Fungal Genet. Biol. 26,33-47.
Koonin EV et al. (2004) Genome Biol. 5, R7.
Kues, U. (2000). Microbiol. Mol. Biol. Rev. 64, 316-353.
Munoz-Rivas A. et al (1986). Mol. Gen. Genet. 205: 103-105, erratum in Mol. Gen. Genet., (1986), 205; 576).
Murakuchi, H., and Kamada, T. (1998). Development 125, 3133-3141.
Murakuchi, H., and Kamada, T. (2000). Fungal Genet. Biol. 29, 49-59.
Murata et al (1998). Genetics 149, 1753-1761.
Needleman and Wunsch (1970) J. Mol. Biol. 48:443-453
Raper JR and Krongelb GS (1958). Mycologia 59, 707-740.
Ohm RA et al. (2010). An efficient gene deletion procedure for the mushroom-forming basidiomycete Schizophyllum commune. World Journal of Microbiology doi:10.1007/s11274-010-0356-0.
Schuren FH et al. (1994). Curr. Genet. 26: 179-183.
Springer J and Wessels JGH (1989). Mol. Gen. Genet. 219, 486-488.
Stamets P and Chilton JS (1983) Mushroom Cultivator: A Practical Guide to Growing Mushrooms at Home. Agarikon Press
Umar MH and van Griensven LJLD (1997). Mycologia 89, 274-277.
Van Griensven, L.J.L.D. (1988). The cultivation of mushrooms. Rustington, Darlington Mushroom Laboratories Ltd.
van Peer AF et al (2009). Appl Environ Microbiol 75, 1243-1247.
Wösten HAB and Wessels JGH (2006). The emergence of fruiting bodies in basidiomycetes. in The Mycota. Part I: Growth, Differentiation and Sexuality (eds. Kues, U. & Fisher, R.) 393-414, (Springer Verlag, Berlin).

## Claims

1. A fungus or a mushroom with an increased expression level of a polypeptide involved in mushroom formation, wherein the polypeptide has at least 40% amino acid identity with SEQ ID NO: 56 and/or has at least 50% amino acid identity with SEQ ID NO: 202, and wherein the increased expression level of the polypeptide is an increased production of the polypeptide and/or a higher activity of said polypeptide than the parental fungus/mushroom this fungus/mushroom is derived from when both are cultured and/or assayed under the same conditions.

2. A fungus or mushroom according to claim 1, further comprising an increased or decreased expression level of a polypeptide, wherein the polypeptide whose expression level is increased or decreased has at least 40% amino acid identity with a sequence selected from SEQ ID NO: 1-55, 57-200 and/or that has at least 50% amino acid identity with a sequence selected from SEQ ID NO: 201, 203-208.

3. A fungus or a mushroom according to claim 1, wherein the polypeptide of which the expression level is increased is a heterologous polypeptide.

4. A fungus or a mushroom according to claim 1, wherein the polypeptide of which the expression level is increased is an endogenous polypeptide.

5. A fungus or a mushroom according to claim 2, wherein the decreased expression level is reached by a decreased production of the polypeptide and/or a lower activity of said polypeptide than the parental fungus/mushroom this fungus/mushroom is derived from when both are cultured and/or assayed under the same conditions.

6. A fungus or a mushroom according to any one of claims 2 and 5, wherein the polypeptide whose expression level is increased comprises an amino acid sequence that has at least 40% amino acid identity with SEQ ID NO: 56 and wherein the polypeptide whose expression level is decreased comprises an amino acid sequence that has at least 40% amino acid identity with a sequence selected from SEQ ID NO: 55; 21; 54.

7. A fungus or a mushroom according to claim 6, wherein the polypeptide whose expression level is increased comprises an amino acid sequence that has at least 50% amino acid identity with SEQ ID NO: 202 and wherein the polypeptide whose expression level is decreased comprises an amino acid sequence that has at least 50% amino acid identity with a sequence selected from the group consisting of SEQ ID NO: 206, 205 and 203.

8. A fungus or a mushroom according to any one of the preceding claims, wherein the fungus/mushroom is an Ascomycete or a Basidiomycete, preferably a Basidiomycete, preferably an Agaricales.

9. A method for the production of the mushrooms as defined in any one of claims 1 to 8.

10. A method for producing a substance of interest using a mushroom as defined in any one of claims 1 to 8.

11. A nucleic acid construct comprising a nucleotide sequence encoding a polypeptide that comprises an amino acid sequence:
(a) that has at least 40 % amino acid identity with SEQ ID NO: 56; and/or,
(b) that has at least 50% amino acid identity with SEQ ID NO: 202;
wherein the nucleotide sequence is operably linked to a promoter that is capable of driving expression of the nucleotide sequence in a fungus or a mushroom.

12. A method for identification of a stimulus capable of influencing the production of a mushroom, the method comprising the steps of:
(a) providing a fungus or a mushroom;
(b) applying said stimulus to said fungus/mushroom;
(c) determining the expression level of a nucleotide sequence or the activity or steady state level of a corresponding encoded polypeptide in the fungus/mushroom of step b) wherein said polypeptide comprises an amino acid sequence that has at least 40% amino acid identity with SEQ ID NO: 56 and/or that has at least 50% amino acid identity with SEQ ID NO: 202,
(d) comparing the expression, activity or steady state level determined in (c) with the expression, activity or steady state level of the nucleotide sequence or of the polypeptide in a fungus/mushroom that had not been provided said stimulus; and,
(e) identifying a stimulus that produces a difference in expression level, activity or steady state level of said nucleotide sequence or polypeptide, between the fungus/mushroom that has been provided with said stimulus and the fungus/mushroom that has not been provided said stimulus

13. A method according to claim 12, whereby the expression levels, activities or steady state levels of more than one nucleotide sequence or more than one polypeptides are compared.

## Patentansprüche

1. Ein Fungus oder Pilz mit einem erhöhten Expressionslevel eines Polypeptids, das bei der Pilzentwicklung beteiligt ist, worin das Polypeptid mindestens 40% Aminosäureidentität mit SEQ ID NO: 56 aufweist und/oder mindestens 50% Aminosäureidentität mit SEQ ID NO: 202 aufweist und worin das erhöhte Expressionslevel des Polypeptids eine erhöhte Produktion des Polypeptids und/oder eine höhere Aktivität dieses Polypeptids, verglichen mit dem Elternfungus/Elternpilz, von dem der Fungus/Pilz abgeleitet ist, aufweist, wenn beide unter denselben Bedingungen gezüchtet und/oder geprüft werden.

2. Ein Fungus oder Pilz nach Anspruch 1, ferner umfassend ein erhöhtes oder erniedrigtes Expressionslevel eines Polypeptids, worin das Polypeptid, dessen Expressionslevel erhöht oder erniedrigt ist, mindestens 40% Aminosäureidentität mit einer Sequenz ausgewählt aus SEQ-ID NO: 1-55, 57-200 und/oder mindestens 50% Aminosäureidentität mit einer Sequenz ausgewählt aus SEQ ID NO: 201, 203-208 aufweist.

3. Ein Fungus oder Pilz nach Anspruch 1, worin das Polypeptid, dessen Expressionslevel erhöht ist, ein heterologes Peptid ist.

4. Ein Fungus oder Pilz nach Anspruch 1, worin das Polypeptid, dessen Expressionslevel erhöht ist, ein endogenes Peptid ist.

5. Ein Fungus oder Pilz nach Anspruch 2, worin das erniedrigte Expressionslevel erreicht wird durch eine erniedrigte Produktion dieses Polypeptids und/oder eine geringere Aktivität dieses Polypeptids verglichen mit dem Elternfungus/Elternpilz, von dem der Fungus/Pilz abgeleitet ist, wenn beide unter denselben Bedingungen gezüchtet und/oder geprüft werden.

6. Ein Fungus oder Pilz nach einem der Ansprüche 2 bis 5, worin das Polypeptid, dessen Expressionslevel erhöht ist, eine Aminosäuresequenz umfasst, die mindestens 40% Aminosäureidentität mit SEQ ID NO: 56 aufweist und worin das Polypeptid, dessen Expressionslevel erniedrigt ist, eine Aminosäuresequenz umfasst, die mindestens 40% Aminosäureidentität mit einer Sequenz ausgewählt aus SEQ ID NO: 55, 21, 54 aufweist.

7. Ein Fungus oder Pilz nach Anspruch 6, worin das Polypeptid, dessen Expressionslevel erhöht ist, eine Aminosäuresequenz umfasst, die mindestens 50% Aminosäureidentität mit SEQ ID NO: 202 aufweist und worin das Polypeptid, dessen Expressionslevel erniedrigt ist, eine Aminsäuresequenz umfasst, die mindestens 50% Aminosäureidentität mit einer Sequenz ausgewählt aus der Gruppe bestehend aus SEQ ID NO: 206, 205 und 203 aufweist.

8. Ein Fungus oder Pilz nach einem der vorhergehenden Ansprüche, worin der Fungus/Pilz ein Ascomycota oder ein Basidiomycota, vorzugsweise ein Basidiomycota, vorzugsweise ein Agaricales ist.

9. Ein Verfahren zur Herstellung der Pilze nach einem der Ansprüche 1 bis 8.

10. Ein Verfahren zur Herstellung einer interessierenden Substanz unter Verwendung eines wie in einem der Ansprüche 1 bis 8 definierten Pilzes.

11. Ein Nukleinsäurenkonstrukt, umfassend eine Nukleotidsequenz, die ein Polypeptid kodiert, welches eine Aminosäuresequenz umfasst,
a) die mindestens 40% Aminosäureidentität mit SEQ ID NO: 56 und/oder
b) die mindestens 50% Aminosäureidentität mit SEQ ID NO: 202 aufweist, worin die Nukleotidsequenz operativ mit einem Promoter verbunden ist, der in der Lage ist, die Expression der Nukleotidsequenz in einem Fungus oder Pilz durchzuführen.

12. Ein Verfahren zur Identifikation eines Stimulus, der befähigt ist, die Produktion eines Pilzes zu beeinflussen, wobei das Verfahren die folgenden Schritte umfasst:
(a) Bereitstellen eines Fungus oder Pilzes,
(b) Anwenden des Stimulus auf diesen Fungus/Pilz,
(c) Bestimmen des Expressionslevels einer Nukleotidsequenz oder der Aktivität oder des Gleichgewichtslevels eines korrespondierenden im Fungus/Pilz von Schritt b) kodierten Polypeptids, worin dieses Polypeptid eine Aminsäuresequenz umfasst, die mindestens 40% Aminsäureidentität mit SEQ ID NO: 56 und/oder die mindestens 50% Aminosäureaktivität mit SEQ ID NO:202 aufweist,
(d) Vergleichen des Levels der Expression, der Aktivität, oder des Gleichgewichts, bestimmt in (c) mit dem Level der Expression, der Aktivität oder des Gleichgewichts der Nukleotidsequenz oder des Polypeptids in einem Fungus/Pilz, der zuvor nicht mit diesem Stimulus versehen worden ist und
(e) Identifizieren eines Stimulus, der eine Differenz zwischen dem Fungus/Pilz, der mit diesem Stimulus versehen worden ist und dem Fungus/Pilz der nicht mit diesem Stimulus versehen worden ist, im Level der Expression, der Aktivität oder des Gleichgewichts dieser Nukleotidsequenz oder dieses Polypeptids erzeugt.

13. Ein Verfahren nach Anspruch 12, wobei die Expressionslevel, die Aktivitätslevel oder die Gleichgewichtslevel von mehr als einer Nukleotidsequenz oder mehr als einem Polypeptid verglichen werden.

## Revendications

1. Organisme fongique ou champignon présentant un taux d'expression accru d'un polypeptide impliqué dans la formation d'un champignon, dans lequel le polypeptide présente une identité d'acides aminés d'au moins 40 % avec SEQ ID NO: 56 et/ou présente une identité d'acides aminés d'au moins 50 % avec SEQ ID NO: 202, et dans lequel le taux d'expression accru du polypeptide est une production accrue du polypeptide et/ou une activité plus élevée dudit polypeptide par rapport à l'organisme fongique/au champignon parental à partir duquel est dérivé cet organisme fongique/ce champignon lorsque les deux sont cultivés et/ou analysés dans les mêmes conditions.

2. Organisme fongique ou champignon selon la revendication 1, comprenant en outre un taux d'expression accru ou réduit d'un polypeptide, dans lequel le polypeptide dont le taux d'expression est accru ou réduit présente une identité d'acides aminés d'au moins 40 % avec une séquence sélectionnée parmi SEQ ID NO: 1-55, 57-200 et/ou présente une identité d'acides aminés d'au moins 50 % avec une séquence sélectionnée parmi SEQ ID NO: 201, 203-208.

3. Organisme fongique ou champignon selon la revendication 1, dans lequel le polypeptide dont le taux d'expression est accru est un polypeptide hétérologue.

4. Organisme fongique ou champignon selon la revendication 1, dans lequel le polypeptide dont le taux d'expression est accru est un polypeptide endogène.

5. Organisme fongique ou champignon selon la revendication 2, dans lequel le taux d'expression réduit est obtenu par une production réduite du polypeptide et/ou une activité moindre dudit polypeptide par rapport à l'organisme fongique/au champignon parental à partir duquel est dérivé cet organisme fongique/ce champignon lorsque les deux sont cultivés et/ou analysés dans les mêmes conditions.

6. Organisme fongique ou champignon selon l'une quelconque des revendications 2 et 5, dans lequel le polypeptide dont le taux d'expression est accru comprend une séquence d'acides aminés qui présente une identité d'acides aminés d'au moins 40 % avec SEQ ID NO: 56 et dans lequel le polypeptide dont le taux d'expression est réduit comprend une séquence d'acides aminés qui présente une identité d'acides aminés d'au moins 40 % avec une séquence sélectionnée parmi SEQ ID NO: 55 ; 21 ; 54.

7. Organisme fongique ou champignon selon la revendication 6, dans lequel le polypeptide dont le taux d'expression est accru comprend une séquence d'acides aminés qui présente une identité d'acides aminés d'au moins 50 % avec SEQ ID NO: 202 et dans lequel le polypeptide dont le taux d'expression est réduit comprend une séquence d'acides aminés qui présente une identité d'acides aminés d'au moins 50 % avec une séquence sélectionnée dans le groupe constitué par SEQ ID NO: 206, 205 et 203.

8. Organisme fongique ou champignon selon l'une quelconque des revendications précédentes, dans lequel l'organisme fongique/le champignon est un Ascomycète ou un Basidiomycète, de préférence un Basidiomycète, de préférence un Agaricales.

9. Procédé de production des champignons tels que définis dans l'une quelconque des revendications 1 à 8.

10. Procédé de production d'une substance d'intérêt en utilisant un champignon tel que défini dans l'une quelconque des revendications 1 à 8.

11. Construction d'acide nucléique comprenant une séquence nucléotidique codant un polypeptide qui comprend une séquence d'acides aminés :
(a) qui présente une identité d'acides aminés d'au moins 40 % avec SEQ ID NO: 56 ; et/ou,
(b) qui présente une identité d'acides aminés d'au moins 50 % avec SEQ ID NO: 202 ;
dans lequel la séquence nucléotidique est liée de manière fonctionnelle avec un promoteur qui est capable de diriger l'expression de la séquence nucléotidique dans un organisme fongique ou un champignon.

12. Procédé d'identification d'un stimulus capable d'influencer la production d'un champignon, le procédé comprenant les étapes qui consistent à :
(a) mettre à disposition un organisme fongique ou un champignon ;
(b) appliquer ledit stimulus audit organisme fongique/champignon ;
(c) déterminer le taux d'expression d'une séquence nucléotidique ou l'activité ou le taux à l'état d'équilibre d'un polypeptide codé correspondant dans l'organisme fongique/le champignon de l'étape b), où ledit polypeptide comprend une séquence d'acides aminés qui présente une identité d'acides aminés d'au moins 40 % avec SEQ ID NO: 56 et/ou qui présente une identité d'acides aminés d'au moins 50 % avec SEQ ID NO: 202,
(d) comparer l'expression, l'activité ou le taux à l'état d'équilibre déterminé(e) en (c) avec l'expression, l'activité ou le taux à l'état d'équilibre de la séquence nucléotidique ou du polypeptide dans un organisme fongique/champignon qui n'a pas été exposé audit stimulus ; et,
(e) identifier un stimulus qui produit une différence du taux d'expression, de l'activité ou du taux à l'état d'équilibre de ladite séquence nucléotidique ou dudit polypeptide, entre l'organisme fongique/le champignon qui a été exposé audit stimulus et l'organisme fongique/le champignon qui n'a pas été exposé audit stimulus.

13. Procédé selon la revendication 12, moyennant quoi les taux d'expression, les activités ou les taux à l'état d'équilibre de plusieurs séquences nucléotidiques ou de plusieurs polypeptides sont comparé(e)s.


### SEQUENCE LISTING

<110> Stichting voor de Technische Wetenschappen Universiteit Holding B.V.
<120> Regulators involved in mushroom formation
<130> P6030560PCT
<150> US 61/212,953
   <151> 2009-04-20
<160> 241
<170> PatentIn version 3.3
<210> 1
   <211> 284
   <212> PRT
   <213> Schizophyllum commune
<400> 1

Met Asp Ser Tyr Asp His Ser Lys Pro Tyr Ser Val Asp Thr Met Asp
1               5                   10                  15

Pro Ser Gln Leu Asn Tyr Ser Ala Asp Met Gly Leu Ser Glu Tyr Ile
            20                  25                  30

His Phe Pro His Glu Ser Pro Met Gln Tyr Ala Asp Met Ser Leu Pro
        35                  40                  45

Pro Val Ala Ser Ser Glu Pro Met Asp Phe Gln Thr Leu Asn Met Ser
    50                  55                  60

Asn Tyr Asp Thr Asn Tyr Ala Ser Ser Ala Thr Tyr Ser Pro Ala Arg
65                  70                  75                  80

Pro Ile Thr Pro Leu Asp Gly Ala Ser Ile Ser Pro His Ala Leu Ala
                85                  90                  95

Tyr Pro Pro Ser Ala Gly Glu Leu Ser Ser Asp Gly Met Thr Ser Gly
            100                 105                 110

Arg Arg Ser Arg Gly Ser Gly Ser Pro Ala Pro Tyr Ser Thr Ala Gln
        115                 120                 125

Arg Ala Ala His Arg Tyr Thr Pro Met Gly Asn Pro Ala Thr Arg Pro
    130                 135                 140

Arg Val Arg Ala Gln Arg Lys Gly Ser Leu Lys Ser Asn Asp Asp Arg
145                 150                 155                 160

Asp Ser Asp Glu Asp Asp Asp Asp Phe Gln Pro Ile Gly Pro Ala Thr
                165                 170                 175